(19) Europäisches Patentamt
European Patent Office
Office européen des brevets

(11) EP 4 470 568 A1

(12) EUROPEAN PATENT APPLICATION
published in accordance with Art. 153(4) EPC

(43) Date of publication:
**04.12.2024 Bulletin 2024/49**

(21) Application number: **23746228.8**

(22) Date of filing: **19.01.2023**

(51) International Patent Classification (IPC):
***A61K 47/68*** *(2017.01)* ***C07K 16/28*** *(2006.01)*
***A61K 31/4745*** *(2006.01)* ***A61K 9/08*** *(2006.01)*
***A61K 9/19*** *(2006.01)* ***A61P 35/00*** *(2006.01)*

(52) Cooperative Patent Classification (CPC):
**A61K 9/08; A61K 9/19; A61K 31/4745; A61K 47/68; A61P 35/00; C07K 16/28**

(86) International application number:
**PCT/CN2023/073047**

(87) International publication number:
**WO 2023/143347 (03.08.2023 Gazette 2023/31)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **26.01.2022 CN 202210093514**

(71) Applicant: **Shanghai Mabgen Biotech Ltd.**
**Shanghai 201206 (CN)**

(72) Inventors:
- **SHEN, Lijun**
  **Shanghai 201206 (CN)**
- **ZHAO, Zhilong**
  **Shanghai 201206 (CN)**
- **TANG, Chenxiang**
  **Shanghai 201206 (CN)**
- **LI, Lei**
  **Shanghai 201206 (CN)**

(74) Representative: **Potter Clarkson**
**Chapel Quarter**
**Mount Street**
**Nottingham NG1 6HQ (GB)**

Remarks:
The complete document including Reference Table(s) and the Sequence Listing(s) can be downloaded from the EPO website

# (54) PHARMACEUTICAL COMPOSITION COMPRISING ANTI-CD79B ANTIBODY-DRUG CONJUGATE AND USE THEREOF

(57) A pharmaceutical composition comprising an anti-CD79b antibody-drug conjugate and use thereof. The pharmaceutical composition comprises a ligand-drug conjugate and a buffer. In the ligand-drug conjugate, the ligand is an anti-CD79b antibody or an antigen-binding fragment thereof, and the drug is selected from MMAE or a derivative thereof, exatecan or a derivative thereof, and eribulin or a derivative thereof. The buffer is selected from an acetate buffer, a histidine salt buffer, a Tris-hydrochloride buffer, a Tris-citrate buffer, a phosphate buffer, and a succinate buffer. The composition is used for preparing a medicament for treating or preventing a proliferative disease.

EP 4 470 568 A1

## Description

The present disclosure claims priority to Chinese Patent Application No. CN202210093514.0, filed on Jan. 26, 2022, entitled "PHARMACEUTICAL COMPOSITION COMPRISING ANTI-CD79B ANTIBODY-DRUG CONJUGATE AND USE THEREOF", which is incorporated herein by reference in its entirety.

## TECHNICAL FIELD

The present disclosure belongs to the field of pharmaceutical formulations and particularly relates to a pharmaceutical composition comprising an anti-CD79b antibody-drug conjugate and use thereof as a medicament.

## BACKGROUND

B cell antigen receptor (BCR) complex is the most prominent molecule on the surface of B cells. The BCR complex consists of a membrane immunoglobulin (mIg) that recognizes and binds to antigens and a heterodimer of Igα (CD79a) and Igβ (CD79b) that delivers antigen-stimulating signals. Igα and Igβ are glycoproteins of 47kDa and 37kDa, respectively, and are members of the immunoglobulin superfamily. The genes encoding Igα and Igβ are referred to as mb-1 and B29, respectively. Both Igα and Igβ have an Ig-like domain at the amino terminus in the extracellular region. Both Igα and Igβ can be used as substrates of protein tyrosine kinases and are involved in BCR signal transduction. BCR is widely expressed on B-cell lymphomas as well as on normal B cells. Given the clinical success and reliable safety of rituximab targeting CD20, development of BCR-targeting therapies should also have good efficacy and safety.

In response to the still unmet medical need associated with CD79b, a number of international pharmaceutical companies, including Roche, are currently active in developing antibodies against CD79b and related products, such as CD79b antibody-drug conjugates.

Antibody-drug conjugates (ADC) are formed by linking monoclonal antibodies or antibody fragments and cytotoxins with biological activity by stable chemical linker compounds, so that the specificity of the antibodies to bind to tumor cell-specific or highly expressed antigens and the high efficiency of the cytotoxins are combined to prevent toxic and side effects on normal cells. This means that antibody-drug conjugates can bind to tumor cells precisely and reduce the impact on normal cells compared to conventional chemotherapeutic drugs. An ADC drug consists of three components: an antibody (targeting part), a linker, and a toxin. The quality of the targeting part determines the specificity of the ADC drug, which not only includes specific targeting and binding but also effective endocytosis.

However, compared to other chemical drugs, antibody drugs, especially ADCs, have greater molecular weights and more complex structures, and are prone to degradation, aggregation, or undesirable chemical modifications that make them unstable. To ensure that antibody-drug conjugates are suitable for administration and remain stable during storage and subsequent use so that they can have better effects, developing stable antibody drug formulations is particularly important. The present disclosure provides a sufficiently stable and administration-friendly pharmaceutical composition comprising a CD79b ADC.

## SUMMARY

The present disclosure provides a pharmaceutical composition, comprising a ligand-drug conjugate (e.g., an anti-CD79b antibody-drug conjugate), and provides a method for preparing the pharmaceutical composition and a method for treating or preventing disease using same or related pharmaceutical use thereof.

The present disclosure provides a pharmaceutical composition, comprising a ligand-drug conjugate and a buffer, wherein the ligand is an anti-CD79b antibody or an antigen-binding fragment thereof; the drug is selected from the group consisting of MMAE or a derivative thereof, exatecan or a derivative thereof, and eribulin or a derivative thereof. In some embodiments, the buffer is selected from the group consisting of an acetate buffer, a histidine salt buffer, a Tris-hydrochloride buffer, a Tris-citrate buffer, a phosphate buffer, and a succinate buffer. In some embodiments, the buffer is a histidine salt buffer or a succinate buffer. In some specific embodiments, the buffer is a histidine-hydrochloride buffer or a succinic acid-sodium succinate buffer. The composition has activity for treating or preventing disease. In addition, the composition may also feature good stability and the like.

In some embodiments, the pharmaceutical composition according to any one of the above is provided, wherein the concentration of the ligand-drug conjugate is 0.1 mg/mL to 50 mg/mL, e.g., 0.5 mg/mL to 50 mg/mL, 1 mg/mL to 45 mg/mL, 1 mg/mL to 30 mg/mL, 1 mg/mL to 25 mg/mL, 5 mg/mL to 50 mg/mL, 10 mg/mL to 45 mg/mL, 15 mg/mL to 40 mg/mL, 20 mg/mL to 35 mg/mL, 25 mg/mL to 30 mg/mL, 5 mg/mL to 30 mg/mL, 10 mg/mL to 15 mg/mL, 10 mg/mL to 20 mg/mL, 10 mg/mL to 25 mg/mL, 10 mg/mL to 30 mg/mL, 15 mg/mL to 45 mg/mL, 15 mg/mL to 35 mg/mL, 15 mg/mL to 25 mg/mL, 15 mg/mL to 20 mg/mL, 20 mg/mL to 25 mg/mL, 20 mg/mL to 30 mg/mL, or any range between these point values. In some embodiments, the concentration of the ligand-drug conjugate is 10 mg/mL to 30 mg/mL. In some embodiments,

the concentration of the ligand-drug conjugate is 15 mg/mL to 25 mg/mL. In some non-limiting examples, the concentration of the ligand-drug conjugate is about 0.1 mg/mL, about 0.5 mg/mL, about 1 mg/mL, about 2 mg/mL, about 5 mg/mL, about 8 mg/mL, about 10 mg/mL, about 11 mg/mL, about 12 mg/mL, about 13 mg/mL, about 14 mg/mL, about 15 mg/mL, about 16 mg/mL, about 17 mg/mL, about 18 mg/mL, about 19 mg/mL, about 20 mg/mL, about 21 mg/mL, about 22 mg/mL, about 23 mg/mL, about 24 mg/mL, about 25 mg/mL, about 26 mg/mL, about 27 mg/mL, about 28 mg/mL, about 29 mg/mL, about 30 mg/mL, about 35 mg/mL, about 40 mg/mL, about 45 mg/mL, or about 50 mg/mL.

In some embodiments, the pharmaceutical composition according to any one of the above is provided, wherein the concentration of the buffer is 0.5 mM to 50 mM, e.g., 1 mM to 40 mM, 1 mM to 30 mM, 1 mM to 20 mM, 1 mM to 15 mM, 1 mM to 10 mM, 5 mM to 45 mM, 5 mM to 35 mM, 5 mM to 25 mM, 5 mM to 15 mM, 5 mM to 10 mM, 10 mM to 15 mM, 10 mM to 20 mM, 10 mM to 30 mM, or any range between these point values. In some embodiments, the pharmaceutical composition according to any one of the above is provided, wherein the concentration of the buffer is 1 mM to 30 mM. In some embodiments, the pharmaceutical composition according to any one of the above is provided, wherein the concentration of the buffer is 5 mM to 15 mM. In some non-limiting examples, the concentration of the buffer is about 0.5 mM, about 1 mM, about 2 mM, about 3 mM, about 4 mM, about 5 mM, about 6 mM, about 7 mM, about 8 mM, about 9 mM, about 10 mM, about 11 mM, about 12 mM, about 13 mM, about 14 mM, about 15 mM, about 16 mM, about 17 mM, about 18 mM, about 19 mM, about 20 mM, about 25 mM, about 30 mM, about 35 mM, about 40 mM, about 45 mM, or about 50 mM, e.g., about 10 mM.

In some embodiments, the pharmaceutical composition according to any one of the above has a pH of 4.0 to 8.5, e.g., 4.0 to 8.0, 4.5 to 7.5, 5.0 to 7.0, 5.6 to 6.5, 6.0 to 7.5, 4.5 to 6.5, 5.0 to 6.5, 5.0 to 6.0, 5.0 to 5.5, 5.6 to 6.0, 5.35 to 5.75 (e.g., about 5.6), 5.2 to 5.8, 5.5 to 6.5, 4.5 to 6.5, 4.5 to 6.0, 4.5 to 5.5, 4.0 to 6.0, 3.5 to 5.8, or any range between these point values. In some embodiments, the pharmaceutical composition according to any one of the above has a pH of 5.0 to 6.5. In some embodiments, the pharmaceutical composition according to any one of the above has a pH of 5.5 to 6.0. In some non-limiting examples, the pharmaceutical composition has a pH of about 4.0, about 4.5, about 4.6, about 4.7, about 4.8, about 4.9, about 4.95, about 5.0, about 5.05, about 5.1, about 5.15, about 5.2, about 5.25, about 5.3, about 5.35, about 5.4, about 5.45, about 5.5, about 5.55, about 5.6, about 5.65, about 5.7, about 5.75, about 5.8, about 5.85, about 5.9, about 5.95, about 6.0, about 6.1, about 6.2, about 6.3, about 6.4, about 6.5, about 7.0, about 7.5, about 8.0, or about 8.5.

Generally, the pH of the pharmaceutical composition obtained by buffer exchange is almost consistent with the pH of the buffer. Also, it is well known to those skilled in the art that in the process of pharmaceutical formulation, there may sometimes be a pH drift, but the pH drift of the pharmaceutical formulation is generally small (within a range of $\pm$0.3). In some embodiments, the pH drift of the pharmaceutical formulation is within a range of $\pm$0.1.

In some embodiments, the pharmaceutical composition according to any one of the above comprises a surfactant. In some embodiments, the surfactant is a nonionic surfactant. In some embodiments, the surfactant is selected from the group consisting of poloxamer (e.g., poloxamer 188), polysorbate (e.g., polysorbate 20 (i.e., PS20) or polysorbate 80 (i.e., PS80)), poloxamer, Triton, sodium dodecyl sulfonate, sodium lauryl sulfonate, sodium octyl glycoside, lauryl-sulfobetaine, myristyl-sulfobetaine, linoleyl-sulfobetaine, stearyl-sulfobetaine, lauryl-sarcosine, myristyl-sarcosine, linoleyl-sarcosine, stearyl-sarcosine, linoleyl-betaine, myristyl-betaine, cetyl-betaine, lauramido propyl-betaine, cocaramide propyl-betaine, linoleinamide propyl-betaine, myristylamide propyl-betaine, palmitamide propyl-betaine, isostearamide propyl-betaine, myristylamide propyl-dimethylamine, palmitamide propyl-dimethylamine, isostearamide propyl-dimethylamine, sodium methyl cocoyl, sodium methyl oleyl taurate, polyethylene glycol, polypropylene glycol, copolymers of ethylene and propylene glycol, and the like, or any combination thereof. In some embodiments, the surfactant is polysorbate or poloxamer. In some embodiments, the surfactant is polysorbate 80, polysorbate 20, or poloxamer 188. In some embodiments, the surfactant is polysorbate 80.

In some embodiments, the concentration of the surfactant is 0.01 mg/mL to 1 mg/mL, e.g., 0.01 mg/mL to 0.8 mg/mL, 0.05 mg/mL to 0.6 mg/mL, 0.08 mg/mL to 0.5 mg/mL, 0.1 mg/mL to 0.4 mg/mL, 0.1 mg/mL to 0.3 mg/mL, 0.15 mg/mL to 0.25 mg/mL, 0.2 mg/mL to 0.3 mg/mL, 0.1 mg/mL to 0.2 mg/mL, or any range between these point values. In some embodiments, the concentration of the surfactant is 0.1 mg/mL to 0.3 mg/mL. In some non-limiting examples, the concentration of the surfactant is about 0.01 mg/mL, about 0.02 mg/mL, about 0.03 mg/mL, about 0.04 mg/mL, about 0.05 mg/mL, about 0.06 mg/mL, about 0.07 mg/mL, about 0.08 mg/mL, about 0.09 mg/mL, about 0.1 mg/mL, about 0.11 mg/mL, about 0.12 mg/mL, about 0.13 mg/mL, about 0.14 mg/mL, about 0.15 mg/mL, about 0.16 mg/mL, about 0.17 mg/mL, about 0.18 mg/mL, about 0.19 mg/mL, about 0.2 mg/mL, about 0.21 mg/mL, about 0.22 mg/mL, about 0.23 mg/mL, about 0.24 mg/mL, about 0.25 mg/mL, about 0.3 mg/mL, about 0.4 mg/mL, about 0.5 mg/mL, about 0.6 mg/mL, about 0.7 mg/mL, about 0.8 mg/mL, about 0.9 mg/mL, or about 1 mg/mL.

In some embodiments, the pharmaceutical composition according to any one of the above comprises an osmotic pressure regulator. In some embodiments, the osmotic pressure regulator is a sugar (including monosaccharide, disaccharide, trisaccharide, polysaccharide, sugar alcohol, reducing sugar, non-reducing sugar, and the like), an amino acid (including arginine, glycine, cysteine, histidine, and the like) or a salt (sodium chloride, potassium chloride, calcium chloride, and the like). In some embodiments, the osmotic pressure regulator is a sugar, and the sugar is selected from the group consisting of glucose, sucrose, trehalose, lactose, fructose, maltose, dextran, glycerin, erythritol, glycerol, arabitol,

sylitol, sorbitol (also known as sorbol), mannitol, melibiose, melezitose, raffinose, mannotriose, stachyose, maltose, lactulose, maltulose, maltitol, lactitol, and iso-maltulose. In some embodiments, the osmotic pressure regulator is selected from the group consisting of one or more from the group consisting of sucrose, trehalose, sorbitol, arginine, proline, glycine, and sodium chloride. In some embodiments, the osmotic pressure regulator is a non-reducing disaccharide. In some embodiments, the osmotic pressure regulator is trehalose and/or sucrose. In some embodiments, the osmotic pressure regulator is sucrose.

In some embodiments, the concentration of the osmotic pressure regulator in the pharmaceutical composition according to any one of the above is 1 mg/mL to 300 mg/mL, e.g., 5 mg/mL to 200 mg/mL, 10 mg/mL to 150 mg/mL, 20 mg/mL to 140 mg/mL, 30 mg/mL to 130 mg/mL, 40 mg/mL to 120 mg/mL, 50 mg/mL to 110 mg/mL, 60 mg/mL to 100 mg/mL, 70 mg/mL to 90 mg/mL (e.g., about 80 mg/mL), or any range between these point values. In some non-limiting embodiments, the concentration of the osmotic pressure regulator is about 10 mg/mL, about 20 mg/mL, about 30 mg/mL, about 40 mg/mL, about 50 mg/mL, about 60 mg/mL, about 70 mg/mL, about 80 mg/mL, about 90 mg/mL, about 100 mg/mL, about 110 mg/mL, about 120 mg/mL, about 130 mg/mL, about 140 mg/mL, about 150 mg/mL, about 160 mg/mL, about 170 mg/mL, about 180 mg/mL, about 190 mg/mL, about 200 mg/mL, about 250 mg/mL, or about 300 mg/mL.

In some embodiments, the pharmaceutical composition according to any one of the above comprises any one of a)-o):

a) 0.1 mg/mL to 50 mg/mL ligand-drug conjugate (e.g., an anti-CD79b antibody-drug conjugate), 0.5 mMto 50 mM, 1 mMto 30 mM, 5 mM to 15 mM (e.g. about 10 mM) histidine salt buffer or succinate buffer, sucrose or trehalose, and polysorbate;

b) 10 mg/mL to 30 mg/mL ligand-drug conjugate (e.g., an anti-CD79b antibody-drug conjugate), 0.5 mMto 50 mM, 1 mMto 30 mM, 5 mM to 15 mM (e.g. about 10 mM) histidine salt buffer or succinate buffer, sucrose and/or trehalose, and polysorbate;

c) 15 mg/mL to 25 mg/mL (e.g. about 20 mg/mL) ligand-drug conjugate (e.g., an anti-CD79b antibody-drug conjugate), 0.5 mM to 50 mM, 1 mM to 30 mM, 5 mM to 15 mM (e.g. 10 mM) histidine salt buffer or succinate buffer, sucrose and/or trehalose, and polysorbate;

l) 10 mg/mL to 50 mg/mL ligand-drug conjugate (e.g., an anti-CD79b antibody-drug conjugate), 0.5 mMto 50 mM, 1 mMto 30 mM, 5 mM to 15 mM (e.g. about 10 mM) histidine salt buffer or succinate buffer, sucrose and/or trehalose, and polysorbate;

wherein the pharmaceutical compositions in a) to c) have a pH of 4.0 to 8.5, 5.0 to 6.5, or 5.5 to 6.0 (e.g. about 5.0 or about 5.6);

in some embodiments, the concentration of sucrose or trehalose in the pharmaceutical compositions in a) to c) and l) is 1 mg/mL to 300 mg/mL, 30 mg/mL to 130 mg/mL, or 70 mg/mL to 90 mg/mL (e.g., about 70 mg/mL, about 80 mg/mL, about 90 mg/mL, about 100 mg/mL, about 110 mg/mL, or about 120 mg/mL);

in some embodiments, the polysorbate in the pharmaceutical compositions in a) to c) and l) is polysorbate 20 or polysorbate 80, and the concentration of the polysorbate is 0.01 mg/mL to 1 mg/mL, 0.05 mg/mL to 0.6 mg/mL, or 0.1 mg/mL to 0.3 mg/mL (e.g., about 0.1 mg/mL, about 0.2 mg/mL, or about 0.3 mg/mL);

d) 0.1 mg/mL to 50 mg/mL ligand-drug conjugate (e.g., an anti-CD79b antibody-drug conjugate), 0.5 mM to 50 mM histidine salt buffer or succinate buffer, 1 mg/mL to 300 mg/mL sucrose and/or trehalose, and 0.01 mg/mL to 1 mg/mL polysorbate 80 or polysorbate 20, pH 4.0 to 8.5;

m) 10 mg/mL to 50 mg/mL ligand-drug conjugate (e.g., an anti-CD79b antibody-drug conjugate), 1 mM to 30 mM histidine salt buffer or succinate buffer, 30 mg/mL to 130 mg/mL sucrose and/or trehalose, and 0.05 mg/mL to 0.6 mg/mL polysorbate 80 or polysorbate 20, pH 5.0 to 6.5;

e) 10 mg/mL to 30 mg/mL ligand-drug conjugate (e.g., an anti-CD79b antibody-drug conjugate), 1 mM to 30 mM histidine salt buffer or succinate buffer, 30 mg/mL to 130 mg/mL sucrose and/or trehalose, and 0.05 mg/mL to 0.6 mg/mL polysorbate 80 or polysorbate 20, pH 5.0 to 6.5;

f) 10 mg/mL to 30 mg/mL ligand-drug conjugate (e.g., an anti-CD79b antibody-drug conjugate), 1 mM to 30 mM histidine salt buffer, 30 mg/mL to 130 mg/mL sucrose, and 0.05 mg/mL to 0.6 mg/mL polysorbate 80, pH 5.0 to 6.5;

g) 15 mg/mL to 25 mg/mL ligand-drug conjugate (e.g., an anti-CD79b antibody-drug conjugate), 5 mM to 15 mM histidine salt buffer, 70 mg/mL to 90 mg/mL sucrose, and 0.1 mg/mL to 0.3 mg/mL polysorbate 80, pH 5.5 to 6.0;

h) 15 mg/mL to 25 mg/mL ligand-drug conjugate (e.g., an anti-CD79b antibody-drug conjugate), 10 mM histidine salt buffer, about 80 mg/mL sucrose, and 0.1 mg/mL to 0.3 mg/mL polysorbate 80, pH 5.5 to 6.0 (e.g., about 5.6);

n) 15 mg/mL to 25 mg/mL ligand-drug conjugate (e.g., an anti-CD79b antibody-drug conjugate), 5 mM to 15 mM histidine-hydrochloride buffer, 70 mg/mL to 90 mg/mL sucrose, and 0.1 mg/mL to 0.3 mg/mL polysorbate 80, pH 5.5 to 6.0;

o) 15 mg/mL to 25 mg/mL ligand-drug conjugate (e.g., an anti-CD79b antibody-drug conjugate), 10 mM histidine-hydrochloride buffer, about 80 mg/mL sucrose, and 0.1 mg/mL to 0.3 mg/mL polysorbate 80, pH 5.5 to 6.0 (e.g. about 5.6);

i) 15 mg/mL to 25 mg/mL ligand-drug conjugate (e.g., an anti-CD79b antibody-drug conjugate), about 10 mM histidine-hydrochloride buffer, about 80 mg/mL sucrose, and about 0.2 mg/mL polysorbate 80, about pH 5.6;

p-1) 15 mg/mL to 25 mg/mL ligand-drug conjugate (e.g., an anti-CD79b antibody-drug conjugate), about 10 mM histidine-histidine hydrochloride, about 80 mg/mL sucrose, and about 0.2 mg/mL polysorbate 80, about pH 5.6;

p-2) 15 mg/mL to 25 mg/mL ligand-drug conjugate (e.g., an anti-CD79b antibody-drug conjugate), about 10 mM histidine-histidine hydrochloride, about 80 mg/mL sucrose, and about 0.2 mg/mL polysorbate 80, about pH 5.5;

p-3) 15 mg/mL to 25 mg/mL ligand-drug conjugate (e.g., an anti-CD79b antibody-drug conjugate), about 10 mM histidine-histidine hydrochloride, about 80 mg/mL sucrose, and about 0.2 mg/mL polysorbate 80, about pH 5.75;

p-4) 15 mg/mL to 25 mg/mL ligand-drug conjugate (e.g., an anti-CD79b antibody-drug conjugate), about 10 mM histidine-histidine hydrochloride, about 80 mg/mL sucrose, and about 0.2 mg/mL polysorbate 80, about pH 6.0;

p-5) 15 mg/mL to 25 mg/mL ligand-drug conjugate (e.g., an anti-CD79b antibody-drug conjugate), about 10 mM histidine-histidine hydrochloride, about 80 mg/mL sucrose, and about 0.3 mg/mL polysorbate 80, about pH 5.5;

p-6) 15 mg/mL to 25 mg/mL ligand-drug conjugate (e.g., an anti-CD79b antibody-drug conjugate), about 10 mM histidine-histidine hydrochloride, about 80 mg/mL sucrose, and about 0.3 mg/mL polysorbate 80, about pH 5.75;

p-7) 15 mg/mL to 25 mg/mL ligand-drug conjugate (e.g., an anti-CD79b antibody-drug conjugate), about 10 mM histidine-histidine hydrochloride, about 80 mg/mL sucrose, and about 0.3 mg/mL polysorbate 80, about pH 6.0;

p-8) 15 mg/mL to 25 mg/mL ligand-drug conjugate (e.g., an anti-CD79b antibody-drug conjugate), about 10 mM histidine-histidine hydrochloride, about 80 mg/mL sucrose, and about 0.1 mg/mL polysorbate 80, about pH 5.5;

p-9) 15 mg/mL to 25 mg/mL ligand-drug conjugate (e.g., an anti-CD79b antibody-drug conjugate), about 10 mM histidine-histidine hydrochloride, about 80 mg/mL sucrose, and about 0.1 mg/mL polysorbate 80, about pH 5.75;

p-10) 15 mg/mL to 25 mg/mL ligand-drug conjugate (e.g., an anti-CD79b antibody-drug conjugate), about 10 mM histidine-histidine hydrochloride, about 80 mg/mL sucrose, and about 0.1 mg/mL polysorbate 80, about pH 6.0;

j) 15 mg/mL to 25 mg/mL ligand-drug conjugate (e.g., an anti-CD79b antibody-drug conjugate), 5 mM to 15 mM succinate buffer, 70 mg/mL to 90 mg/mL sucrose, and 0.1 mg/mL to 0.3 mg/mL polysorbate 80, pH 4.5 to 6.5 (e.g., 5.0 to 6.5);

k) 15 mg/mL to 25 mg/mL ligand-drug conjugate (e.g., an anti-CD79b antibody-drug conjugate), about 10 mM succinic acid-sodium succinate buffer, about 80 mg/mL sucrose, and 0.1 mg/mL to 0.3 mg/mL polysorbate 80, about pH 5.0.

The anti-CD79b antibody-drug conjugate may be ADC-3 of Example 3-1, or ADC-5 of Example 3-2, or ADC-6 of Example 3-3, or ADC-7 of Example 3-4 of the present disclosure; for example, the anti-CD79b antibody-drug conjugate is ADC-6 of Example 3-3.

The pharmaceutical composition of the present disclosure also comprises a solvent. The solvent in the pharmaceutical composition is selected from, without limitation, a non-toxic physiologically acceptable liquid carrier, such as normal saline, water for injection, or a glucose solution (e.g. 5% glucose injection or glucose-sodium chloride injection).

The present disclosure also provides a pharmaceutical composition, obtained by diluting the pharmaceutical composition according to any one of the foregoing with 0.9% normal saline or 5% glucose solution, or comprising an anti-CD79b antibody-drug conjugate, a histidine salt, sucrose, and polysorbate 80 each at a concentration suitable for intravenous injection obtained by diluting the pharmaceutical composition according to any one of the foregoing with 0.9% normal saline or 5% glucose solution.

<u>Ligand-Drug Conjugate</u>

In some embodiments, the ligand-drug conjugate in the pharmaceutical composition according to any one of the above is provided, wherein:

the drug is selected from the group consisting of MMAE or a derivative thereof, exatecan or a derivative thereof, and eribulin or a derivative thereof; the ligand is an anti-CD79b antibody or an antigen-binding fragment thereof, and the anti-CD79b antibody or the antigen-binding fragment thereof comprises a heavy chain variable region (VH) and a light chain variable region (VL), wherein: the VH comprises a HCDR1, a HCDR2, and a HCDR3 of the amino acid sequences set forth in SEQ ID NOs: 11, 6, and 7, respectively, and the VL comprises a LCDR1, a LCDR2, and a LCDR3 of the amino acid sequences set forth in SEQ ID NOs: 8, 9, and 10, respectively;

the VH comprises a HCDR1, a HCDR2, and a HCDR3 of the amino acid sequences set forth in SEQ ID NOs: 5, 6, and 7, respectively, and the VL comprises a LCDR1, a LCDR2, and a LCDR3 of the amino acid sequences set forth in SEQ ID NOs: 8, 9, and 10, respectively.

In some embodiments, the anti-CD79b antibody or the antigen-binding fragment thereof in the pharmaceutical composition may be selected from the group consisting of a murine antibody, a chimeric antibody, and a humanized antibody, e.g., a humanized antibody.

In optional embodiments, the light chain and the light and heavy chain FR sequences in the VH of the humanized anti-CD79b antibody in the pharmaceutical composition are derived from human germline light and heavy chain FRs or mutated sequences thereof, respectively.

In some embodiments, the anti-CD79b antibody or the antigen-binding fragment thereof in the pharmaceutical composition comprises an antibody VH and an antibody VL, wherein:
the VH sequence is set forth in SEQ ID NO: 3 or is an amino acid sequence having at least 80%, at least 85%, or at least 90% identity thereto, and the VL sequence is set forth in SEQ ID NO: 4 or is an amino acid sequence having at least 80%, at least 85%, or at least 90% identity thereto.

In some specific embodiments, the anti-CD79b antibody or the antigen-binding fragment thereof in the pharmaceutical composition comprises an antibody VH and an antibody VL, wherein: the VH sequence is set forth in SEQ ID NO: 3, and the VL sequence is set forth in SEQ ID NO: 4.

In some embodiments, the anti-CD79b antibody or the antigen-binding fragment thereof further comprises a heavy chain constant region and a light chain constant region. In optional embodiments, the heavy chain constant region is selected from the group consisting of human IgG1, IgG2, IgG3, and IgG4 constant regions and conventional variants thereof, and the light chain constant region is selected from the group consisting of human $\kappa$ and $\lambda$ chain constant regions and conventional variants thereof. In some specific embodiments, the heavy chain constant region is human IgG1 or IgG4.

In some embodiments, the anti-CD79b antibody or the antigen-binding fragment thereof comprises an Fc region of an IgG, e.g., an Fc region of a human IgG, e.g., an Fc region of human IgG1, IgG2, or IgG4.

In some embodiments, the anti-CD79b antibody or the antigen-binding fragment thereof comprises a heavy chain and a light chain, wherein: the heavy chain has the sequence set forth in SEQ ID NO: 12 or a sequence having at least 80%, at least 85%, or at least 90% identity thereto, and the light chain has the sequence set forth in SEQ ID NO: 13 or a sequence having at least 80%, at least 85%, or at least 90% identity thereto.

In the present disclosure, the "at least 90% identity" encompasses at least 90%, at least 95%, at least 96%, at least 97%, at least 98%, and at least 99% identity.

In some specific embodiments, the anti-CD79b antibody or the antigen-binding fragment thereof in the pharmaceutical composition comprises an antibody heavy chain and an antibody light chain, wherein: the heavy chain sequence is set forth in SEQ ID NO: 12, and the light chain sequence is set forth in SEQ ID NO: 13.

In some specific embodiments, the anti-CD79b antibody or the fragment thereof described above may be a variant that has 1 to 10 (e.g., 1, 2, 3, 4, 5, 6, 7, 8, 9, or 10) amino acid changes in the VL and/or 1 to 10 (e.g., 1, 2, 3, 4, 5, 6, 7, 8, 9, or 10) amino acid changes in the VH.

In some specific embodiments, the variant described above has biological functions or effects that are identical or similar to those of the original anti-CD79b antibody or the fragment thereof.

In some embodiments, the antigen-binding fragment of the anti-CD79b antibody is a Fab, an Fv, an sFv, a Fab', a F(ab')2, a linear antibody, a single-chain antibody, an scFv, an sdAb, an sdFv, a nanobody, a peptibody, a domain antibody, and a multispecific antibody (a bispecific antibody, a diabody, a triabody, and a tetrabody, a tandem di-scFv, a tandem tri-scFv), for example, specifically an scFv, Fv, Fab, or Fab' fragment.

Ligand-Drug (Exatecan or Derivative Thereof) Conjugate

In some embodiments, the ligand-drug conjugate in the pharmaceutical composition according to any one of the foregoing is a ligand-exatecan (or a derivative thereof) conjugate represented by general formula (Pc-L-Y-D) of formula (I):

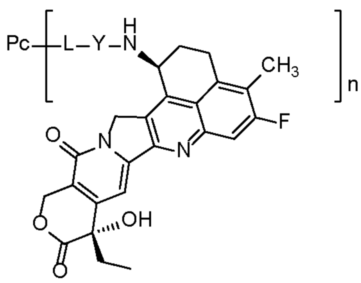

(Pc-L-Y-D) formula (I)

wherein:

Y is selected from the group consisting of -O-$(CR^aR^b)_m$-$CR^1R^2$-C(O)-, -O-$CR^1R^2$-$(CR^aR^b)_m$-, -O-$CR^1R^2$-, -NH-$(CRaR^b)_m$-CR1$R^2$-C(O)-, and -S-$(CR^aR^b)_m$-$CR^1R^2$-C(O)-;

$R^a$ and $R^b$ are identical or different and are each independently selected from the group consisting of a hydrogen atom, a deuterium atom, halogen, alkyl, haloalkyl, deuterated alkyl, alkoxy, hydroxy, amino, cyano, nitro, hydroxyalkyl, cycloalkyl, and heterocyclyl; or, $R^a$ and $R^b$, together with the carbon atom to which they are attached, form cycloalkyl and heterocyclyl;

$R^1$ is selected from the group consisting of a hydrogen atom, halogen, haloalkyl, deuterated alkyl, cycloalkyl, cycloalkylalkyl, alkoxyalkyl, heterocyclyl, aryl, and heteroaryl; $R^2$ is selected from the group consisting of a hydrogen atom, halogen, haloalkyl, deuterated alkyl, cycloalkyl, cycloalkylalkyl, alkoxyalkyl, heterocyclyl, aryl, and heteroaryl; or, $R^1$ and $R^2$, together with the carbon atom to which they are attached, form cycloalkyl or heterocyclyl;

or, $R^a$ and $R^2$, together with the carbon atom to which they are attached, form cycloalkyl or heterocyclyl;

m is an integer from 0 to 4;

n is 1 to 10, and n is a decimal or integer; preferably, n is 2 to 8 or 5 to 9;

L is a linker unit;

Pc is any of the aforementioned anti-CD79b antibodies or antigen-binding fragments thereof of the present disclosure.

In some embodiments, the ligand-exatecan (or the derivative thereof) conjugate of the present disclosure or a pharmaceutically acceptable salt or solvate thereof is provided, wherein -Y- is -O-$(CR^aR^b)$m-$CR^1R^2$-C(O)-;

$R^a$ and $R^b$ are identical or different and are each independently selected from the group consisting of a hydrogen atom, a deuterium atom, halogen, and alkyl;

$R^1$ is $C_{3-6}$ cycloalkylalkyl or $C_{3-6}$ cycloalkyl, or a hydrogen atom;

$R^2$ is selected from the group consisting of a hydrogen atom, haloalkyl, and $C_{3-6}$ cycloalkyl; preferably a hydrogen atom;

or, $R^1$ and $R^2$, together with the carbon atom to which they are attached, form $C_{3-6}$ cycloalkyl; m is 0 or 1.

In some embodiments, the ligand-exatecan (or the derivative thereof) conjugate of the present disclosure is provided, wherein the structural unit -Y- is -O-$(CH_2)$m-$CR^1R^2$-C(O)-;

$R^1$ is $C_{3-6}$ cycloalkylalkyl or $C_{3-6}$ cycloalkyl, or a hydrogen atom;

$R^2$ is selected from the group consisting of a hydrogen atom, haloalkyl, and $C_{3-6}$ cycloalkyl;

or, $R^1$ and $R^2$, together with the carbon atom to which they are attached, form $C_{3-6}$ cycloalkyl;

m is 0 or 1.

In some embodiments, the ligand-exatecan (or the derivative thereof) conjugate of the present disclosure is provided, wherein the structural unit -Y- is -O-$(CH_2)$m-$CR^1R^2$-C(O)-;

$R^1$ is $C_{3-6}$ cycloalkylalkyl or $C_{3-6}$ cycloalkyl, or a hydrogen atom;

$R^2$ is a hydrogen atom;

or, $R^1$ and $R^2$, together with the carbon atom to which they are attached, form $C_{3-6}$ cycloalkyl;

m is 0 or 1.

In some embodiments, the ligand-exatecan (or the derivative thereof) conjugate of the present disclosure is provided, wherein the structural unit -Y- is -O-$(CH_2)$m-$CR^1R^2$-C(O)-;

$R^1$ is $C_{3-6}$ cycloalkylalkyl or $C_{3-6}$ cycloalkyl, or a hydrogen atom;

$R^2$ is a hydrogen atom;

or, $R^1$ and $R^2$, together with the carbon atom to which they are attached, form $C_{3-6}$ cycloalkyl;

m is 0.

In some embodiments, the ligand-exatecan (or the derivative thereof) conjugate of the present disclosure is provided, wherein the structural unit -Y- is -O-$(CH_2)$m-$CR^1R^2$-C(O)-;

$R^1$ is a hydrogen atom;

$R^2$ is a hydrogen atom;

or, $R^1$ and $R^2$, together with the carbon atom to which they are attached, form $C_{3-6}$ cycloalkyl;

m is 0.

In some embodiments, in the ligand-exatecan (or the derivative thereof) conjugate of the present disclosure, Y is selected from the group consisting of:

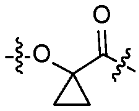, 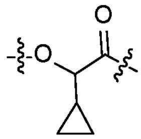, 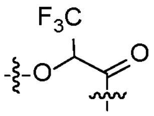, 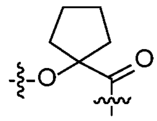, 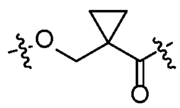, 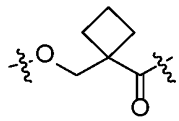, 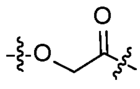,

and

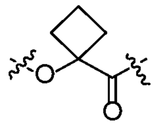;

wherein the O-terminus of Y is attached to the linker unit L.

In some other embodiments, the present disclosure provides a ligand-exatecan (or a derivative thereof) conjugate represented by general formula (Pc-L-D1) of formula (II):

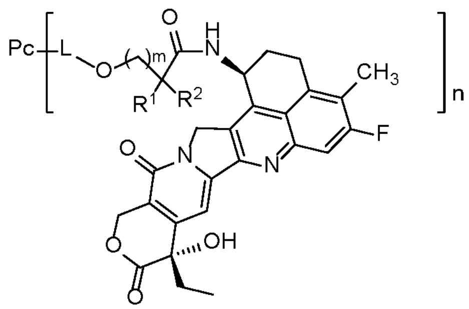

(Pc-L-D1) formula (II)

wherein:

$R^1$ is a hydrogen atom, $C_{3-6}$ cycloalkylalkyl, or $C_{3-6}$ cycloalkyl;

$R^2$ is selected from the group consisting of a hydrogen atom, haloalkyl, and $C_{3-6}$ cycloalkyl; preferably a hydrogen atom;

or, $R^1$ and $R^2$, together with the carbon atom to which they are attached, form $C_{3-6}$ cycloalkyl;

m is 0 or 1;

n is 1 to 10 and may be an integer or decimal; preferably, n is a decimal or integer from 1 to 8 or 1 to 6; more preferably, n is a decimal or integer from 1 to 5 or 2 to 4.

In some specific embodiments, the present disclosure provides a ligand-exatecan (or a derivative thereof) conjugate, wherein n is 1 to 8 and may be an integer or decimal; preferably, n is 1 to 6 and may be an integer or decimal.

In some embodiments, the present disclosure provides a ligand-exatecan (or a derivative thereof) conjugate, wherein the linker unit -L- is $-L^1-L^2-L^3-L^4-$;

$L^1$ is selected from the group consisting of -(succinimid-3-yl-*N*)-W-C(O)-, $-CH_2$-C(O)-$NR^3$-W-C(O)-, and -C(O)-W-C(O)-, wherein W is selected from the group consisting of $C_{1-8}$ alkyl, $C_{1-8}$ alkyl-cycloalkyl, and linear heteroalkyl of 1 to 8 atoms, and the heteroalkyl comprises 1 to 3 heteroatoms selected from the group consisting of N, O, and S, wherein the $C_{1-8}$ alkyl, cycloalkyl, and linear heteroalkyl are each independently optionally further substituted with one or more substituents selected from the group consisting of halogen, hydroxy, cyano, amino, alkyl, chloroalkyl, deuterated alkyl, alkoxy, and cycloalkyl;

$L^2$ is selected from the group consisting of $-NR^4(CH_2CH_2O)p^1CH_2CH_2C(O)-$, - $NR^4(CH_2CH_2O)p^1CH_2C(O)-$, $-S(CH_2)p^1C(O)-$, and a chemical bond, wherein $p^1$ is an integer from 1 to 20;

$L^3$ is a peptide residue consisting of 2 to 7 amino acids, wherein the amino acids are selected from the group consisting of amino acid residues formed from amino acids from phenylalanine, glycine, valine, lysine, citrulline, serine, glutamic

acid, and aspartic acid, and are optionally further substituted with one or more substituents selected from the group consisting of halogen, hydroxy, cyano, amino, alkyl, chloroalkyl, deuterated alkyl, alkoxy, and cycloalkyl;

$L^4$ is selected from the group consisting of $-NR^5(CR^6R^7)_t-$, $-C(O)NR^5$, $-C(O)NR^5(CH_2)_t-$, and a chemical bond, wherein t is an integer from 1 to 6;

$R^3$, $R^4$, and $R^5$ are identical or different and are each independently selected from the group consisting of a hydrogen atom, alkyl, haloalkyl, deuterated alkyl, and hydroxyalkyl;

$R^6$ and $R^7$ are identical or different and are each independently selected from the group consisting of a hydrogen atom, halogen, alkyl, haloalkyl, deuterated alkyl, and hydroxyalkyl.

In some embodiments, the present disclosure provides a ligand-exatecan (or a derivative thereof) conjugate, wherein the linker unit $L^1$ is selected from the group consisting of -(succinimid-3-yl-*N*)-$(CH_2)s^1$-C(O)-, -(succinimid-3-yl-*N*)-CH2-cyclohexyl-C(O)-, -(succinimid-3-yl-*N*)-$(CH_2CH_2O)s^2$-$CH_2CH_2$-C(O)-, -$CH_2$-C(O)-$NR^3$-$(CH_2)s^3$-C(O)-, and -C(O)-$(CH_2)s^4$C(O)-, wherein $s^1$ is an integer from 2 to 8, $s^2$ is an integer from 1 to 3, $s^3$ is an integer from 1 to 8, and $s^4$ is an integer from 1 to 8; $s^1$ is preferably 5.

In some specific embodiments, the present disclosure provides a ligand-exatecan (or a derivative thereof) conjugate, wherein the linker unit $L^2$ is selected from the group consisting of - $NR^4(CH_2CH_2O)p^1CH_2C(O)$- and a chemical bond, and $p^1$ is an integer from 6 to 12.

In some specific embodiments, the present disclosure provides a ligand-exatecan (or a derivative thereof) conjugate, wherein $L^4$ is selected from the group consisting of $-NR^5(CR^6R^7)t-$; $R^5$ is selected from the group consisting of a hydrogen atom and alkyl; $R^6$ and $R^7$ are identical or different and are each independently a hydrogen atom or alkyl; t is 1 or 2, preferably 2; $L^4$ is preferably $-NR^5CR^6R^7-$; $L^4$ is more preferably $-NHCH_2-$.

In some specific embodiments, the present disclosure provides a ligand-exatecan (or a derivative thereof) conjugate, wherein the linker unit -L- is $-L^1-L^2-L^3-L^4-$;

$L^1$ is

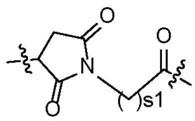

,

and $s^1$ is an integer from 2 to 8;

$L^2$ is a chemical bond;

$L^3$ is a tetrapeptide residue;

$L^4$ is $-NR^5(CR^6R^7)t-$; $R^5$ is selected from the group consisting of a hydrogen atom and alkyl; $R^6$ and $R^7$ are identical or different and are each independently a hydrogen atom or alkyl; t is 1 or 2.

In some specific embodiments, the present disclosure provides a ligand-exatecan (or a derivative thereof) conjugate, wherein the linker unit -L- is $-L^1-L^2-L^3-L^4-$;

$L^1$ is -(succinimid-3-yl-*N*)-$CH_2$-cyclohexyl-C(O)-;

$L^2$ is $-NR^4(CH_2CH_2O)_9CH_2C(O)-$;

$L^3$ is a tetrapeptide residue;

$L^4$ is $-NR^5(CR^6R^7)t-$; $R^5$ is selected from the group consisting of a hydrogen atom and alkyl; $R^6$ and $R^7$ are identical or different and are each independently a hydrogen atom or alkyl; t is 1 or 2.

In some specific embodiments, the present disclosure provides a ligand-exatecan (or a derivative thereof) conjugate, wherein the peptide residue of $L^3$ is an amino acid residue formed from one, two, or more amino acids selected from the group consisting of phenylalanine (E), glycine (G), valine (V), lysine (K), citrulline, serine (S), glutamic acid (E), and aspartic acid (N), preferably an amino acid residue formed from one, two, or more amino acids selected from the group consisting of phenylalanine and glycine, more preferably a tetrapeptide residue, and most preferably a tetrapeptide residue of GGFG (glycine-glycine-phenylalanine-glycine).

In some embodiments, the present disclosure provides a ligand-exatecan (or a derivative thereof) conjugate, wherein the $L^1$ terminus of the linker unit -L- is attached to the ligand, and the $L^4$ terminus is attached to Y

In some embodiments, the present disclosure provides a ligand-exatecan (or a derivative thereof) conjugate, wherein the -L-Y- is:

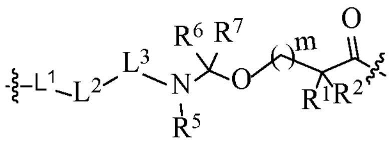

$L^1$ is selected from the group consisting of -(succinimid-3-yl-*N*)-$(CH_2)s^1$-C(O)- and -(succinimid-3-yl-*N*)-$CH_2$-cyclohexyl-C(O)-;
$L^2$ is -$NR^4(CH_2CH_2O)p^1CH_2C(O)$- or a chemical bond, and $p^1$ is an integer from 6 to 12;
$L^3$ is a tetrapeptide residue of GGFG;
$R^1$ is a hydrogen atom, cycloalkylalkyl, or cycloalkyl, preferably $C_{3-6}$ cycloalkylalkyl or $C_{3-6}$ cycloalkyl;
$R^2$ is selected from the group consisting of a hydrogen atom, haloalkyl, and $C_{3-6}$ cycloalkyl; preferably a hydrogen atom;
or, $R^1$ and $R^2$, together with the carbon atom to which they are attached, form $C_{3-6}$ cycloalkyl;
$R^5$ is selected from the group consisting of a hydrogen atom and alkyl; $R^6$ and $R^7$ are identical or different and are each independently a hydrogen atom or alkyl;
$s^1$ is an integer from 2 to 8; preferably 5;
m is an integer from 0 to 4.

In some embodiments, the present disclosure provides a ligand-exatecan (or a derivative thereof) conjugate,

wherein the -L-Y- is:

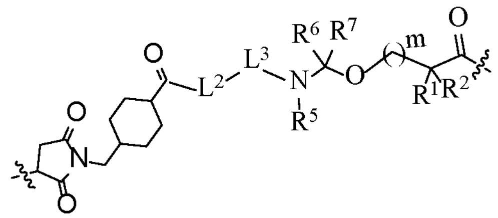

preferably:

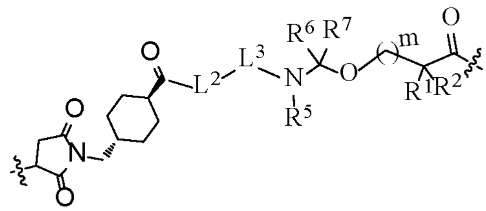

$L^2$ is -$NR^4(CH_2CH_2O)_9CH_2C(O)$-;
$L^3$ is a tetrapeptide residue of GGFG;
$R^1$ is a hydrogen atom, cycloalkylalkyl, or cycloalkyl, preferably $C_{3-6}$ cycloalkylalkyl or $C_{3-6}$ cycloalkyl;
$R^2$ is selected from the group consisting of a hydrogen atom, haloalkyl, and $C_{3-6}$ cycloalkyl; preferably a hydrogen atom;
or, $R^1$ and $R^2$, together with the carbon atom to which they are attached, form $C_{3-6}$ cycloalkyl;
$R^5$ is selected from the group consisting of a hydrogen atom and alkyl; $R^6$ and $R^7$ are identical or different and are each independently a hydrogen atom or alkyl;
m is an integer from 0 to 4.

In some embodiments, the present disclosure provides a ligand-exatecan (or a derivative thereof) conjugate, wherein the ligand-exatecan (or the derivative thereof) conjugate comprises a structure represented by formula (III):

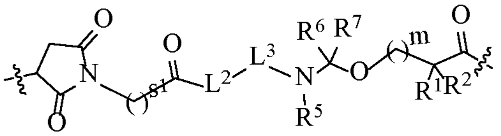

(-L-Y-) formula (III)

wherein:

$L^2$ is a chemical bond;
$L^3$ is a tetrapeptide residue of GGFG;
$R^1$ is a hydrogen atom, $C_{3-6}$ cycloalkylalkyl, or $C_{3-6}$ cycloalkyl;
$R^2$ is selected from the group consisting of a hydrogen atom, haloalkyl, and $C_{3-6}$ cycloalkyl;
or, $R^1$ and $R^2$, together with the carbon atom to which they are attached, form $C_{3-6}$ cycloalkyl;
$R^5$ is selected from the group consisting of a hydrogen atom and alkyl; $R^6$ and $R^7$ are identical or different and are each independently a hydrogen atom or alkyl;
$s^1$ is an integer from 2 to 8;
m is an integer from 0 to 4.

In some embodiments, the present disclosure provides a ligand-exatecan (or a derivative thereof) conjugate, wherein the ligand-exatecan (or the derivative thereof) conjugate comprises a structure represented by formula (-L-Y-):

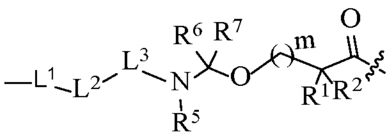

which can be used to obtain a ligand-drug conjugate formed by linking a drug and a ligand by a linker fragment; wherein:

$L^1$ is selected from the group consisting of -(succinimid-3-yl-*N*)-$(CH_2)s^1$-C(O)- and -(succinimid-3-yl-*N*)-$CH_2$-cyclohexyl-C(O)-;
$L^2$ is -$NR^4(CH_2CH_2O)p^1CH_2C(O)$- or a chemical bond, and $p^1$ is an integer from 1 to 20;
$L^3$ is a tetrapeptide residue of GGFG;
$R^1$ is a hydrogen atom, cycloalkylalkyl, or cycloalkyl, preferably $C_{3-6}$ cycloalkylalkyl or $C_{3-6}$ cycloalkyl;
$R^2$ is selected from the group consisting of a hydrogen atom, haloalkyl, and $C_{3-6}$ cycloalkyl; preferably a hydrogen atom;
or, $R^1$ and $R^2$, together with the carbon atom to which they are attached, form $C_{3-6}$ cycloalkyl;
$R^5$, $R^6$, or $R^7$ is identical or different and is each independently a hydrogen atom or alkyl;
$s^1$ is an integer from 2 to 8;
m is an integer from 0 to 4.

In some embodiments, the present disclosure provides a ligand-exatecan (or a derivative thereof) conjugate, wherein the ligand-exatecan (or the derivative thereof) conjugate comprises a structure represented by formula (-L-Y-):

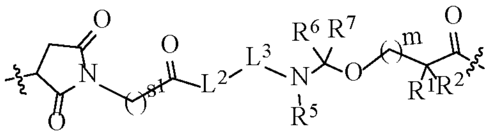

(-L-Y-)

wherein:

$L^2$ is a chemical bond;
$L^3$ is a tetrapeptide residue of GGFG;

$R^1$ is a hydrogen atom, cycloalkylalkyl, or cycloalkyl, preferably $C_{3-6}$ cycloalkylalkyl or $C_{3-6}$ cycloalkyl;
$R^2$ is selected from the group consisting of a hydrogen atom, haloalkyl, and $C_{3-6}$ cycloalkyl; preferably a hydrogen atom;
or, $R^1$ and $R^2$, together with the carbon atom to which they are attached, form $C_{3-6}$ cycloalkyl;
$R^5$ is selected from the group consisting of a hydrogen atom and alkyl; $R^6$ and $R^7$ are identical or different and are each independently a hydrogen atom or alkyl;
$s^1$ is an integer from 2 to 8;
m is an integer from 0 to 4.

In some embodiments, the present disclosure provides a ligand-exatecan (or a derivative thereof) conjugate, which is a ligand-exatecan (or a derivative thereof) conjugate represented by general formula ($Pc-L_a-Y-Dr$) of formula (IV):

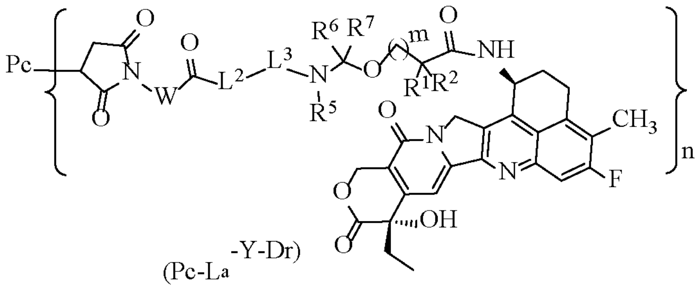

($Pc-L_a-Y-Dr$)

formula (IV)

wherein:

W is selected from the group consisting of $C_{1-8}$ alkyl, $C_{1-8}$ alkyl-cycloalkyl, and linear heteroalkyl of 1 to 8 atoms, the heteroalkyl comprising 1 to 3 heteroatoms selected from the group consisting of N, O, and S, wherein the $C_{1-8}$ alkyl, cycloalkyl, and linear heteroalkyl are each independently optionally further substituted with one or more substituents selected from the group consisting of halogen, hydroxy, cyano, amino, alkyl, chloroalkyl, deuterated alkyl, alkoxy, and cycloalkyl;
$L^2$ is selected from the group consisting of $-NR^4(CH_2CH_2O)p^1CH_2CH_2C(O)-$, $-NR^4(CH_2CH_2O)p^1CH_2C(O)-$, $-S(CH_2)p^1C(O)-$, and a chemical bond, and $p^1$ is an integer from 1 to 20;
$L^3$ is a peptide residue consisting of 2 to 7 amino acids, wherein the amino acids are optionally further substituted with one or more substituents selected from the group consisting of halogen, hydroxy, cyano, amino, alkyl, chloroalkyl, deuterated alkyl, alkoxy, and cycloalkyl;
$R^1$ is selected from the group consisting of a hydrogen atom, halogen, cycloalkylalkyl, deuterated alkyl, cycloalkyl, heterocyclyl, aryl, and heteroaryl;
$R^2$ is selected from the group consisting of a hydrogen atom, halogen, haloalkyl, deuterated alkyl, cycloalkyl, heterocyclyl, aryl, and heteroaryl;
or, $R^1$ and $R^2$, together with the carbon atom to which they are attached, form cycloalkyl or heterocyclyl;
$R^4$ and $R^5$ are identical or different and are each independently selected from the group consisting of a hydrogen atom, alkyl, haloalkyl, deuterated alkyl, and hydroxyalkyl;
$R^6$ and $R^7$ are identical or different and are each independently selected from the group consisting of a hydrogen atom, halogen, alkyl, haloalkyl, deuterated alkyl, and hydroxyalkyl;
m is an integer from 0 to 4;
n is 1 to 10 and may be an integer or decimal;
Pc is the anti-CD79b antibody or the antigen-binding fragment thereof provided by the present disclosure.

In some embodiments, the present disclosure provides a ligand-exatecan (or a derivative thereof) conjugate, which is a ligand-exatecan (or a derivative thereof) conjugate represented by general formula ($Pc-L_b-Y-Dr$) of formula (V):

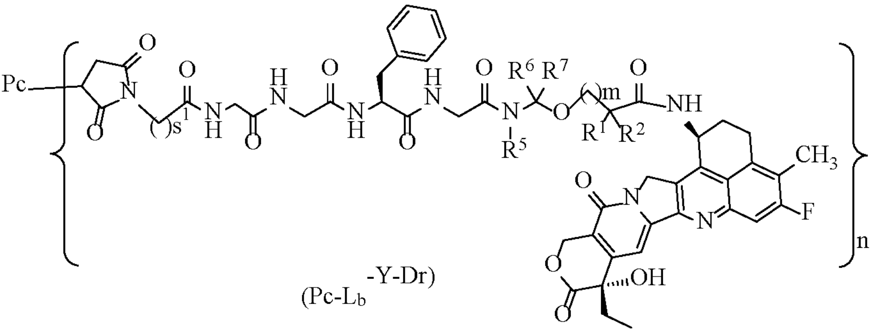

(Pc-$L_b$-Y-Dr)

formula (V)

wherein:

$s^1$ is an integer from 2 to 8; preferably 5;
Pc, $R^1$, $R^2$, $R^5$-$R^7$, m, and n are as defined in formula (IV).

In some embodiments, the linker unit -L-Y- of the ligand-exatecan (or the derivative thereof) conjugate of the present disclosure includes, but is not limited to:

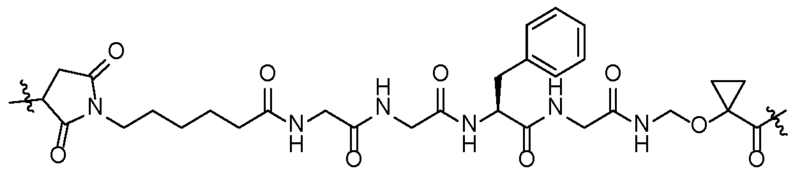

,

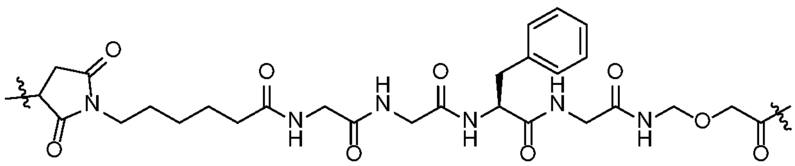

,

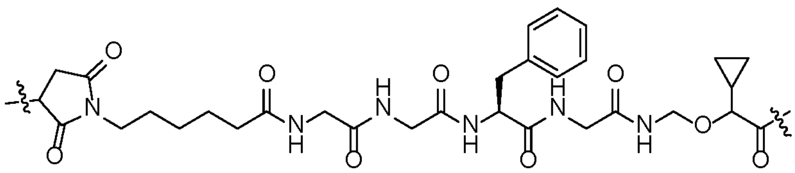

,

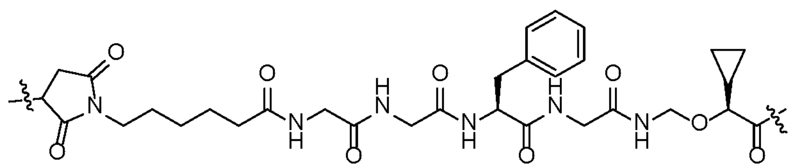

,

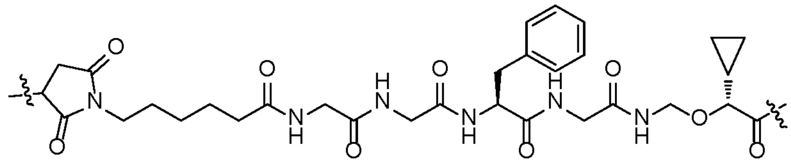

,

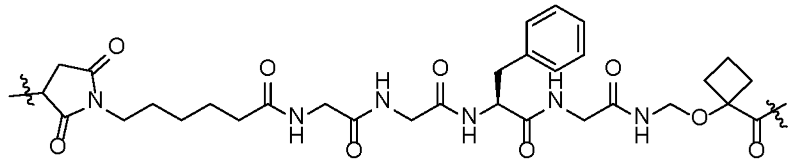

,

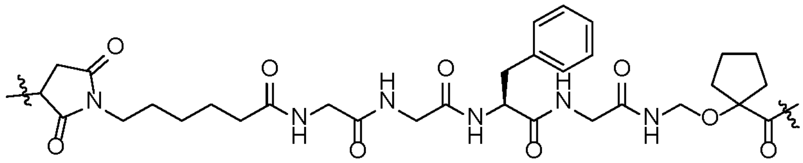

,

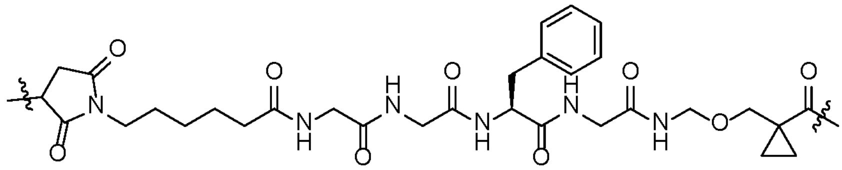

,

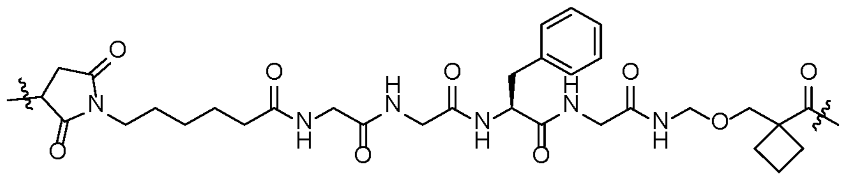

,

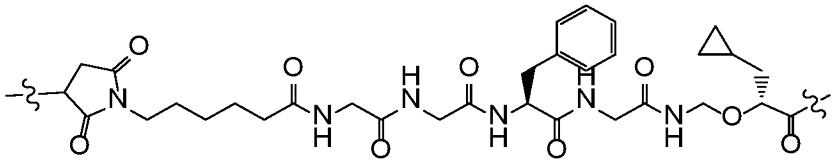

,

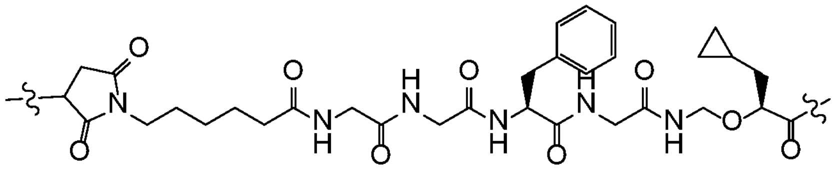

,

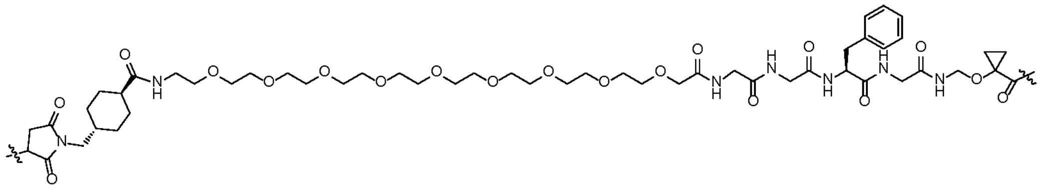

,

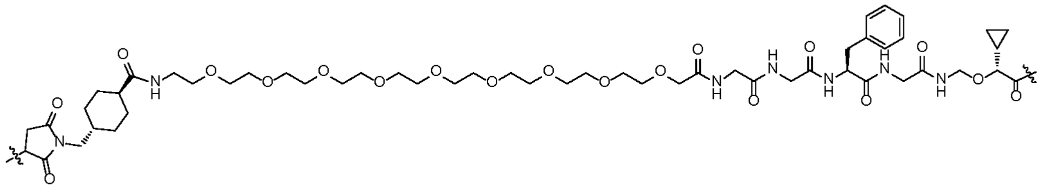

,

and

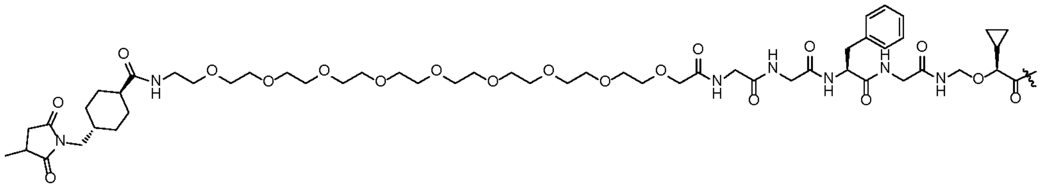

In some embodiments, the present disclosure provides a ligand-exatecan (or a derivative thereof) conjugate, including, but not limited to:

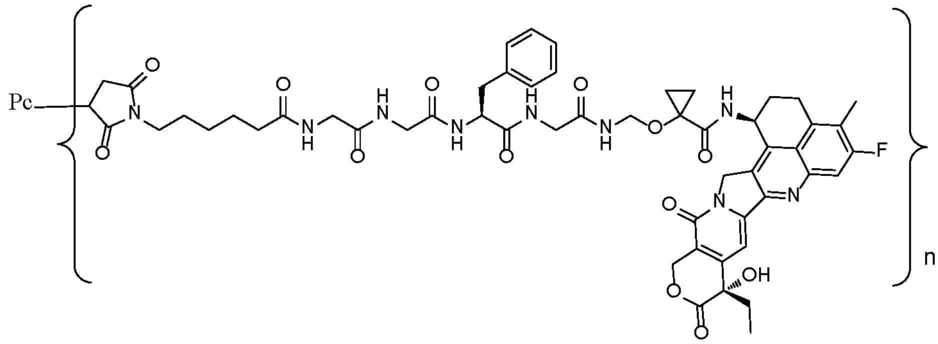

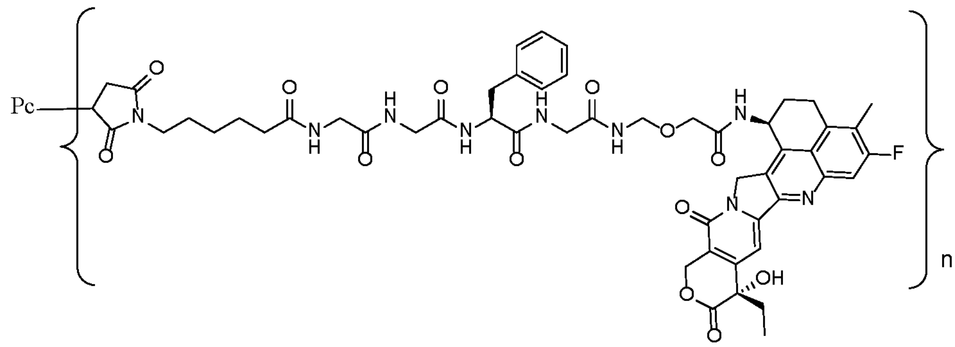

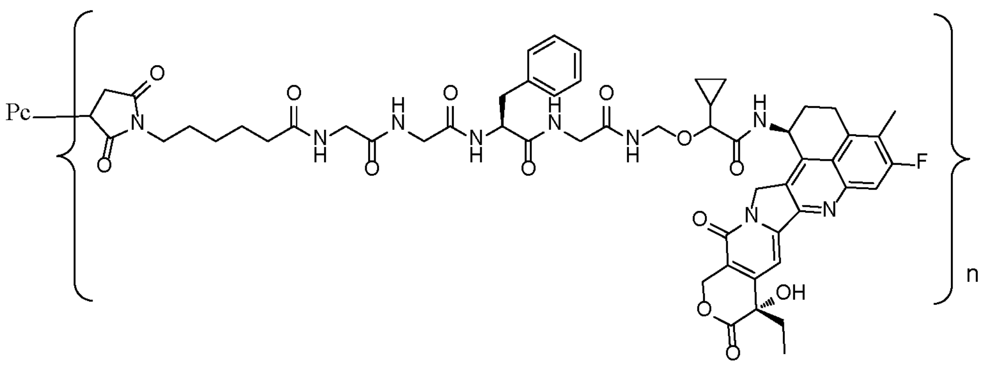

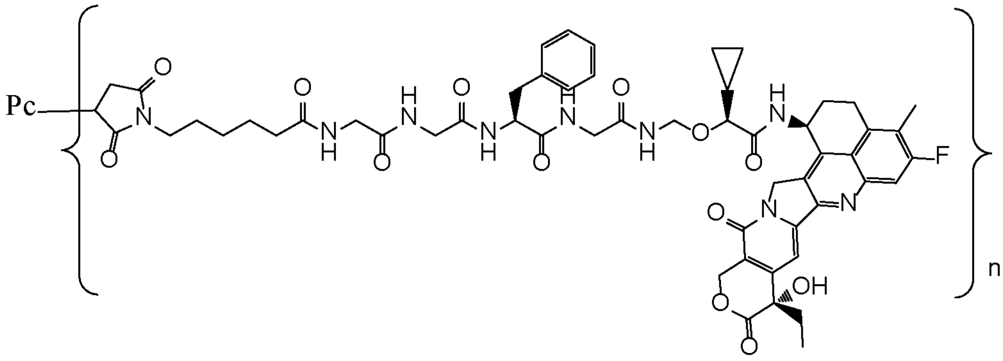

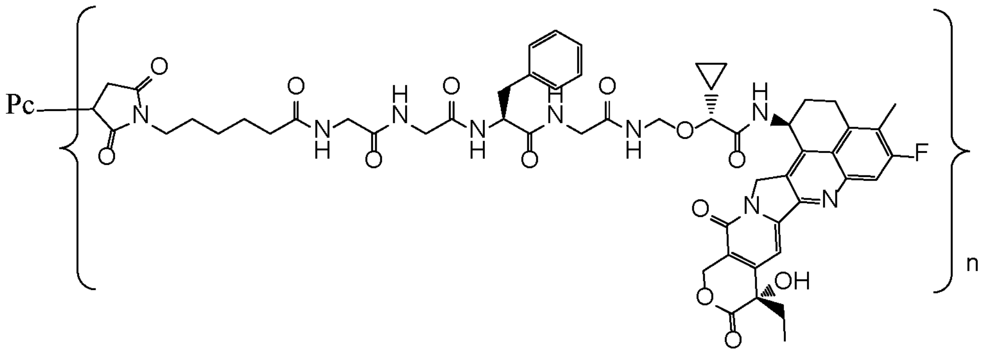

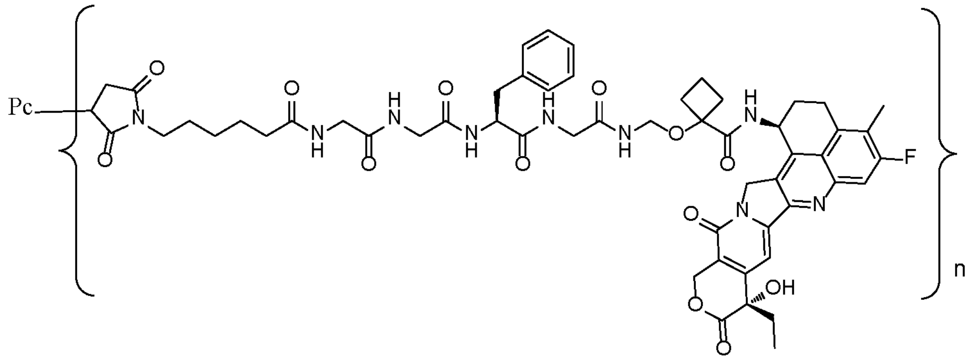

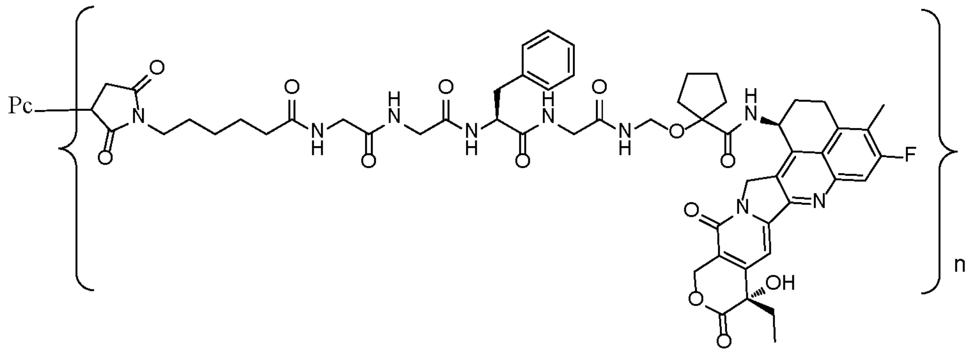

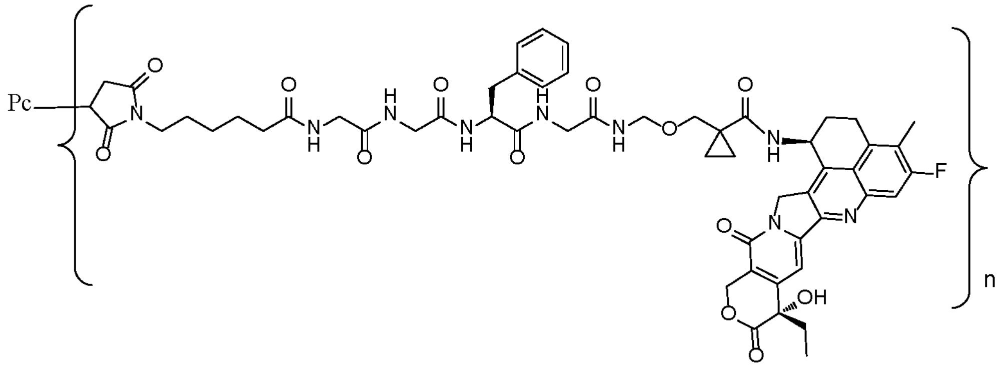

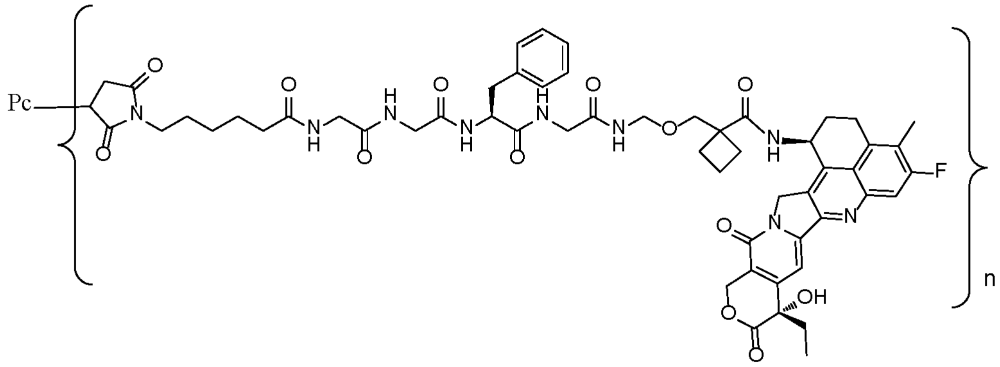

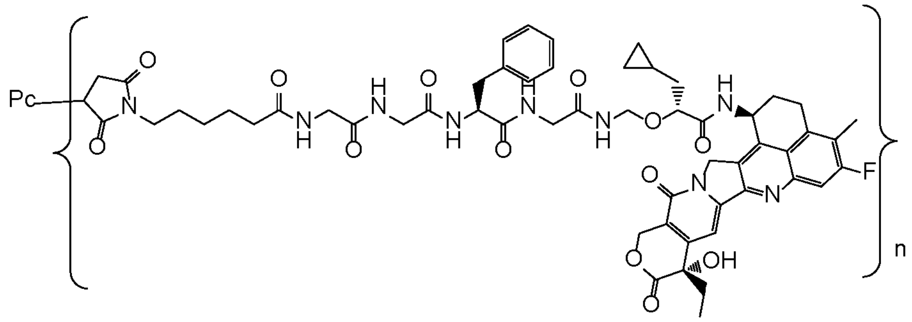

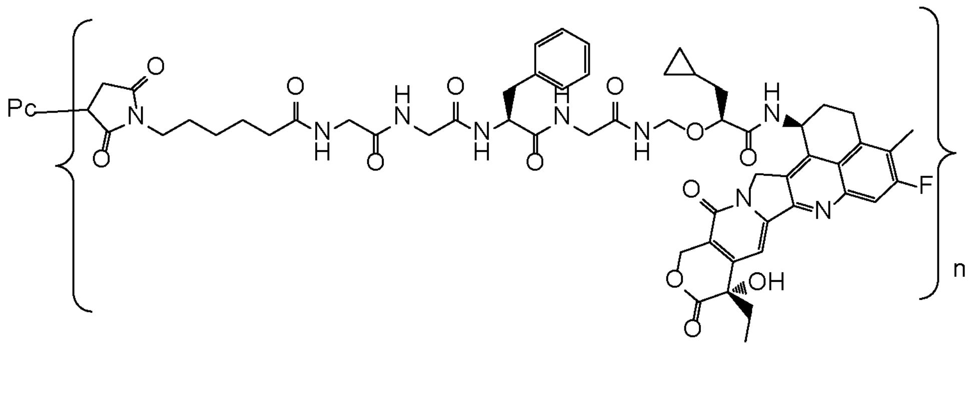

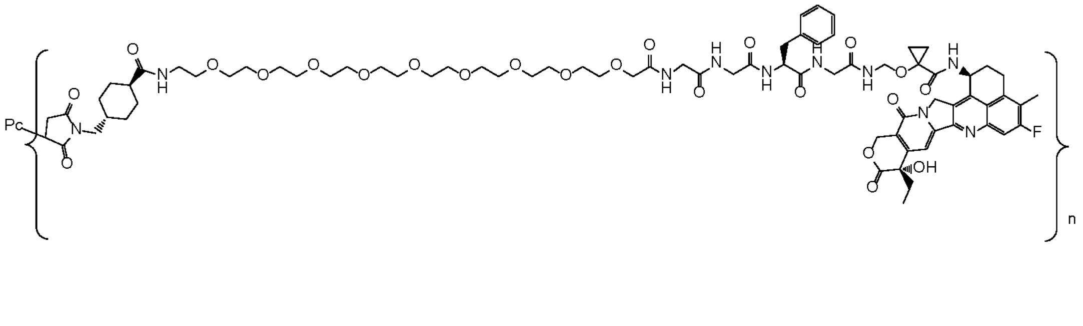

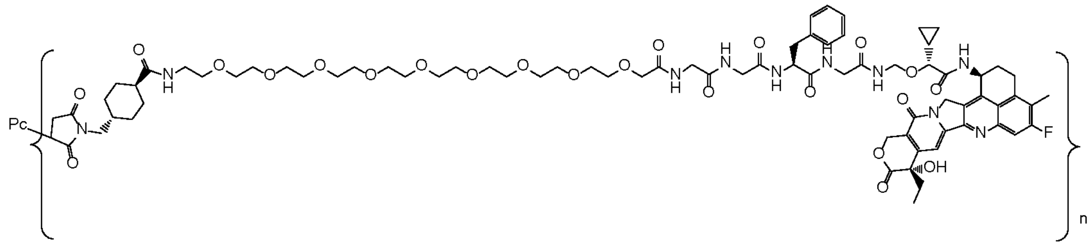

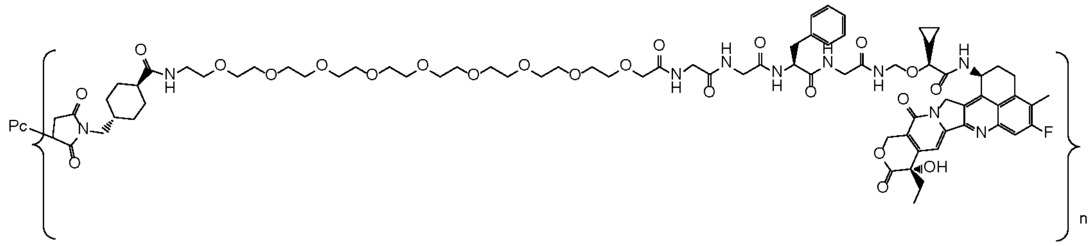

wherein:

n is 1 to 10 and may be an integer or decimal;
Pc is the aforementioned anti-CD79b antibody or antigen-binding fragment thereof of the present disclosure.

In some embodiments, a method for preparing a ligand-exatecan (or a derivative thereof) conjugate represented by general formula (Pc-$L_a$-Y-D) comprises the following step:

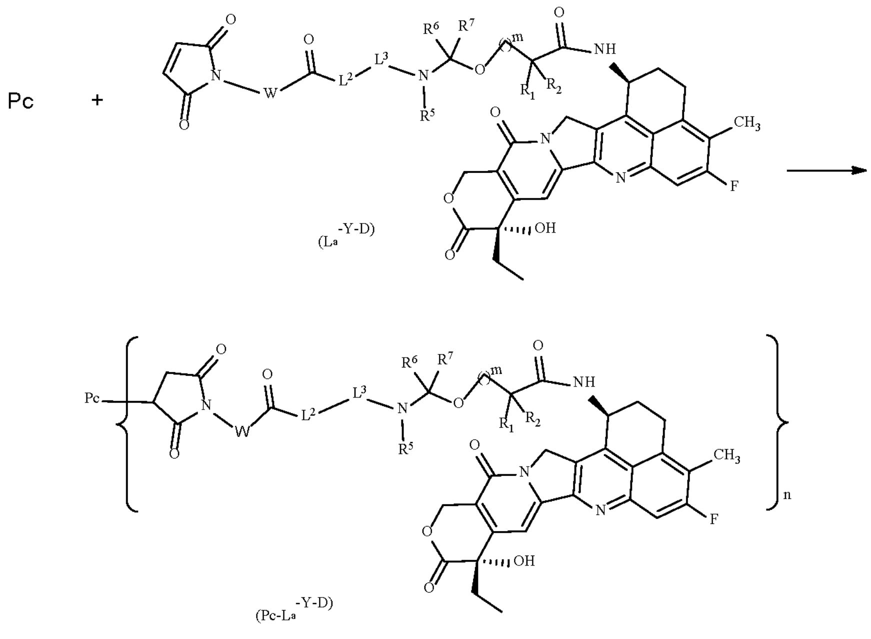

(La-Y-D)

(Pc-La-Y-D)

conducting a coupling reaction of reduced Pc with general formula ($L_a$-Y-D) to give a compound represented by general formula (Pc-$L_a$-Y-D);
wherein Pc is the anti-CD79b antibody or the antigen-binding fragment thereof of the present disclosure; W, $L^2$, $L^3$, $R^1$, $R^2$, $R^5$-$R^7$, m, and n are as defined in formula (IV).

In the above embodiments, Pc is any of the anti-CD79b antibodies or the antigen-binding fragments thereof of the present disclosure; preferably an anti-CD79b antibody or an antigen-binding fragment thereof from the examples; and more preferably an antibody comprising a heavy chain set forth in SEQ ID NO: 12 and a light chain set forth in SEQ ID NO: 13.

In some specific embodiments, the ligand-exatecan (or the derivative thereof) conjugate of the present

disclosure includes a tautomer, mesomer, racemate, enantiomer, diastereomer, or deuterated form thereof or a mixture thereof.

The compound and the preparation method therefor in WO2020063673 are incorporated herein in their entirety.

Ligand-Drug (MMAE or Derivative Thereof) Conjugate

The present disclosure provides a novel MMAE analog/derivative, which is a compound represented by general formula (D(MMAE)):

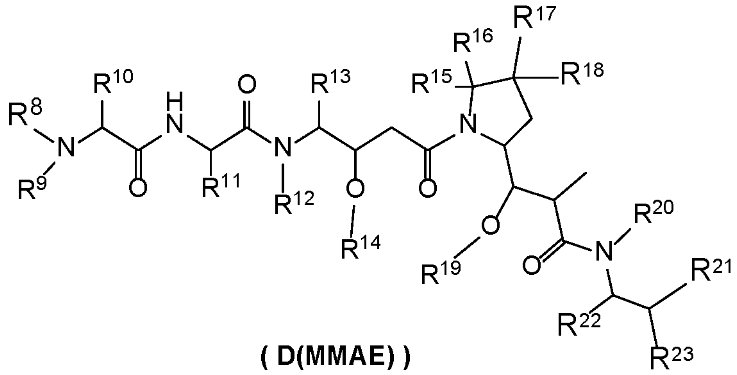

**( D(MMAE) )**

or a tautomer, mesomer, racemate, enantiomer, or diastereomer thereof or a mixture thereof, or a pharmaceutically acceptable salt thereof, wherein:

$R^8$-$R^{13}$ are selected from the group consisting of a hydrogen atom, halogen, hydroxy, cyano, alkyl, alkoxy, and cycloalkyl;
$R^{14}$ is selected from the group consisting of a hydrogen atom, alkyl, alkoxy, and cycloalkyl;
any two of $R^{15}$-$R^{18}$ form cycloalkyl, and the remaining two groups are selected from the group consisting of a hydrogen atom, alkyl, and cycloalkyl;
$R^{19}$ is selected from the group consisting of a hydrogen atom and alkyl;
$R^{20}$-$R^{22}$ are selected from the group consisting of a hydrogen atom, hydroxy, alkyl, alkoxy, and halogen;
$R^{23}$ is selected from the group consisting of aryl and heteroaryl, wherein the aryl and heteroaryl are optionally further substituted with a substituent selected from the group consisting of a hydrogen atom, halogen, hydroxy, alkyl, alkoxy, and cycloalkyl.

In some embodiments of the present disclosure, the compound represented by general formula (D(MMAE)) is a compound represented by general formula ($D(MMAE)_1$):

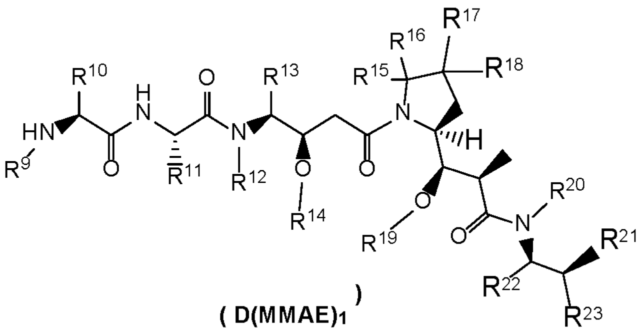

**( $D(MMAE)_1$ )**

or a tautomer, mesomer, racemate, enantiomer, or diastereomer thereof or a mixture thereof, or a pharmaceutically acceptable salt thereof;
$R^{16}$ and $R^{17}$ form cycloalkyl;
$R^9$-$R^{15}$ and $R^{18}$-$R^{23}$ are as defined in general formula (D).

In some embodiments of the present disclosure, the compound represented by general formula (D(MMAE)) is:

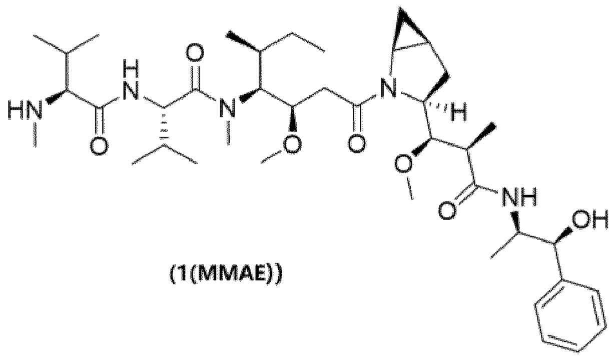

(1(MMAE))

Another aspect of the present disclosure relates to a ligand-drug conjugate or a pharmaceutically acceptable salt or solvate thereof, wherein the ligand-drug conjugate comprises a structure represented by formula (-D(MMAE)):

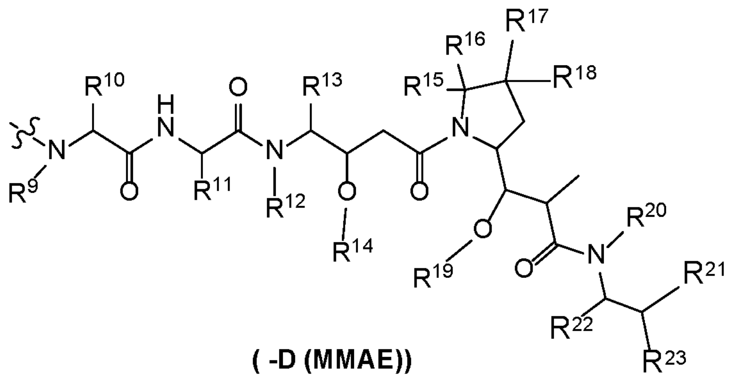

( -D (MMAE))

or a tautomer, mesomer, racemate, enantiomer, or diastereomer thereof or a mixture thereof, or a pharmaceutically acceptable salt thereof, wherein:

$R^9$-$R^{13}$ are selected from the group consisting of a hydrogen atom, halogen, hydroxy, cyano, alkyl, alkoxy, and cycloalkyl;
$R^{14}$ is selected from the group consisting of a hydrogen atom, alkyl, alkoxy, and cycloalkyl;
any two of $R^{15}$-$R^{18}$ form cycloalkyl, and the remaining two groups are selected from the group consisting of a hydrogen atom, alkyl, and cycloalkyl;
$R^{19}$ is selected from the group consisting of a hydrogen atom and alkyl;
$R^{20}$-$R^{22}$ are selected from the group consisting of a hydrogen atom, hydroxy, alkyl, alkoxy, and halogen;
$R^{23}$ is selected from the group consisting of aryl and heteroaryl, wherein the aryl and heteroaryl are optionally further substituted with a substituent selected from the group consisting of a hydrogen atom, halogen, hydroxy, alkyl, alkoxy, and cycloalkyl;
the wavy line indicates a hydrogen atom, or covalent attachment to a linker unit or to an antibody that binds to an antigen expressed by the target cell.

In some embodiments of the present disclosure, the ligand-MMAE (or the derivative thereof) conjugate or the pharmaceutically acceptable salt or solvate thereof is provided, wherein the ligand-drug conjugate comprises a structure represented by formula (-D(MMAE)$_1$):

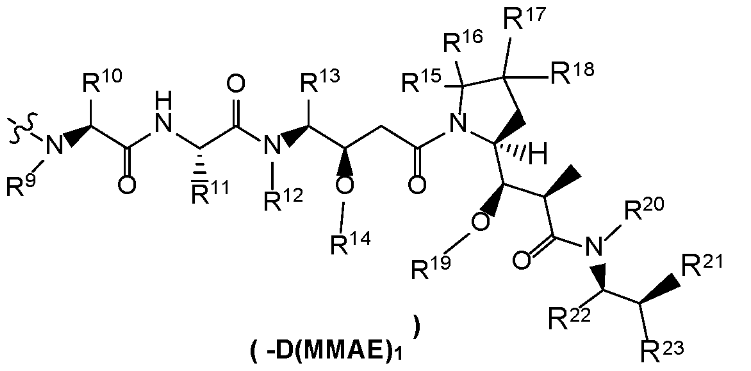

( -D(MMAE)$_1$ )

wherein:

$R^{16}$ and $R^{17}$ form cycloalkyl;
the wavy line, $R^9$-$R^{15}$, and $R^{18}$-$R^{23}$ are as defined in general formula (D(MMAE)).

In some embodiments of the present disclosure, the ligand-MMAE (or the derivative thereof) conjugate or the pharmaceutically acceptable salt or solvate thereof is provided, wherein the ligand-MMAE (or the derivative thereof) conjugate comprises a structure represented by the following formula:

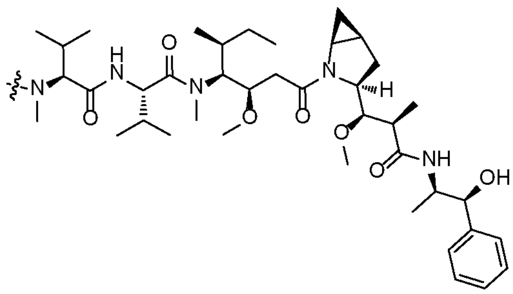

the wavy line indicates a hydrogen atom, or covalent attachment to a linker unit or to an antibody that binds to an antigen expressed by the target cell.

In some embodiments of the present disclosure, the ligand-MMAE (or the derivative thereof) conjugate or the pharmaceutically acceptable salt or solvate thereof is a ligand-drug conjugate represented by general formula (Pc-L-D(MMAE)) or a pharmaceutically acceptable salt or solvate thereof:

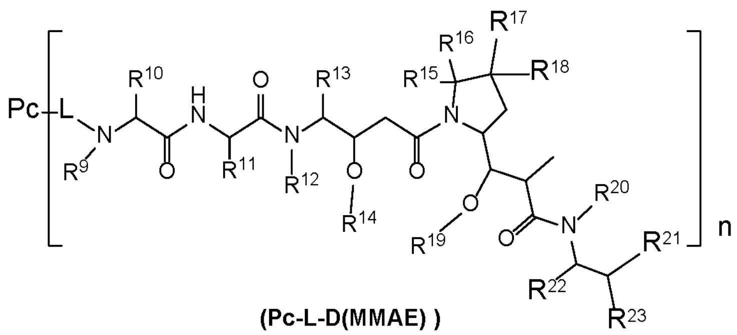

(Pc-L-D(MMAE) )

wherein:

$R^9$-$R^{23}$ are as defined in general formula (D(MMAE));
n is 1 to 10 and may be an integer or decimal;
Pc is the anti-CD79B antibody or the antigen-binding fragment thereof of the present disclosure; L is a linker unit.

In some embodiments of the present disclosure, the ligand-MMAE (or the derivative thereof) conjugate or the pharmaceutically acceptable salt or solvate thereof is a ligand-MMAE (or a derivative thereof) conjugate represented by

general formula (Pc-L-D1) or a pharmaceutically acceptable salt or solvate thereof

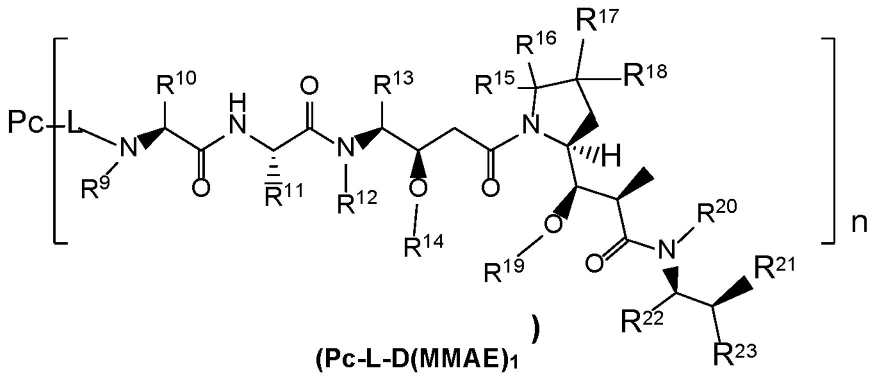

**(Pc-L-D(MMAE)$_1$)**

wherein:

$R^9$-$R^{23}$ are as defined in general formula (-D(MMAE));
Pc, L, and n are as defined in general formula (Pc-L-D(MMAE)).

In some embodiments of the present disclosure, the ligand-MMAE (or the derivative thereof) conjugate or the pharmaceutically acceptable salt or solvate thereof is a ligand-MMAE (or a derivative thereof) conjugate represented by the following general formula or a pharmaceutically acceptable salt or solvate thereof:

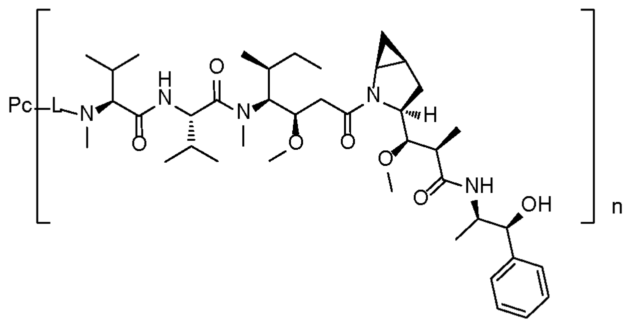

,

Pc, L, and n are as defined in general formula (Pc-L-D(MMAE)).

In some embodiments of the present disclosure, the ligand-MMAE (or the derivative thereof) conjugate or the pharmaceutically acceptable salt or solvate thereof is provided, wherein n is 1 to 8 and may be an integer or decimal; preferably, n is 1 to 6 and may be an integer or decimal.

In some embodiments of the present disclosure, the ligand-MMAE (or the derivative thereof) conjugate or the pharmaceutically acceptable salt or solvate thereof is provided, wherein the linker unit -L- is -Y-$L^5$-$L^6$-$L^7$-$L^8$;

Y is a stretcher unit selected from the group consisting of

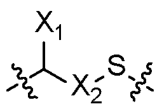

and a chemical bond, wherein $X_1$ is selected from the group consisting of a hydrogen atom, alkyl, alkoxy, aryl, and halogen, and $X_2$ is selected from the group consisting of alkylene, the alkylene being optionally further substituted with one or more substituents selected from the group consisting of halogen, hydroxy, cyano, amino, alkyl, chloroalkyl, deuterated alkyl, alkoxy, and cycloalkyl;
$L^5$ is a stretcher unit selected from the group consisting of -(succinimid-3-yl-*N*)-W-C(O)-, -$CH_2$-C(O)-$NR^{24}$-W-C(O)-, and -C(O)-W-C(O)-, wherein W is selected from the group consisting of $C_{1-8}$ alkyl, $C_{1-8}$ alkyl-cycloalkyl, and linear heteroalkyl of 1 to 8 atoms, and the heteroalkyl comprises 1 to 3 heteroatoms selected from the group consisting of N, O, and S, wherein the $C_{1-8}$ alkyl, cycloalkyl, and linear heteroalkyl are each independently optionally further substituted with one or more substituents selected from the group consisting of halogen, hydroxy, cyano, amino,

alkyl, chloroalkyl, deuterated alkyl, alkoxy, and cycloalkyl;

$L^6$ is selected from the group consisting of $-NR^{25}(CH_2CH_2O)p^1CH_2CH_2C(O)-$, - $NR^{25}(CH_2CH_2O)p^1CH_2C(O)-$, $-S(CH_2)p^1C(O)-$, and a chemical bond, wherein $p^1$ is an integer from 1 to 20; preferably, $L^6$ is a chemical bond;

$L^7$ is a peptide residue consisting of 2 to 7 amino acids preferably selected from the group consisting of valine, citrulline, and methyl valine; wherein the amino acids are optionally further substituted with one or more substituents selected from the group consisting of halogen, hydroxy, cyano, amino, alkyl, chloroalkyl, deuterated alkyl, alkoxy, and cycloalkyl;

$R^{24}$ and $R^{25}$ are identical or different and are each independently selected from the group consisting of a hydrogen atom, alkyl, haloalkyl, deuterated alkyl, and hydroxyalkyl;

$L^8$ is an extension unit, preferably PAB.

In some embodiments of the present disclosure, the ligand-MMAE (or the derivative thereof) conjugate or the pharmaceutically acceptable salt or solvate thereof is provided, wherein Y is selected from the group consisting of

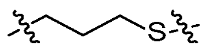

In some embodiments of the present disclosure, the ligand-MMAE (or the derivative thereof) conjugate or the pharmaceutically acceptable salt or solvate thereof is provided, wherein $L^5$ is selected from the group consisting of -(succinimid-3-yl-*N*)-$(CH_2)s^1$-C(O)-, wherein $s^1$ is an integer from 2 to 8; preferably, $L^5$ is

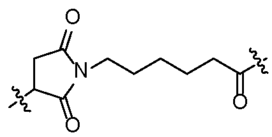

In some embodiments of the present disclosure, the ligand-MMAE (or the derivative thereof) conjugate or the pharmaceutically acceptable salt or solvate thereof is provided, wherein $L^7$ is a dipeptide amino acid unit, preferably selected from the group consisting of valine-citrulline.

In some embodiments of the present disclosure, the ligand-MMAE (or the derivative thereof) conjugate or the pharmaceutically acceptable salt or solvate thereof is provided, wherein the linker unit -L- is selected from the group consisting of:

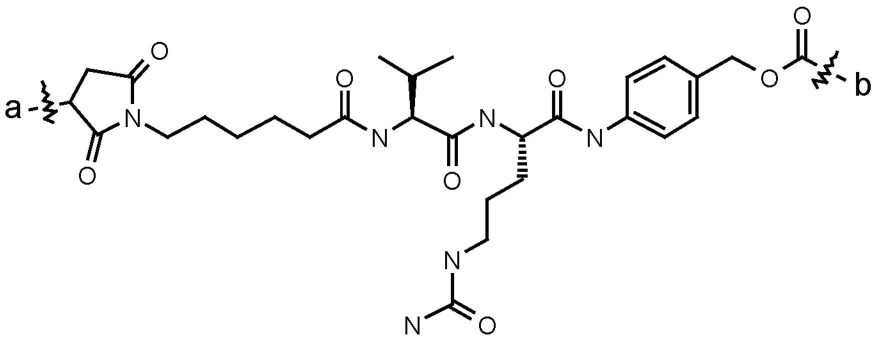

and

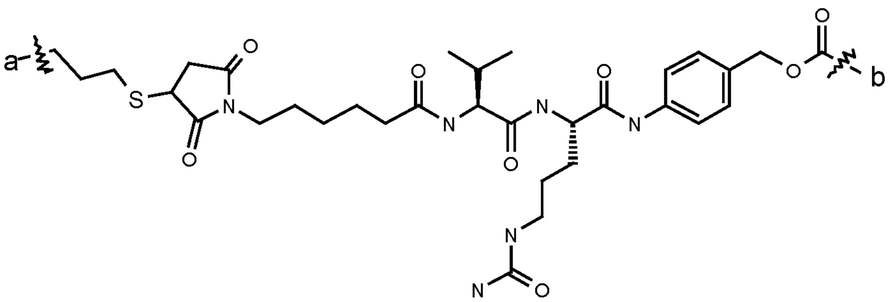

wherein the a-terminus is attached to a ligand, and the b-terminus is attached to a drug.

In some embodiments of the present disclosure, the ligand-MMAE (or the derivative thereof) conjugate or the pharmaceutically acceptable salt or solvate thereof is selected from the group consisting of the following formulas:

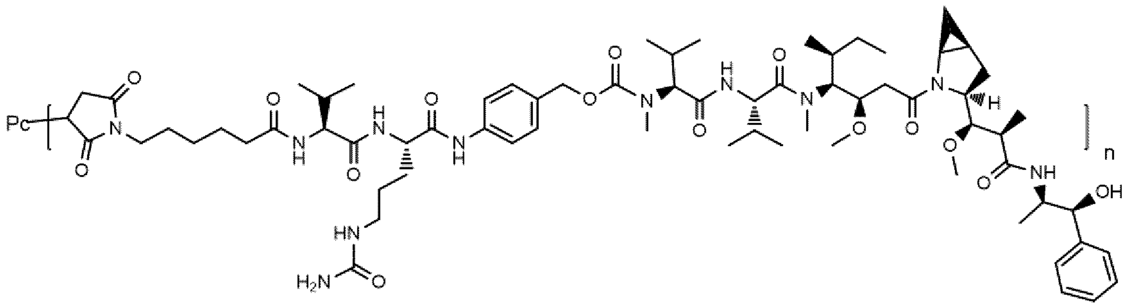

and

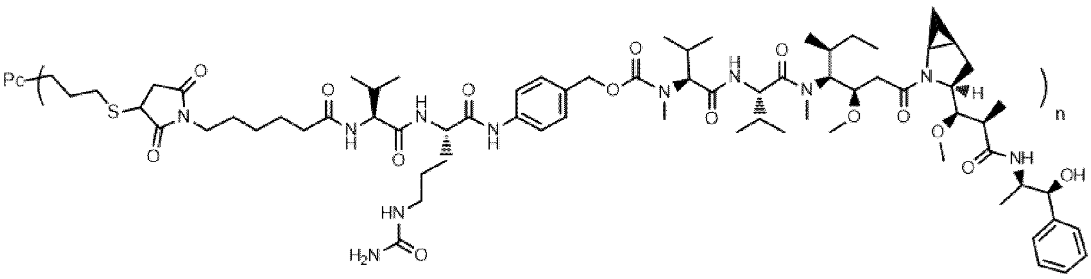

,

wherein:

n is 1 to 10 and may be an integer or decimal;

Pc is the anti-CD79B antibody or the antigen-binding fragment thereof of the present disclosure; preferably an anti-CD79B antibody or an antigen-binding fragment thereof from the examples; and more preferably an antibody comprising a heavy chain set forth in SEQ ID NO: 12 and a light chain set forth in SEQ ID NO: 13.

Another aspect of the present disclosure relates to a method for preparing a compound represented by general formula (D(MMAE)) or a tautomer, mesomer, racemate, enantiomer, or diastereomer thereof or a mixture thereof, or a pharmaceutically acceptable salt thereof, comprising the following step:

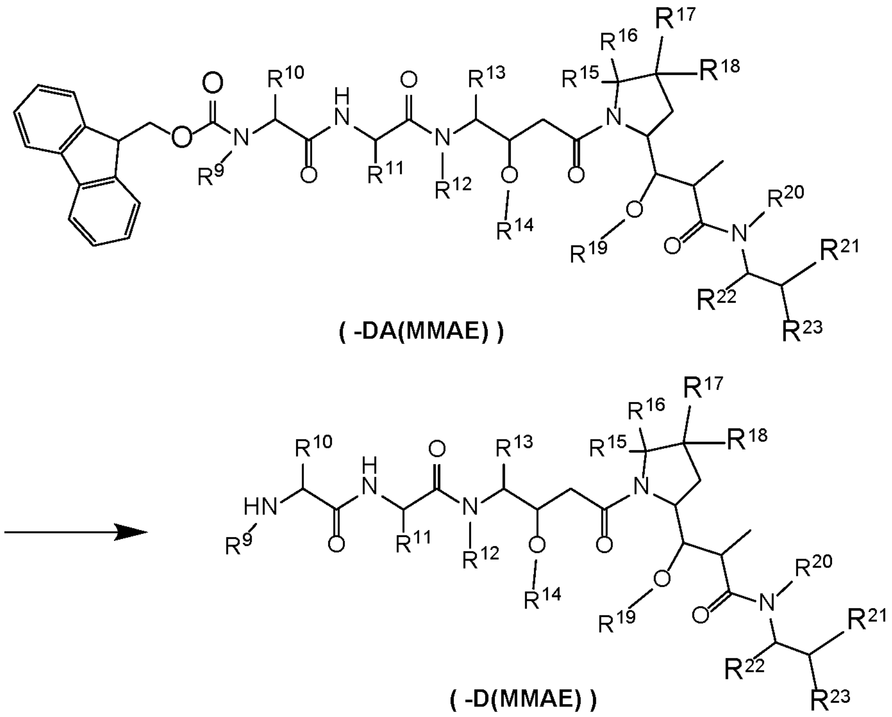

( -DA(MMAE) )

( -D(MMAE) )

conducting a deprotection reaction of general formula (DA(MMAE)) to give the compound represented by general formula (D(MMAE)),

wherein: $R^9$-$R^{23}$ are as defined in general formula (D).

Another aspect of the present disclosure relates to a compound as shown below:

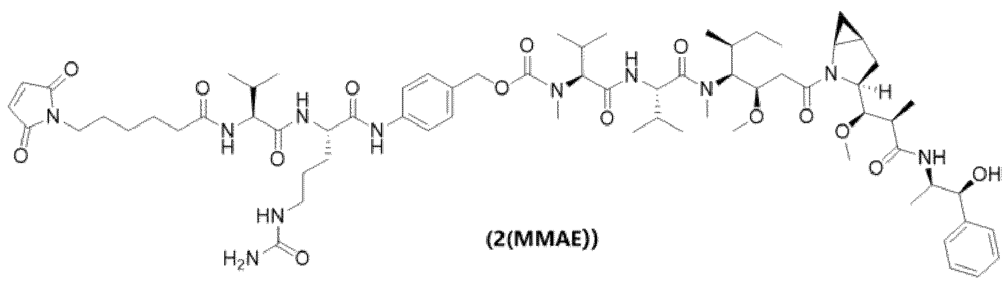

(2(MMAE))

or a tautomer, mesomer, racemate, enantiomer, or diastereomer thereof or a mixture thereof, or a pharmaceutically acceptable salt thereof, which can be used as an intermediate for preparing the ligand-drug conjugate of the present disclosure.

Another aspect of the present disclosure relates to a method for preparing compound 2(MMAE) or a tautomer, mesomer, racemate, enantiomer, or diastereomer thereof or a mixture thereof, or a pharmaceutically acceptable salt thereof, comprising the following step:

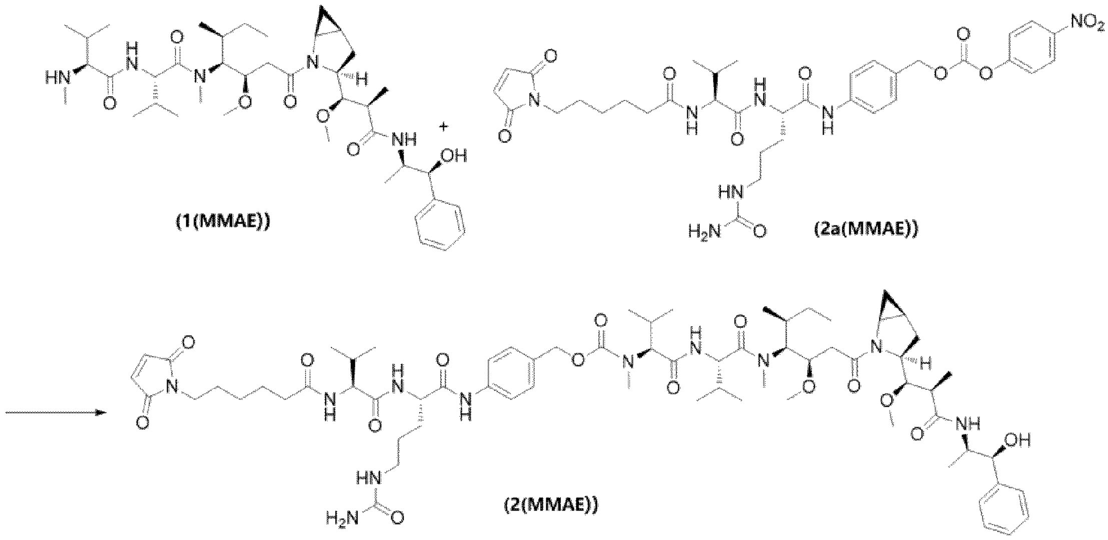

(1(MMAE)) (2a(MMAE)) (2(MMAE))

conducting a condensation reaction of compound 1(MMAE)with compound 2a(MMAE) to give compound 2(MMAE).

Another aspect of the present disclosure relates to a method for preparing a ligand-drug conjugate represented by general formula (Pc-L-D(MMAE)) or a pharmaceutically acceptable salt or solvate thereof, comprising the following step:

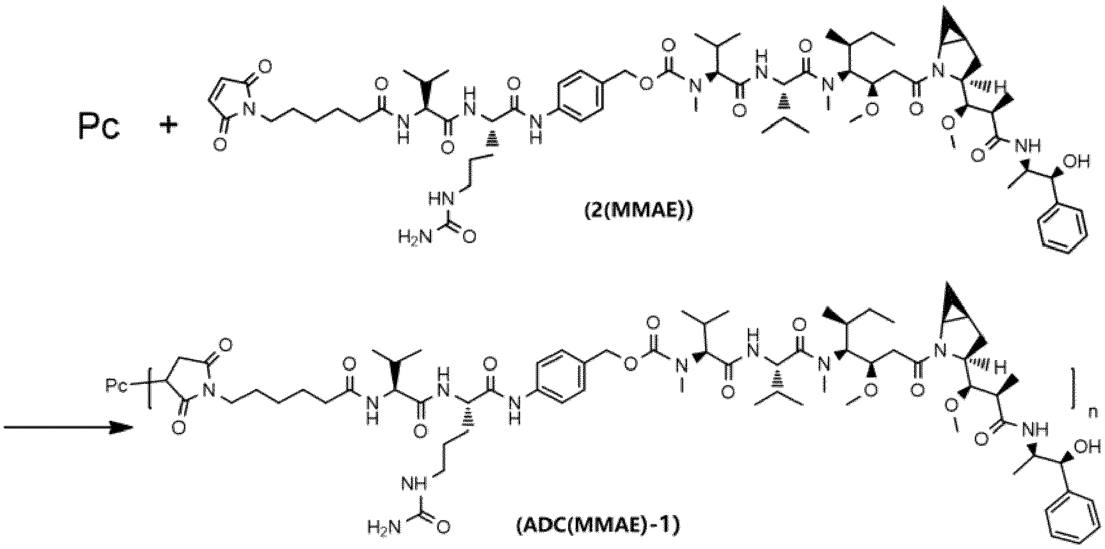

(2(MMAE)) (ADC(MMAE)-1)

conducting a coupling reaction of reduced Pc with a compound to give a compound represented by general formula (ADC(MMAE)-1);
wherein Pc and n are as defined in general formula (Pc-L-D(MMAE)).

In order to achieve the purpose of synthesizing the MMAE and the derivative thereof of the present disclosure, the present disclosure uses the following technical solutions of synthesis:

Solution 1:

A method for the compound represented by general formula (D(MMAE)) or the tautomer, mesomer, racemate, enantiomer, or diastereomer thereof or the mixture thereof, or the pharmaceutically acceptable salt thereof of the present disclosure, comprising the following step:

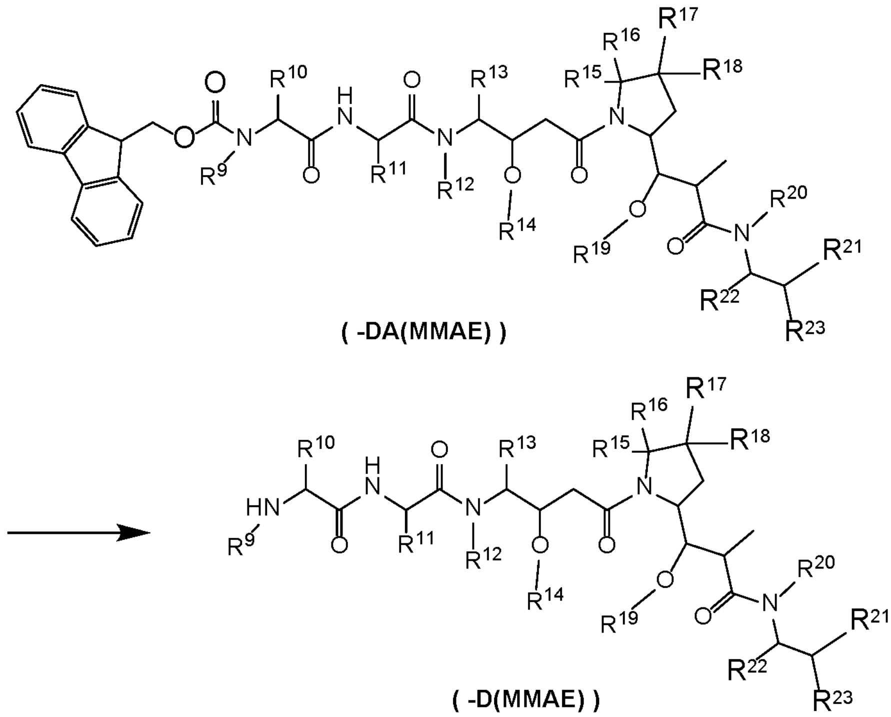

( -DA(MMAE) )

( -D(MMAE) )

conducting a deprotection reaction of general formula (DA(MMAE)) under alkaline conditions to give the compound represented by general formula (D(MMAE)),
wherein: $R^9$-$R^{23}$ are as defined in general formula (D(MMAE)).

Reagents that provide the alkaline conditions include organic and inorganic bases, wherein the organic bases include, but are not limited to, triethylamine, diethylamine, *N*-methylmorpholine, pyridine, piperidine, N,N-diisopropylethylamine, n-butyllithium, lithium diisopropylamide, potassium acetate, sodium tert-butoxide or potassium tert-butoxide, and the inorganic bases include, but are not limited to, sodium hydride, potassium phosphate, sodium carbonate, potassium carbonate, cesium carbonate, sodium hydroxide, and lithium hydroxide; preferably diethylamine.

<u>Solution 2:</u>

A method for preparing compound 2(MMAE) or the pharmaceutically acceptable salt or solvate thereof of the present disclosure, comprising:

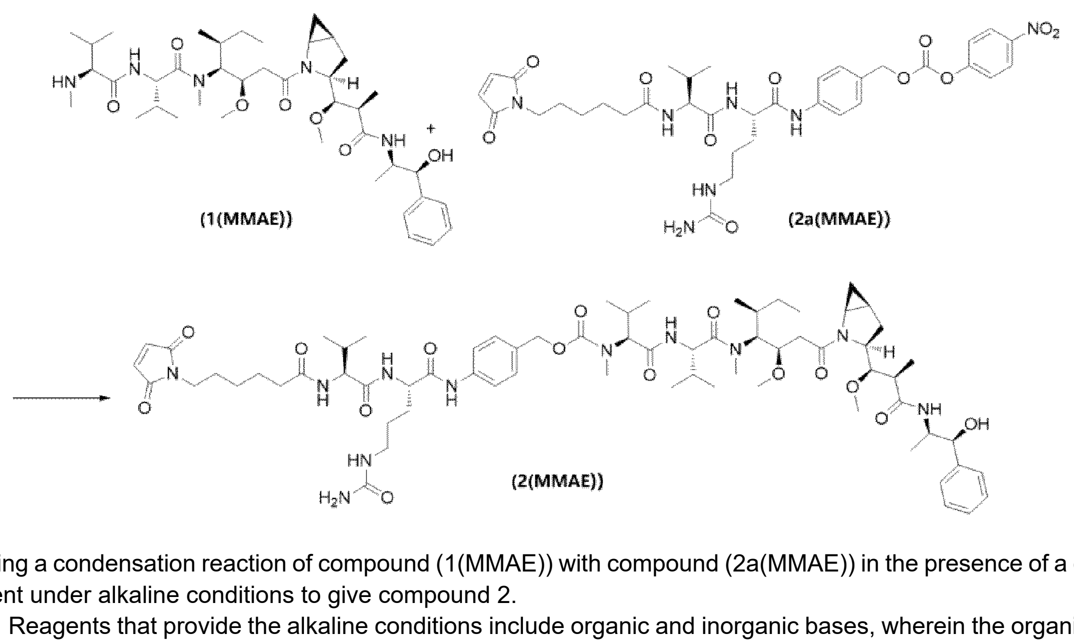

(1(MMAE))

(2a(MMAE))

(2(MMAE))

conducting a condensation reaction of compound (1(MMAE)) with compound (2a(MMAE)) in the presence of a condensing agent under alkaline conditions to give compound 2.

Reagents that provide the alkaline conditions include organic and inorganic bases, wherein the organic bases

include, but are not limited to, triethylamine, diethylamine, N-methylmorpholine, pyridine, piperidine, N,N-diisopropylethylamine, n-butyllithium, lithium diisopropylamide, potassium acetate, sodium tert-butoxide or potassium tert-butoxide, and the inorganic bases include, but are not limited to, sodium hydride, potassium phosphate, sodium carbonate, potassium carbonate, cesium carbonate, sodium hydroxide, and lithium hydroxide, preferably N,N-diisopropylethylamine. The condensing agent is selected from the group consisting of 4-(4,6-dimethoxy-1,3,5-triazin-2-yl)-4-methylmorpholinium chloride, 1-hydroxybenzotriazole, 1-(3-dimethylaminopropyl)-3-ethylcarbodiimide hydrochloride, N,N'-dicyclohexylcarbodiimide, N,N'-diisopropylcarbodiimide, O-benzotriazole-N,N,N',N'-tetramethyluronium tetrafluoroborate, 1-hydroxybenzotriazole, 1-hydroxy-7-azobenzotriazole, O-benzotriazole-N,N,N',N'-tetramethyluronium hexafluorophosphate, 2-(7-azobenzotriazole)-N,N,N',N'-tetramethyluronium hexafluorophosphate, benzotriazol-1-yloxytris(dimethylamino)phosphonium hexafluorophosphate, and benzotriazol-1-yl-oxytripyrrolidinophosphonium hexafluorophosphate, preferably 4-(4,6-dimethoxy-1,3,5-triazin-2-yl)-4-methylmorpholinium chloride or 1-hydroxybenzotriazole or 1-(3-dimethylaminopropyl)-3-ethylcarbodiimide hydrochloride, preferably 1-hydroxybenzotriazole.

Solution 3:

A method for preparing the ligand-drug conjugate represented by general formula (Pc-L-D) or the pharmaceutically acceptable salt or solvate thereof of the present disclosure, comprising the following step:

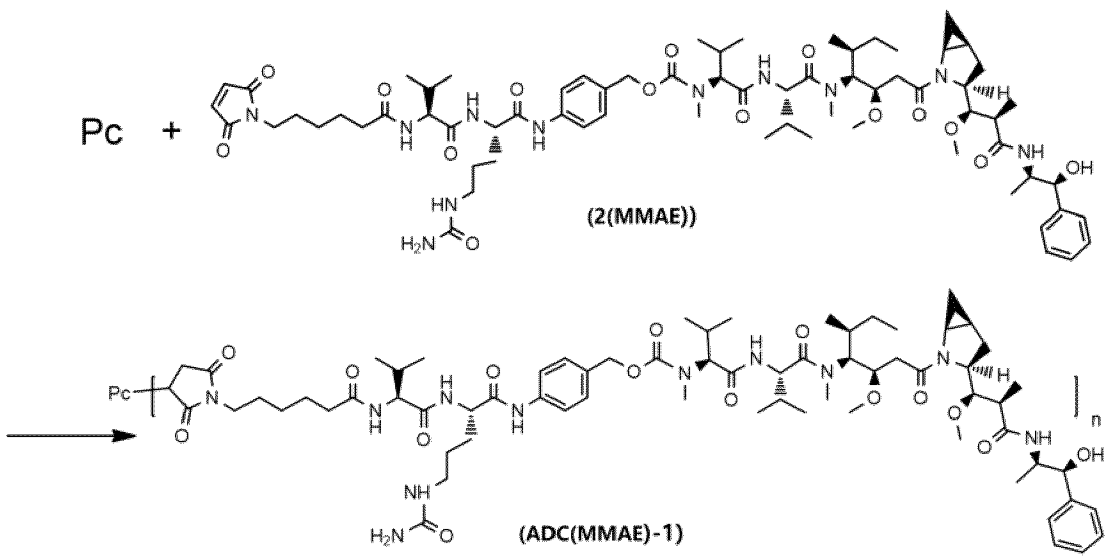

(2(MMAE))

(ADC(MMAE)-1)

conducting a coupling reaction of reduced Pc with compound 2(MMAE) to give a compound represented by general formula (ADC(MMAE)-1); wherein the reductant is preferably TCEP; particularly, disulfide bonds on the antibody are preferably reduced;
wherein Pc and n are as defined in general formula (Pc-L-D(MMAE)).

Example Compounds

The present disclosure provides a ligand-drug conjugate, selected from the group consisting of:

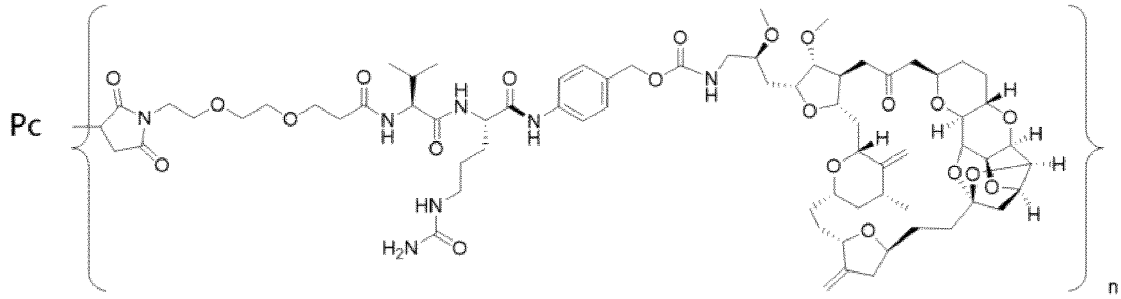

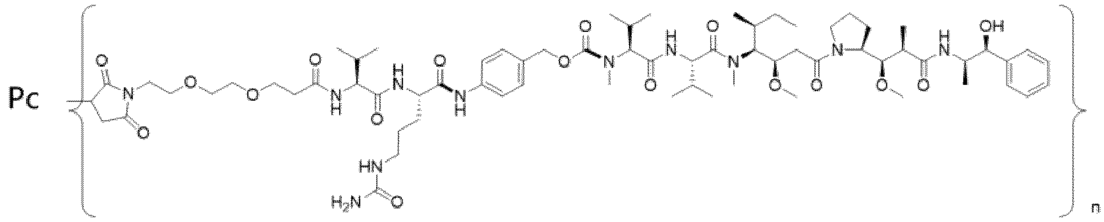

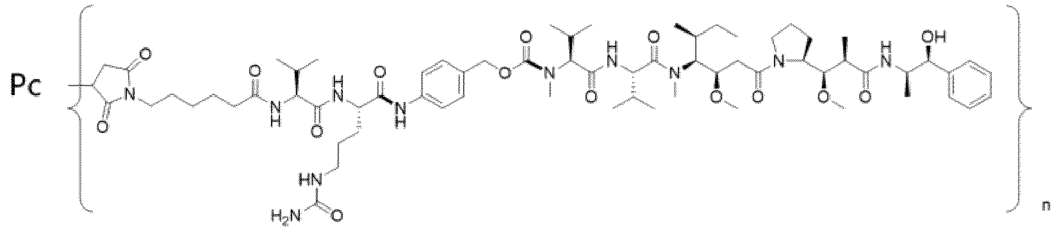

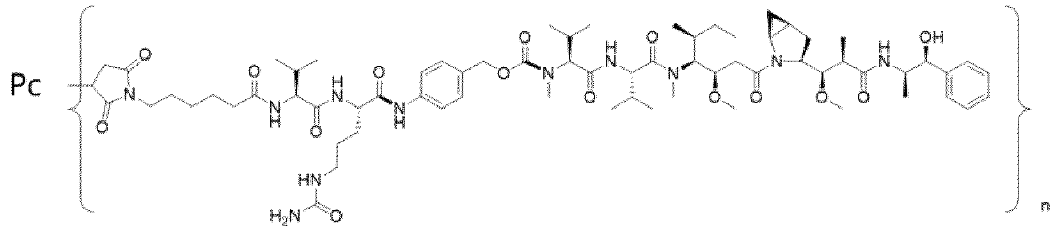

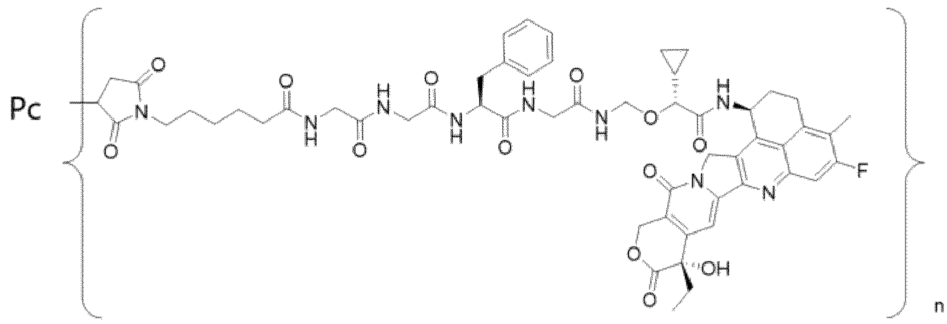

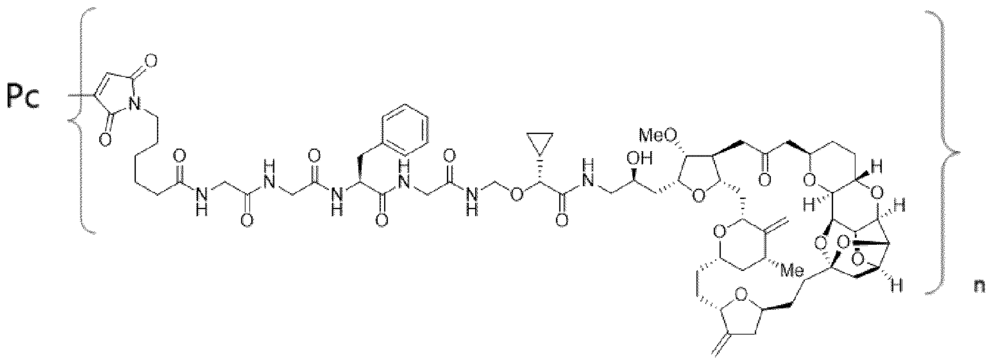

wherein Pc is any of the anti-CD79b antibodies or the antigen-binding fragments thereof of the present disclosure, and n is 1 to 10 and may be an integer or decimal.

In some specific embodiments, Pc is an anti-CD79b antibody or an antigen-binding fragment thereof from the examples of the present disclosure, e.g., an antibody comprising a heavy chain set forth in SEQ ID NO: 12 and a light chain set forth in SEQ ID NO: 13, and n is an integer or decimal between 1 and 6.

In some specific embodiments, the antibody-drug conjugate of the present disclosure may have a mean DAR value of 1-10, e.g., any value between 2-8, or 2-6, or 1-6, or 4-6. In some embodiments, the DAR is between about 1 and about 6, e.g., about 1, 1.5, 2, 2.5, 3, 3.5, 4, 4.5, 5, 5.5, 6, 6.5, 7.0, 7.5, or 8.0. In some embodiments, at least 50% by weight of

the sample is a compound having a mean DAR ± 2; in some specific embodiments, at least 50% by weight of the sample is a conjugate having a mean DAR ± 1. For example, immunoconjugates in which the DAR is about 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, 5.0, 5.1, 5.2, 5.4, 5.4, 5.5, 5.6, 5.7, 5.8, 5.9, 6.0, 6.1, 6.2, 6.3, 6.4, 6.5, 6.6, 6.7, 6.8, 6.9, or 7.0 are included. In some embodiments, a DAR of 'about x' means that the DAR measurement is within 20% of x.

In some embodiments, the pharmaceutical composition according to any one of the foregoing may be an agent for intravenous, subcutaneous, intraperitoneal, or intramuscular injection.

In some embodiments, the present disclosure provides a method for preparing the pharmaceutical composition according to any one of the foregoing, the method comprising the step of subjecting a stock solution of a ligand-drug conjugate (e.g., an anti-CD79b antibody-drug conjugate) to buffer exchange.

The present disclosure also provides a liquid formulation, comprising the pharmaceutical composition according to any one of the foregoing.

The pharmaceutical composition of the present disclosure already has sufficient stability for being prepared into a drug and can be stored stable for a long time.

The present disclosure also provides a method for preparing a lyophilized formulation of a pharmaceutical composition comprising a ligand-drug conjugate (e.g., an anti-CD79b antibody-drug conjugate), the method comprising the step of lyophilizing the aforementioned pharmaceutical composition.

The present disclosure also provides a lyophilized formulation of a pharmaceutical composition comprising a ligand-drug conjugate (e.g., an anti-CD79b antibody-drug conjugate), wherein the lyophilized formulation is obtained by lyophilizing any one of the aforementioned pharmaceutical compositions.

In some embodiments, the lyophilized formulation is stored in a dark place at 2-8 °C and is stable for at least 1 month, at least 3 months, at least 6 months, at least 12 months, at least 18 months, at least 24 months, or at least 30 months.

In some embodiments, the lyophilized formulation is stable at 25 °C for at least 1 month, at least 3 months, at least 6 months, or at least 12 months.

In some embodiments, the lyophilized formulation is stable at 40 °C for at least 7 days, at least 14 days, or at least 30 days.

The present disclosure also provides a reconstituted solution comprising a ligand-drug conjugate (e.g., the aforementioned anti-CD79b antibody-drug conjugate of the present disclosure) pharmaceutical composition, wherein the reconstituted solution is prepared by reconstituting the lyophilized formulation according to any one of the foregoing.

The present disclosure further provides a product, comprising a container containing the pharmaceutical composition, liquid formulation, lyophilized formulation, or reconstituted solution according to any one of the above. In some embodiments, the container may be, but is not limited to, a tubular injection vial made of neutral borosilicate glass.

The present disclosure also provides use of the pharmaceutical composition according to any one of the above, the liquid formulation according to any one of the above, the lyophilized formulation according to any one of the above, the reconstituted solution according to any one of the above, or the product according to any one of the above in the preparation of a medicament for treating or preventing a proliferative disease or delaying the progression of a proliferative disease.

The present disclosure also provides use of the pharmaceutical composition according to any one of the above, the liquid formulation according to any one of the above, the lyophilized formulation according to any one of the above, the reconstituted solution according to any one of the above, or the product according to any one of the above for treating or preventing a proliferative disease or delaying the progression of a proliferative disease.

In some embodiments, the proliferative disease according to any one of the above may be a cancer or tumor; the cancer or tumor is selected from the group consisting of lymphoma, diffuse large B-cell lymphoma, non-Hodgkin's lymphoma (NHL), aggressive NHL, recurrent and aggressive NHL, recurrent and indolent NHL, refractory NHL, refractory and indolent NHL, chronic lymphocytic leukemia (CLL), small lymphocytic lymphoma, leukemia, hairy cell leukemia (HCL), acute lymphocytic leukemia (ALL), and/or mantle cell lymphoma.

The present disclosure provides a method for treating or preventing a proliferative disease or delaying the progression of a proliferative disease, comprising administering to a subject in need thereof a therapeutically or prophylactically effective amount of the pharmaceutical composition according to any one of the above, the liquid formulation according to any one of the above, the lyophilized formulation according to any one of the above, the reconstituted solution according to any one of the above, or the product according to any one of the above, wherein: the proliferative disease may be a cancer or tumor; the cancer or tumor is selected from the group consisting of lymphoma, diffuse large B-cell lymphoma, non-Hodgkin's lymphoma (NHL), aggressive NHL, recurrent and aggressive NHL, recurrent and indolent NHL, refractory NHL, refractory and indolent NHL, chronic lymphocytic leukemia (CLL), small lymphocytic lymphoma, leukemia, hairy cell leukemia (HCL), acute lymphocytic leukemia (ALL), and/or mantle cell lymphoma.

The present disclosure provides a method for enhancing immune function in a subject having a B-cell

proliferative disorder or autoimmune disorder, comprising administering to a subject in need thereof an amount, effective in treating or delaying the disease, of the pharmaceutical composition according to any one of the above, the liquid formulation according to any one of the above, the lyophilized formulation according to any one of the above, the reconstituted solution according to any one of the above, or the product according to any one of the above, wherein: the B-cell proliferative disorder is a cancer or tumor; the B-cell proliferative disorder is lymphoma, non-Hodgkin's lymphoma (NHL), aggressive NHL, recurrent and aggressive NHL, recurrent and indolent NHL, refractory NHL, refractory and indolent NHL, chronic lymphocytic leukemia (CLL), small lymphocytic lymphoma, leukemia, hairy cell leukemia (HCL), acute lymphocytic leukemia (ALL), and/or mantle cell lymphoma.

## BRIEF DESCRIPTION OF THE DRAWINGS

FIG. 1: A-C shows the efficacy of different ADCs against human diffuse large B-cell lymphoma WSU-DLCL2 nude mouse subcutaneous xenograft tumors.
FIG. 2: a graph showing the effects of different ADCs on the body weight of tumor-bearing nude mice.
FIG. 3: the efficacy of different ADCs against human diffuse large B-cell lymphoma WSU-DLCL2 nude mouse subcutaneous xenograft tumors.
FIG. 4: the effects of different ADCs on the body weight of WSU-DLCL2 tumor-bearing nude mice.
FIG. 5: photos of tumors showing the efficacy of different ADCs against human diffuse large B-cell lymphoma WSU-DLCL2 nude mouse subcutaneous xenograft tumors.
FIG. 6: the efficacy of different ADCs against human follicular lymphoma DOHH-2 nude mouse subcutaneous xenograft tumors.
FIG. 7: the effects of different ADCs on the body weight of DOHH-2 tumor-bearing nude mice.
FIG. 8: screening for a buffer system pH value for an ADC composition formulation, where FIGs. 8A and 8B show the trends of change in SEC and RCE, respectively, under different pH/buffer conditions.
FIG. 9: screening for an auxiliary material and a surfactant for an ADC composition, where FIGs. 9A and 9B show the trends of change in SEC and RCE, respectively, under different pH/buffer conditions.
FIG. 10: further screening for a pH for an ADC composition and screening for a polysorbate 80 concentration, where FIGs. 10A and 10B show the trends of change in SEC and RCE, respectively, under different pH/buffer conditions.

ADC-1, ADC-2, and ADC-4 are all other anti-CD79b antibody-drug conjugates obtained by screening in the present disclosure, and the specific structures are not shown.

## DETAILED DESCRIPTION

### Terminology

In order to facilitate the understanding of the present disclosure, some technical and scientific terms are specifically defined below. Unless otherwise specifically defined herein, all other technical and scientific terms used herein have the meanings generally understood by those of ordinary skill in the art to which the present disclosure pertains.
"Antibody-drug conjugates" (ADC) are formed by linking antibodies or antibody fragments and cytotoxins with biological activity by stable chemical linker compounds, so that the specificity of the antibodies to bind to tumor cell-specific or highly expressed antigens and the high efficiency of the cytotoxins are combined to prevent toxic and side effects on normal cells. Antibody-drug conjugates can bind to tumor cells precisely and reduce the impact on normal cells compared to conventional chemotherapeutic drugs.
"Buffer" refers to a buffer that resists changes in pH by the action of its acid-base conjugate components. Examples of buffers that maintain the pH within an appropriate range include acetate, succinate, gluconate, histidine, oxalate, lactate, phosphate, citrate, tartrate, fumarate, glycylglycine, and other organic acid buffers.
"Histidine salt buffer" is a buffer comprising histidine ions. Examples of histidine salt buffers include histidine-hydrochloride, histidine-acetate, histidine-phosphate, histidine-sulfate, etc. buffers; histidine-hydrochloride buffers are preferred. Histidine-hydrochloride buffers are prepared with histidine and hydrochloric acid, or histidine and histidine hydrochloride.
"Tris-citrate buffer" is a buffer that comprises citrate ions. Examples of Tris-citrate buffers include Tris-hydrochloride, Tris-acetate, Tris-phosphate, Tris-sulfate, Tris-citrate, etc. buffers; Tris-citrate is preferred.
"Tris-hydrochloride buffer" is a buffer that comprises hydrochloride ions. Examples of Tris-hydrochloride buffers include Tris-hydrochloride, Tris-acetate, Tris-phosphate, Tris-sulfate, Tris-citrate, etc. buffers. A preferred citrate buffer is a Tris-hydrochloride buffer.

"Phosphate buffer" is a buffer that comprises phosphate ions. Examples of phosphate buffers include disodium hydrogen phosphate-sodium dihydrogen phosphate, disodium hydrogen phosphate-potassium dihydrogen phosphate, and the like. A preferred phosphate buffer is a disodium hydrogen phosphate-sodium dihydrogen phosphate buffer.

"Acetate buffer" is a buffer that comprises acetate ions. Examples of acetate buffers include acetic acid-sodium acetate, acetic acid-histidine salt, acetic acid-potassium acetate, acetic acid-calcium acetate, acetic acid-magnesium acetate, and the like. A preferred acetate buffer is an acetic acid-sodium acetate buffer.

"Succinate buffer" is a buffer that comprises succinate ions. Examples of succinate buffers include succinic acid-sodium succinate, succinic acid-potassium succinate, succinic acid-calcium succinate, and the like. A preferred succinate buffer is succinic acid-sodium succinate. Illustratively, the succinic acid-sodium succinate may be prepared with succinic acid and sodium hydroxide, or succinic acid and sodium succinate.

"Pharmaceutical composition" refers to a mixture containing one or more of the compounds described herein or physiologically/pharmaceutically acceptable salts or prodrugs thereof, and other chemical components, wherein the other components are, for example, physiologically/pharmaceutically acceptable carriers and excipients. The pharmaceutical composition is intended to promote administration to an organism and facilitate the absorption of the active ingredient so that it can exert its biological activity. As used herein, "pharmaceutical composition" and "formulation" are not mutually exclusive.

Unless otherwise stated, the solvent in the pharmaceutical composition described in the present disclosure in solution form is water.

The term "about" as used herein means that a numerical value is within an acceptable error range for the particular value determined by one of ordinary skill in the art, and the numerical value depends in part on how the value is measured or determined (i.e., the limits of the measurement system). For example, "about" may mean a standard deviation within 1 or more than 1 in each practice in the art. Or, "about" or "substantially comprising" may mean a range of up to 20%. Furthermore, particularly for biological systems or processes, the term may mean up to an order of magnitude or up to 5-fold of a numerical value. Unless otherwise specified, when a specific value is provided in the present application and claims, the meaning of "about" or "substantially comprising" should be assumed to be within an acceptable error range for that specific value.

The pharmaceutical composition of the present disclosure can achieve the effect of being stable: the antibody in the pharmaceutical composition substantially retains its physical and/or chemical stability and/or biological activity after storage; preferably, the pharmaceutical composition substantially retains its physical and chemical stability as well as its biological activity after storage. The storage period is generally selected based on a predetermined shelf life of the pharmaceutical composition. There are a variety of analytical techniques currently available for measuring protein stability, and the stability after storage for a selected period of time at a selected temperature can be measured.

A stable pharmaceutical antibody formulation is one in which no significant change is observed under the following conditions: stored at refrigeration temperature (2-8 °C) for at least 3 months, preferably 6 months, more preferably 1 year, and even more preferably at most 2 years. In addition, stable liquid formulations include liquid formulations that exhibit desirable features after storage at 25 °C for periods including 1 month, 3 months, and 6 months, or storage at 40 °C for periods including 1 month. Typical acceptable criteria for stability are as follows: typically, no more than about 10%, preferably no more than about 5%, of antibody monomer is degraded as measured by SEC-HPLC. The drug antibody formulation is colorless or yellow, clear to slightly opalescent, by visual analysis. The concentration, pH, and osmolality of the formulation have a change of no more than ±10%. Typically, clippings of no more than about 10%, preferably no more than about 5%, are observed. Typically, aggregation of no more than about 10%, preferably no more than about 5%, is formed.

An antibody "retains its physical stability" in a pharmaceutical formulation if it shows no significant increase in aggregation, precipitation and/or denaturation upon visual inspection of color and/or clarity, or as determined by UV light scattering, size exclusion chromatography (SEC), and dynamic light scattering (DLS). Changes in protein conformation can be evaluated by fluorescence spectroscopy (which determines the protein tertiary structure) and by FTIR spectroscopy (which determines the protein secondary structure).

An antibody "retains its chemical stability" in a pharmaceutical formulation if it shows no significant chemical change. Chemical stability can be evaluated by detecting and quantifying chemically changed protein. Degradation processes that often change the chemical structure of proteins include hydrolysis or clipping (assessed by methods such as size exclusion chromatography and SDS-PAGE), oxidation (assessed by methods such as peptide mapping in combination with mass spectroscopy or MALDI/TOF/MS), deamidation (assessed by methods such as ion-exchange chromatography, capillary isoelectric focusing, peptide mapping, and isoaspartic acid determination), and isomerization (assessed by isoaspartic acid content determination, peptide mapping, etc.).

An antibody "retains its biological activity" in a pharmaceutical formulation if the biological activity of the antibody at a given time is within a predetermined range of the biological activity exhibited during the preparation of the pharmaceutical formulation. The biological activity of an antibody can be determined, for example, by an antigen-binding assay.

The engineered antibody or antigen-binding fragment of the present disclosure can be prepared and purified by conventional methods. For example, cDNA sequences encoding the heavy and light chains can be cloned and recombined into a GS expression vector. Recombinant immunoglobulin expression vectors can be stably transfected into CHO cells. As a more recommended prior art, mammalian expression systems will result in glycosylation of antibodies, particularly at the highly conserved N-terminal site of the Fc region. Stable clones are obtained by expression of the antibody that specifically binds to human FXI. Positive clones are expanded in a serum-free medium of a bioreactor to produce antibodies. The culture medium with the secreted antibody can be purified by conventional techniques. For example, purification is performed using an A or G Sepharose FF column containing an adjusted buffer. Non-specifically bound fractions are washed away. The bound antibody is eluted by the pH gradient method, and the antibody fragments are detected by SDS-PAGE and collected. The antibody can be filtered and concentrated by conventional methods. Soluble mixtures and polymers can also be removed by conventional methods, such as molecular sieves and ion exchange. The resulting product needs to be immediately frozen, e.g., at -70 °C, or lyophilized.

The three-letter and single-letter codes for amino acids used in the present application are as described in J. Biol. Chem, 243, p3558 (1968).

The term "antibody" described in the present application is used in the broadest sense and encompasses a variety of antibody structures. Illustratively, an antibody refers to an immunoglobulin, which is a four-peptide-chain structure formed by connecting two identical heavy chains and two identical light chains by interchain disulfide bonds. The heavy chain constant regions of an immunoglobulin differ in their amino acid composition and arrangement, and thus in their antigenicity. Accordingly, immunoglobulins can be divided into five classes, or isotypes of immunoglobulins, namely IgM, IgD, IgG, IgA, and IgE, with their corresponding heavy chains being μ chain, δ chain, γ chain, α chain, and ε chain, respectively. Ig of the same class can be divided into different subclasses according to differences in the amino acid composition of the hinge regions and the number and positions of disulfide bonds of the heavy chains; for example, IgG can be divided into IgG1, IgG2, IgG3, and IgG4. Light chains are divided into κ or λ chains according to differences in the constant regions. Each of the five classes of Ig may have a κ chain or λ chain.

In the present application, the antibody light chain variable region described in the present application may further comprise a light chain constant region comprising human or murine κ and λ chains or variants thereof.

In the present application, the antibody heavy chain variable region described in the present application may further comprise a heavy chain constant region comprising human or murine IgG1, 2, 3, or 4 or variants thereof.

In the antibody heavy and light chains, the sequences of about 110 amino acids near the N-terminus vary considerably and thus are referred to as variable regions (V regions); the remaining amino acid sequences near the C-terminus are relatively stable and thus are referred to as constant regions (C regions). The variable regions comprise 3 hypervariable regions (HVRs) and 4 framework regions (FRs) with relatively conservative sequences. The 3 hypervariable regions determine the specificity of the antibody and thus are also known as complementarity determining regions (CDRs). Each of the light chain variable regions (VLs) and the heavy chain variable regions (VHs) consists of 3 CDRs and 4 FRs arranged from the amino-terminus to the carboxy-terminus in the following order: FR1, CDR1, FR2, CDR2, FR3, CDR3, and FR4. The 3 CDRs of the light chain refer to LCDR1, LCDR2, and LCDR3; and the 3 CDRs of the heavy chain refer to HCDR1, HCDR2, and HCDR3. The CDR amino acid residues of the VL and VH regions of the antibody or the antigen-binding fragment accord with the known Chothia (ABM) numbering scheme in terms of number and positions.

The term "recombinant human antibody" includes human antibodies prepared, expressed, created, or isolated by recombinant methods, involving techniques and methods well known in the art, such as:

(1) antibodies isolated from transgenic human immunoglobulin genes, transchromosomal animals (e.g., mice), or hybridomas prepared therefrom;
(2) antibodies isolated from host cells that have been transformed to express the antibodies, such as transfectomas;
(3) antibodies isolated from a recombinant combinatorial human antibody library; and
(4) antibodies prepared, expressed, created, or isolated by a method such as splicing human immunoglobulin gene sequences to other DNA sequences.

Such recombinant human antibodies comprise variable and constant regions that utilize specific human germline immunoglobulin sequences encoded by germline genes, and also include subsequent rearrangements and mutations which occur, for example, during antibody maturation.

The term "murine antibody" in the present application refers to a monoclonal antibody against human CD79b or an epitope thereof prepared according to the knowledge and skill in the art. During the preparation, a test subject is injected with a CD79b antigen, and then hybridoma of antibodies expressing the desired sequence or functional properties is isolated. In one specific embodiment of the present application, the murine anti-human CD79b antibody or the antigen-binding fragment thereof may further comprise a light chain constant region of a murine κ or λ chain or a variant thereof, or further comprise a heavy chain constant region of a murine IgG1, IgG2, IgG3, or IgG4 or a variant thereof.

The term "human antibody" includes antibodies having variable and constant regions derived from human

germline immunoglobulin sequences. The human antibody of the present application may include amino acid residues not encoded by human germline immunoglobulin sequences (e.g., mutations introduced by random or site-specific mutagenesis *in vitro* or by somatic mutations *in vivo*). However, the term "human antibody" does not include antibodies in which CDR sequences derived from the germline of another mammalian species (such as mice) have been grafted into human framework sequences (i.e., "humanized antibody").

The term "humanized antibody", also known as a CDR-grafted antibody, refers to an antibody produced by grafting murine CDR sequences into the framework of variable regions of a human antibody. Such an antibody can overcome the strong immune response induced by the chimeric antibody because of carrying a large amount of mouse protein components. To avoid the decrease in activity caused by the decrease in immunogenicity, the variable regions of a human antibody can be subjected to minimum reverse mutation to maintain activity.

The term "chimeric antibody" refers to an antibody obtained by fusing a variable region of a murine antibody to a constant region of a human antibody, which can reduce an immune response induced by the murine antibody. The chimeric antibody is established by firstly establishing hybridoma secreting murine specific monoclonal antibody, then cloning a variable region gene from the mouse hybridoma cells, cloning a constant region gene of a human antibody as required, linking the mouse variable region gene and the human constant region gene into a chimeric gene, inserting the chimeric gene into a human vector, and finally expressing chimeric antibody molecules in a eukaryotic industrial system or prokaryotic industrial system. The constant region of the human antibody may be selected from the group consisting of the heavy chain constant regions of human IgG1, IgG2, IgG3, and IgG4 or variants thereof, preferably comprising heavy chain constant regions of human IgG2 or IgG4, or IgG1 mutated at amino acids without ADCC (antibody-dependent cell-mediated cytotoxicity) toxicity.

The "antigen-binding fragment" described in the present application refers to a Fab fragment, a Fab' fragment, and a F(ab')2 fragment that have antigen-binding activity, and an Fv fragment and an sFv fragment that binds to human CD79b. The Fv fragment comprises the heavy chain variable region and the light chain variable region of the antibody but does not comprise the constant region, and has the smallest antibody fragment of the entire antigen-binding sites. Generally, the Fv antibody also comprises a polypeptide linker between the VH and VL domains, and is capable of forming the structure required for antigen binding. Two antibody variable regions can also be linked into a single polypeptide chain using different linkers, known as single-chain antibody or single-chain Fv (sFv). The term "single-chain antibody", "single-chain Fv" or "scFv" refers to a molecule comprising an antibody heavy chain variable domain (or region; VH) and an antibody light chain variable domain (or region; VL) linked by a linker. Such scFv molecules may have a general structure: $NH_2$-VL-linker-VH-COOH or $NH_2$-VH-linker-VL-COOH. Suitable linkers in the prior art consist of repeated GGGGS amino acid sequences or variants thereof, for example, 1-4 repeated variants (Holliger et al. (1993), Proc. Natl. Acad. Sci. USA 90: 6444-6448). Other linkers that can be used in the present disclosure are described in Alfthan et al. (1995), Protein Eng. 8:725-731; Choi et al. (2001), Eur. J. Immunol. 31:94-106; Hu et al. (1996), Cancer Res. 56:3055-3061, Kipriyanov et al. (1999), J. Mol. Biol. 293:41-56; and Roovers et al. (2001), Cancer Immunol.

The term "CDR" refers to one of the 6 hypervariable regions within the variable domain of an antibody which primarily contribute to antigen binding. One of the most common definitions for the 6 CDRs is provided in Kabat E.A. et al., (1991) Sequences of proteins of immunological interest. NIH Publication 91-3242). As used herein, the Kabat definition for CDRs may be only applied to CDR1, CDR2, and CDR3 of the light chain variable domain (CDR L1, CDR L2, CDR L3 or L1, L2, L3), and CDR2 and CDR3 of the heavy chain variable domain (CDR H2, CDR H3 or H2, H3). Generally, there are three CDRs (HCDR1, HCDR2 and HCDR3) in each heavy chain variable region and three CDRs (LCDR1, LCDR2 and LCDR3) in each light chain variable region. Any one of a variety of well-known schemes can be used to determine the amino acid sequence boundaries of the CDRs, including the "Kabat" numbering scheme (see Kabat et al., (1991), "Sequences of Proteins of Immunological Interest", 5th edition, Public Health Service, National Institutes of Health, Bethesda, MD), the "Chothia" numbering scheme (see Al-Lazikani et al., (1997) JMB 273: 927-948), the ImMunoGenTics (IMGT) numbering scheme (Lefranc M. P., Immunologist, 7, 132-136 (1999); Lefranc, M. P., et al., Dev. Comp. Immunol., 27, 55-77 (2003)), etc. For example, for the classical format, according to the Kabat scheme, the CDR amino acid residues in the heavy chain variable domain (VH) are numbered 31-35 (HCDR1), 50-65 (HCDR2) and 95-102 (HCDR3); the CDR amino acid residues in the light chain variable domain (VL) are numbered 24-34 (LCDR1), 50-56 (LCDR2) and 89-97 (LCDR3). According to the Chothia scheme, the CDR amino acids in VH are numbered 26-32 (HCDR1), 52-56 (HCDR2) and 95-102 (HCDR3); and the amino acid residues in VL are numbered 26-32 (LCDR1), 50-52 (LCDR2) and 91-96 (LCDR3). According to the combination of CDR definitions provided in the Kabat scheme and the Chothia scheme, the CDR is composed of amino acid residues 26-35 (HCDR1), 50-65 (HCDR2) and 95-102 (HCDR3) in the human VH and amino acid residues 24-34 (LCDR1), 50-56 (LCDR2) and 89-97 (LCDR3) in the human VL. According to the IMGT scheme, the CDR amino acid residues in VH are roughly numbered 26-35 (CDR1), 51-57 (CDR2) and 93-102 (CDR3), and the CDR amino acid residues in VL are roughly numbered 27-32 (CDR1), 50-52 (CDR2) and 89-97 (CDR3). According to the IMGT scheme, the CDRs of the antibody can be determined using the program IMGT/DomainGap Align.

The term "antibody framework" refers to a portion of a variable domain VL or VH, which serves as a framework for the antigen-binding loops (CDRs) of the variable domain. It is essentially a variable domain without CDRs.

The term "binding to CD79b" of the present application refers to the ability to interact with CD79b or an epitope thereof, wherein the CD79b or the epitope thereof may be derived from humans. The term "antigen-binding site" of the present application refers to a discontinuous three-dimensional spatial site on an antigen that is recognized by the antibody or the antigen-binding fragment of the present application.

The term "epitope" or "antigenic determinant" refers to a site on an antigen to which an immunoglobulin or antibody specifically binds. Epitopes generally comprise at least 3, 4, 5, 6, 7, 8, 9, 10, 11, 12, 13, 14, or 15 contiguous or non-contiguous amino acids in a unique spatial conformation. See, e.g., Epitope Mapping Protocols in Methods in Molecular Biology, volume 66, G.E. Morris, Ed. (1996).

The terms "specific binding", "selective binding", "selectively bind to" and "specifically bind to" refer to the binding of an antibody to an epitope on a predetermined antigen. Generally, the antibody binds with an affinity (KD) of less than about $10^{-7}$ M, e.g., less than about $10^{-8}$ M, $10^{-9}$ M, or $10^{-10}$ M or less.

The amino acid sequence "identity" refers to the percentage of amino acid residues shared by a first sequence and a second sequence, wherein in aligning the amino acid sequences and when necessary, gaps are introduced to achieve maximum percent sequence identity, and any conservative substitution is not considered as part of the sequence identity. For the purpose of determining percent amino acid sequence identity, alignments can be achieved in a variety of ways that are within the scope of skills in the art, for example, using publicly available computer software such as BLAST, BLAST-2, ALIGN, ALIGN-2 or Megalign (DNASTAR) software. Those skilled in the art can determine parameters suitable for measuring alignment, including any algorithms required to achieve maximum alignment of the full length of the aligned sequences.

The term "cross-reactivity" refers to the ability of the antibody of the present application to bind to CD79b from different species. For example, the antibody of the present application that binds to human CD79b may also bind to CD79b from another species. Cross-reactivity is determined by detecting specific reactivity with a purified antigen in binding assays (e.g., SPR and ELISA) or binding or functional interactions with cells physiologically expressing CD79b. Methods for determining cross-reactivity include standard binding assays as described herein, for example, surface plasmon resonance analysis or flow cytometry.

The terms "inhibition" and "blocking" are used interchangeably and encompass both partial and complete inhibition/blocking. Inhibition/blocking of CD79b preferably reduces or alters the normal level or type of activity that occurs when CD79b binding occurs without inhibition or blocking. Inhibition and blocking are also intended to include any measurable decrease in CD79b binding affinity when in contact with an anti-CD79b antibody as compared to CD79b not in contact with an anti-CD79b antibody.

The term "inhibition of growth" (e.g., involving cells) is intended to include any measurable reduction in cell growth.

The terms "inducing immune response" and "enhancing immune response" can be used interchangeably and refer to the stimulation (i.e., passive or adaptive) of an immune response to a particular antigen. The term "induce" specific for inducing CDC or ADCC refers to stimulating specific direct cell killing mechanism.

"ADCC", i.e., antibody-dependent cell-mediated cytotoxicity, described in the present application means that the Fc receptor-expressing cells directly kill antibody-coated target cells by recognition of the Fc segment of the antibody. The ADCC effector function of the antibody may be reduced or eliminated by modification of the Fc segment of the IgG. The modification refers to a mutation in the heavy chain constant region of the antibody, such as a mutation selected from the group consisting of N297A, L234A, and L235A of IgG 1; IgG2/4 chimera, F235E of IgG4, and L234A/E235A mutation. The term "linker", "linker unit", or "linker fragment" refers to a chemical structural fragment or bond, which is linked to a ligand at one end and linked to a drug at the other end, and also may be linked to other linkers and then linked to the drug.

The linker may comprise one or more linker components. Exemplary linker components include 6-maleimidocaproyl ("MC"), maleimidopropionyl ("MP"), valine-citrulline ("val-cit" or "vc"), alanine-phenylalanine ("ala-phe"), p-aminobenzyloxycarbonyl ("PAB"), and those derived from coupling to a linker reagent: N-succinimidyl 4-(2-pyridylthio) pentanoate ("SPP"), N-succinimidyl 4-(N-maleimidomethyl)cyclohexane-1 carboxylate ("SMCC", also referred to herein as "MCC"), and N-succinimidyl(4-iodo-acetyl)aminobenzoate ("SIAB"). The linker may include a stretcher unit, a spacer unit, an amino acid unit, and an extension unit, and may be synthesized by methods known in the art, such as those described in US2005-0238649A1. A linker may be a "cleavable linker" favoring the release of drugs in cells. For example, acid-labile linkers (e.g., hydrazones), protease-sensitive (e.g., peptidase-sensitive) linkers, photolabile linkers, dimethyl linkers or disulfide-containing linkers can be used (Chari et al., Cancer Research 52: 127-131(1992); U.S. Patent No. 5,208,020).

The term "amino acid unit" refers to an amino acid that can attach a carbonyl group in the following structural formula $Y_R$ to an extension unit in the presence of the extension unit, or directly attaching $Y_R$ to a cytotoxic drug in the absence of the extension unit. In embodiments of the present disclosure, the amino acid unit is represented by $-K_k-$:

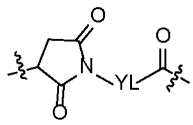

YR

- $K_k$- is a dipeptide, tripeptide, tetrapeptide, pentapeptide, hexapeptide, heptapeptide, octapeptide, nonapeptide or decapeptide-, wherein each -K- unit independently has the following structural formula $K_a$ or $K_b$, and k is an integer between 0 and 10:

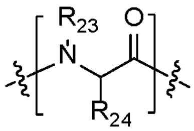 $K_a$ or 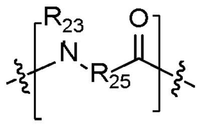 $K_b$

wherein:

$R_{23}$ in the above amino acid unit is -H or methyl;

$R_{24}$ is H, methyl, isopropyl, isobutyl, sec-butyl, benzyl, p-hydroxybenzyl, $-CH_2OH$, $-CH(OH)CH_3$, $-CH_2CH_2SCH_3$, $-CH_2CONH_2$, $-CH_2COOH$, $-CH_2CH_2CONH_2$, $-CH_2CH_2COOH$, $-(CH_2)_3NHC(=NH)NH_2$, $-(CH_2)_3NH_2$, $-(CH_2)_3NHCOCH_3$, $-(CH_2)_3NHCHO$, $-(CH_2)_4NHC(=NH)NH_2$, $-(CH_2)_4NH_2$, $-(CH_2)_4NHCOCH_3$, $-(CH_2)_4NHCHO$, $-(CH_2)_3NHCONH_2$, $-(CH_2)_4NHCONH_2$, $-CH_2CH_2CH(OH)CH_2NH_2$, 2-pyridylmethyl-, 3-pyridylmethyl-, 4-pyridylmethyl-, phenyl-, cyclohexyl,

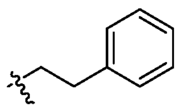 , 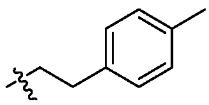 , 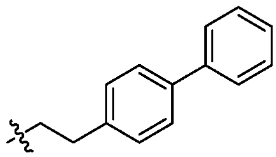 ,

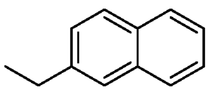 , 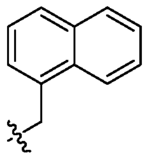 , 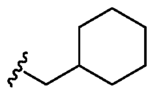 ,

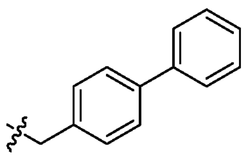 , 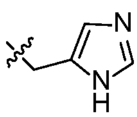 , 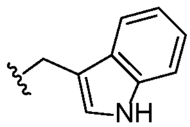 ;

$R_{25}$ is -aryl-, -alkyl-aryl-, -cycloalkyl-, -alkyl-cycloalkyl-, -cycloalkyl-alkyl-, -alkyl-cycloalkyl-alkyl-, - heterocyclyl-, -alkyl-heterocyclyl-, -heterocyclyl-alkyl-, -alkyl-heterocyclyl-alkyl-, -aryl-, -alkyl-aryl-, -aryl-alkyl-, -alkyl-aryl-alkyl-, -heteroaryl-, -alkyl-heteroaryl-, -heteroaryl-alkyl-, or -alkyl-heteroaryl-alkyl-.

In one embodiment, -$K_k$- is a dipeptide, preferably -valine-citrulline-, -phenylalanine-lysine- or -N-methylvaline-citrulline-, further preferably -valine-citrulline-.

The term "stretcher unit" refers to a chemical structure segment that is covalently linked to a ligand through a carbon atom at one end and to a cytotoxic drug through a sulfur atom at the other end. The term "spacer unit" is a bifunctional compound structural fragment that can be used to couple a linker unit to a cytotoxic drug to form a ligand-cytotoxic drug conjugate, in such a way that the cytotoxic drug is selectively linked to the linker unit.

The term "extension unit" refers to a chemical structure that can couple an amino acid unit to a cytotoxic drug in the presence of the amino acid unit or to a cytotoxic drug via the carbonyl group on YR in the absence of the amino acid unit. In an embodiment of the present disclosure, the extension unit is represented by -$Q_q$-, and q is selected from the group consisting of 0, 1, and 2.

In the present disclosure, the extension unit is PAB with a structure of 4-iminobenzylcarbamoyl fragment shown as the following formula, and is linked to D,

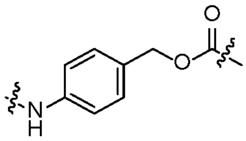

;

Abbreviation

Linker components include, but are not limited to:

MC = 6-maleimidocaproyl, whose structure is shown below:

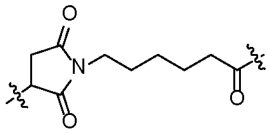

Val-Cit or "vc" = valine-citrulline (an exemplary dipeptide in a protease cleavable linker)
citrulline = 2-amino-5-ureidopentanoic acid
PAB = p-aminobenzyloxycarbonyl (an example of "self-immolative" linker components) Me-Val-Cit = N-methyl-valine-citrulline (wherein the linker peptide bond has been modified to prevent it from being cleaved by cathepsin B)
MC(PEG)6-OH = maleimidocaproyl-polyethylene glycol (attachable to antibody cysteine)
SPP = *N*-succinimidyl 4-(2-pyridylthio)valerate
SPDP = *N*-succinimidyl 3-(2-pyridyldithio)propionate
SMCC = succinimidyl-4-(*N*-maleimidomethyl)cyclohexane-1 -carboxylate
IT = iminothiolane
PBS = phosphate-buffered saline

The term "drug loading" refers to the mean number of cytotoxic drug molecules loaded on each ligand in an ADC, and may also be denoted as the ratio of the number of drug molecules to the number of antibody molecules. The drug loading may range from 1-20, preferably 1-10, cytotoxic drug (D) molecules attached to each antibody (Pc) molecule. In the embodiments of the present disclosure, the drug loading is represented by n or k; examples are 1, 2, 3, 4, 5, 6, 7, 8, 9, 10, 11, 12, 13, 14, 15, 16, 17, 18, 19, 20, or a mean of any two values. 1-10 is preferred, and a mean value of 1-8, or 1-8, or 1-7, or 2-8, or 2-7, or 2-6, or 2-5, or 2-3, or 1-2, or 2-4, or 1-4, or 1-5, or 1-6, or 3-8, or 3-7, or 3-6, or 4-7, or 4-6, or 4-5 is more preferred. The average number of drug molecules per ADC molecule after a coupling reaction can be characterized by conventional methods such as UV/visible spectroscopy, mass spectrometry, ELISA assays, monoclonal antibody molecule size variant assay (CE-SDS), and HPLC.

The monoclonal antibody molecular size variant assay (CE-SDS) of the present disclosure may be used for quantitatively determining the purity of a recombinant monoclonal antibody product by adopting capillary electrophoresis-sodium dodecyl sulfate (CE-SDS) ultraviolet assay based on the molecular weight under reduced and non-reduced conditions and according to a capillary electrophoresis method (Chinese Pharmacopoeia 0542, 2015 Edition).

In one embodiment of the present disclosure, the cytotoxic drug is coupled to the N-terminal amino of the ligand and/or ε-amino of the lysine residue through a linker unit, and generally, the number of drug molecules that can be coupled to the antibody in the coupling reaction will be less than the theoretical maximum.

The loading of the ligand cytotoxic drug conjugate can be controlled by the following non-limiting methods, including:

(1) controlling a molar ratio of a linking reagent to a monoclonal antibody,
(2) controlling reaction time and temperature, and
(3) selecting different reaction reagents.

Although the drug-to-antibody ratio has an exact value (e.g. n in formula (I)) for a specific conjugate molecule, it will be understood that when used to describe a sample containing many molecules, the value will often be a mean value, which attributed to a certain degree of non-uniformity typically associated with the conjugation step. The mean loading of

an immunoconjugate sample is referred to herein as the drug-to-antibody ratio or "DAR". In some embodiments, the DAR is between about 1 and about 6, and typically about 1, 1.5, 2, 2.5, 3, 3.5, 4, 4.5, 5, 5.5, 6, 6.5, 7.0, 7.5 or 8.0. In some embodiments, at least 50% by weight of the sample is a compound with mean DAR ± 2, and preferably at least 50% of the sample is a conjugate containing mean DAR ± 1. Embodiments include those with a DAR of about 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, 5.0, 5.1, 5.2, 5.4, 5.4, 5.5, 5.6, 5.7, 5.8, 5.9, 6.0, 6.1, 6.2, 6.3, 6.4, 6.5, 6.6, 6.7, 6.8, 6.9, or 7.0 are included. In some embodiments, a DAR of 'about x' means that the measurement value of the DAR is within 20% of x.

The DAR was determined for example by extrapolating DAR values from LC-MS data of reduced and deglycosylated samples. LC/MS allows for quantification of the average number of payload (drug moiety) molecules linked to the antibody in the ADC. HPLC separates the antibody into light and heavy chains, and also separates the heavy (HC) and light (LC) chains according to the number of linker-payload groups in each chain. Mass spectrometry data enables identification of the types of components in a mixture, e.g., LC, LC+1, LC+2, HC+1, HC+2, etc. From the mean loading of the LC and HC chains, the mean DAR of the ADC can be calculated. The DAR of a given immunoconjugate sample represents the average number of drug (payload) molecules linked to a tetrameric antibody containing two light chains and two heavy chains. An example is the determination method for DAR described in WO2018142322.

The term "alkyl" refers to a saturated aliphatic hydrocarbon group that is a linear or branched group containing 1 to 20 carbon atoms, preferably alkyl containing 1 to 12 carbon atoms, more preferably alkyl containing 1 to 10 carbon atoms, and most preferably alkyl containing 1 to 6 carbon atoms. Non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, n-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, n-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, n-nonyl, 2-methyl-2-ethylhexyl, 2-methyl-3-ethylhexyl, 2,2-diethylpentyl, n-decyl, 3,3-diethylhexyl, 2,2-diethylhexyl, and various branched isomers thereof, and the like. More preferred is lower alkyl groups having 1 to 6 carbon atoms, and non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, and the like. Alkyl may be substituted or unsubstituted, and when it is substituted, the substituent may be substituted at any accessible point of attachment, and the substituent is preferably one or more of the following groups, independently selected from the group consisting of alkyl, alkenyl, alkynyl, alkoxy, alkylthio, alkylamino, halogen, sulfhydryl, hydroxy, nitro, cyano, cycloalkyl, heterocycloalkyl, aryl, heteroaryl, cycloalkoxy, heterocycloalkoxy, cycloalkylthio, heterocycloalkylthio, and oxo.

The term "heteroalkyl" refers to alkyl containing one or more heteroatoms selected from the group consisting of N, O, and S, wherein the alkyl is as defined above.

The term "alkylene" refers to a saturated linear or branched aliphatic hydrocarbon group having 2 residues derived from the parent alkane by removal of two hydrogen atoms from the same carbon atom or two different carbon atoms, which is a linear or branched group containing 1 to 20 carbon atoms, preferably alkylene containing 1 to 12 carbon atoms, and more preferably alkylene containing 1 to 6 carbon atoms. Non-limiting examples of alkylene include, but are not limited to, methylene($-CH_2-$), 1,1-ethylidene($-CH(CH_3)-$), 1,2-ethylidene($-CH_2CH_2$)-, 1,1-propylidene($-CH (CH_2CH_3)-$), 1,2-propylidene($-CH_2CH(CH_3)-$), 1,3-propylidene($-CH_2CH_2CH_2-$), 1,4-butylidene ($-CH_2CH_2CH_2CH_2-$), 1,5-butylidene($-CH_2CH_2CH_2CH_2CH_2-$), and the like. Alkylene may be substituted or unsubstituted, and when it is substituted, the substituent may be substituted at any accessible point of attachment, and the substituent is preferably substituted with one or more substituents independently optionally selected from the group consisting of alkyl, alkenyl, alkynyl, alkoxy, alkylthio, alkylamino, halogen, sulfhydryl, hydroxy, nitro, cyano, cycloalkyl, heterocyclyl, aryl, heteroaryl, cycloalkoxy, heterocycloalkoxy, cycloalkylthio, heterocycloalkylthio, and oxo.

The term "alkoxy" refers to -O-(alkyl) and -O-(unsubstituted cycloalkyl), wherein the alkyl or cycloalkyl is as defined above. Non-limiting examples of alkoxy include: methoxy, ethoxy, propoxy, butoxy, cyclopropyloxy, cyclobutoxy, cyclopentyloxy, and cyclohexyloxy. Alkoxy may be optionally substituted or unsubstituted, and when it is substituted, the substituent is preferably one or more of the following groups, independently selected from the group consisting of alkyl, alkenyl, alkynyl, alkoxy, alkylthio, alkylamino, halogen, sulfhydryl, hydroxy, nitro, cyano, cycloalkyl, heterocycloalkyl, aryl, heteroaryl, cycloalkoxy, heterocycloalkoxy, cycloalkylthio, and heterocycloalkylthio.

The term "cycloalkyl" refers to a saturated or partially unsaturated monocyclic or polycyclic hydrocarbon substituent; the cycloalkyl ring contains 3 to 20 carbon atoms, preferably 3 to 12 carbon atoms, more preferably 3 to 10 carbon atoms, and most preferably 3 to 8 carbon atoms. Non-limiting examples of monocyclic cycloalkyl include cyclopropyl, cyclobutyl, cyclopentyl, cyclopentenyl, cyclohexyl, cyclohexenyl, cyclohexadienyl, cycloheptyl, cycloheptatrienyl, cyclooctyl, and the like. Polycyclic cycloalkyl includes spirocycloalkyl, fused cycloalkyl, and bridged cycloalkyl.

The term "heterocyclyl" refers to a saturated or partially unsaturated monocyclic or polycyclic hydrocarbon substituent containing 3 to 20 ring atoms, wherein one or more of the ring atoms are heteroatoms selected from the group consisting of nitrogen, oxygen, and $S(O)_m$ (where m is an integer from 0 to 2), excluding a cyclic portion of -O-O-, -O-S- or -S-S-, and the other ring atoms are carbon atoms. It preferably contains 3 to 12 ring atoms, of which 1 to 4 are heteroatoms; more preferably, the cycloalkyl ring contains 3 to 10 ring atoms. Non-limiting examples of monocyclic heterocyclyl include pyrrolidinyl, piperidinyl, piperazinyl, morpholinyl, thiomorpholinyl, homopiperazinyl, and the like. Polycyclic heterocyclyl includes spiroheterocyclyl, fused heterocyclyl, and bridged heterocyclyl.

The term "spiroheterocyclyl" refers to a 5- to 20-membered polycyclic heterocyclyl group in which one atom (referred to as a spiro atom) is shared between monocyclic rings, wherein one or more ring atoms are heteroatoms selected from the group consisting of nitrogen, oxygen, and $S(O)_m$ (where m is an integer from 0 to 2), and the other ring atoms are carbon atoms. It may contain one or more double bonds, but none of the rings has a fully conjugated π-electron system. For example, it is 6- to 14-membered, for example, 7- to 10-membered. According to the number of spiro atoms shared between rings, spiroheterocyclyl may be monospiroheterocyclyl, bispiroheterocyclyl, or polyspiroheterocyclyl, preferably, monospiroheterocyclyl and bispiroheterocyclyl, for example, 4-membered/4-membered, 4-membered/5-membered, 4-membered/6-membered, 5-membered/ 5-membered, or 5-membered/6-membered monospiroheterocyclyl. Non-limiting examples of spiroheterocyclyl include:

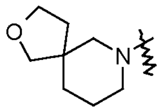 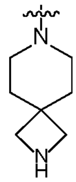 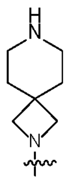 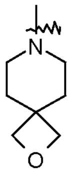 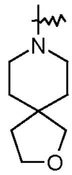 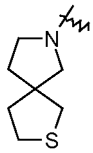 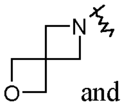 and 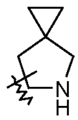

The term "fused heterocyclyl" refers to a 5- to 20-membered polycyclic heterocyclyl group in which each ring shares a pair of adjacent atoms with the other rings in the system, wherein one or more of the rings may contain one or more double bonds, but none of them has a fully conjugated π-electron system, wherein one or more of the ring atoms are heteroatoms selected from the group consisting of nitrogen, oxygen, and $S(O)_m$ (where m is an integer from 0 to 2), and the other ring atoms are carbon atoms. For example, it is 6- to 14-membered, for example, 7- to 10-membered. According to the number of constituent rings, it may be bicyclic, tricyclic, tetracyclic, or polycyclic fused heterocyclyl, for example, bicyclic or tricyclic, for example, 5-membered/5-membered or 5-membered/6-membered bicyclic fused heterocyclyl. Non-limiting examples of fused heterocyclyl include:

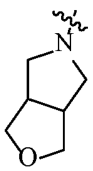 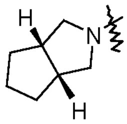 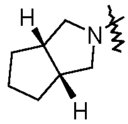 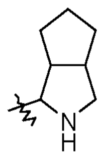 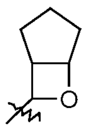 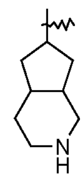 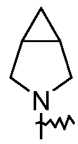 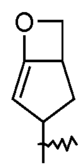 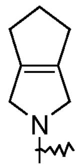 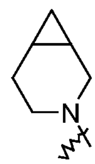

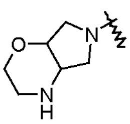 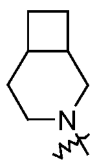 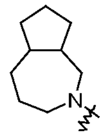 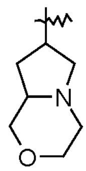 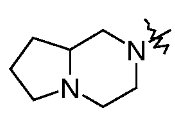 , and 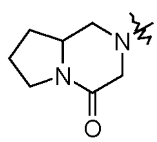

The term "bridged heterocyclyl" refers to a 5- to 14-membered polycyclic heterocyclyl group in which any two rings share two atoms that are not directly linked to each other, which may contain one or more double bonds, but none of the rings has a fully conjugated π-electron system, wherein one or more of the ring atoms are heteroatoms selected from the group consisting of nitrogen, oxygen, and $S(O)_m$ (where m is an integer from 0 to 2), and the other ring atoms are carbon atoms. For example, it is 6- to 14-membered, for example, 7- to 10-membered. According to the number of constituent rings, it may be bicyclic, tricyclic, tetracyclic, or polycyclic bridged heterocyclyl, for example, bicyclic, tricyclic, or tetracyclic, and more preferably bicyclic or tricyclic. Non-limiting examples of bridged heterocyclyl include:

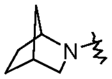 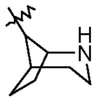 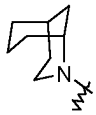 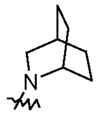

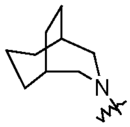 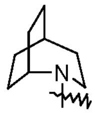 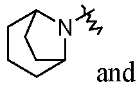 and 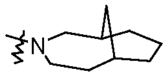

The heterocyclyl ring may be fused to an aryl, heteroaryl, or cycloalkyl ring, wherein the ring attached to the parent structure is heterocyclyl; its non-limiting examples include:

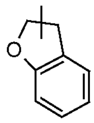, 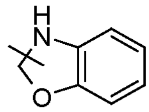, 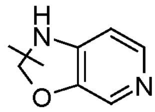, 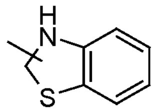,

and the like.

Heterocyclyl may be optionally substituted or unsubstituted, and when it is substituted, the substituent is preferably one or more of the following groups, independently selected from the group consisting of alkyl, alkenyl, alkynyl, alkoxy, alkylthio, alkylamino, halogen, sulfhydryl, hydroxy, nitro, cyano, cycloalkyl, heterocycloalkyl, aryl, heteroaryl, cycloalkoxy, heterocycloalkoxy, cycloalkylthio, heterocycloalkylthio, and oxo.

The term "aryl" refers to a 6- to 14-membered, for example, 6- to 10-membered, carbon monocyclic or fused polycyclic (i.e., rings sharing a pair of adjacent carbon atoms) group having a conjugated $\pi$-electron system, such as phenyl and naphthyl, specifically phenyl. The aryl ring may be fused to a heteroaryl, heterocyclyl, or cycloalkyl ring, wherein the ring attached to the parent structure is the aryl ring; its non-limiting examples include:

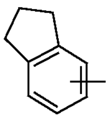, 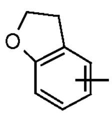, 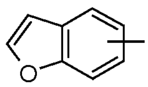, 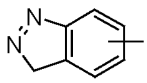, 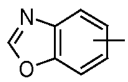, 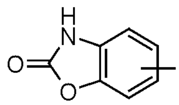, 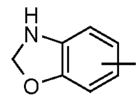,

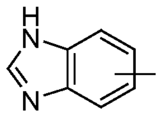, 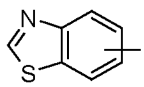, 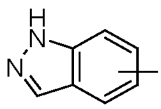, 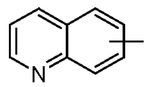, 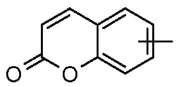, and 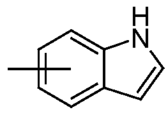

Aryl may be substituted or unsubstituted, and when it is substituted, the substituent is preferably one or more of the following groups, independently selected from the group consisting of alkyl, alkenyl, alkynyl, alkoxy, alkylthio, alkylamino, halogen, sulfhydryl, hydroxy, nitro, cyano, cycloalkyl, heterocycloalkyl, aryl, heteroaryl, cycloalkoxy, heterocycloalkoxy, cycloalkylthio, and heterocycloalkylthio.

The term "heteroaryl" refers to a heteroaromatic system containing 1 to 4 heteroatoms and 5 to 14 ring atoms, wherein the heteroatoms are selected from the group consisting of oxygen, sulfur, and nitrogen. Heteroaryl is preferably 5- to 10-membered, and is more preferably 5- or 6-membered, e.g., furanyl, thienyl, pyridyl, pyrrolyl, N-alkylpyrrolyl, pyrimidinyl, pyrazinyl, imidazolyl, and tetrazolyl. The heteroaryl ring may be fused to an aryl, heterocyclyl, or cycloalkyl ring, wherein the ring attached to the parent structure is the heteroaryl ring; its non-limiting examples include:

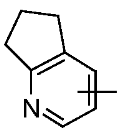, 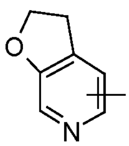, 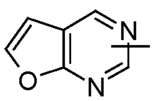, 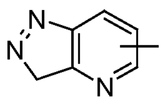, 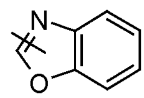, 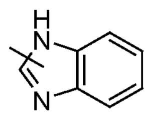,

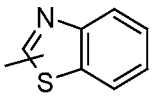, 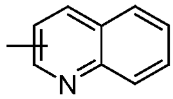, 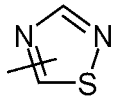, and 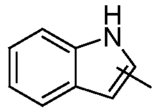

Heteroaryl may be optionally substituted or unsubstituted, and when it is substituted, the substituent is preferably one or more of the following groups, independently selected from the group consisting of alkyl, alkenyl, alkynyl, alkoxy, alkylthio, alkylamino, halogen, sulfhydryl, hydroxy, nitro, cyano, cycloalkyl, heterocycloalkyl, aryl, heteroaryl, cycloalkoxy, heterocycloalkoxy, cycloalkylthio, and heterocycloalkylthio.

The term "amino protecting group" refers to an easily removable group for protecting an amino group from being changed when reactions are taking place elsewhere in the molecule. Non-limiting examples of amino protecting groups include 9-fluorenylmethoxycarbonyl, tert-butoxycarbonyl, acetyl, benzyl, allyl, p-methoxybenzyl, etc. These groups may be optionally substituted with 1-3 substituents selected from the group consisting of halogen, alkoxy, and nitro. The amino protecting group is preferably 9-fluorenylmethoxycarbonyl.

The term "aminoheterocyclyl" refers to heterocyclyl substituted with one or more amino groups, preferably one amino group, wherein the heterocyclyl is as defined above, and "amino group" means $-NH_2$. Representative examples of the present disclosure are as follows:

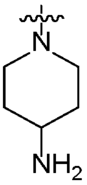 and 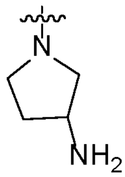

The term "heterocyclylamino" refers to amino substituted with one or more heterocyclyl groups, preferably one heterocyclyl group, wherein the amino is as defined above, and the heterocyclyl is as defined above. Representative examples of the present disclosure are as follows:

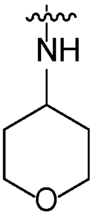

The term "cycloalkylamino" refers to amino substituted with one or more cycloalkyl groups, preferably one cycloalkyl group, wherein the amino is as defined above, and the cycloalkyl is as defined above. Representative examples of the present disclosure are as follows:

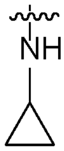 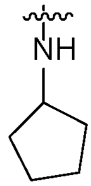

The term "cycloalkylalkyl" refers to alkyl substituted with one or more cycloalkyl groups, preferably one cycloalkyl group, wherein the alkyl is as defined above, and the cycloalkyl is as defined above. The term "haloalkyl" refers to alkyl substituted with one or more halogens, wherein the alkyl is as defined above.

The term "deuterated alkyl" refers to alkyl substituted with one or more deuterium atoms, wherein the alkyl is as defined above.

The term "hydroxy" refers to the -OH group.

The term "halogen" refers to fluorine, chlorine, bromine, or iodine.

The term "amino" refers to $-NH_2$.

The term "nitro" refers to $-NO_2$.

In chemical formulas, the abbreviation "Me" refers to methyl.

The present disclosure further comprises various deuterated forms of the compounds. Each available hydrogen atom connected to a carbon atom may be independently replaced by a deuterium atom. Those skilled in the art can synthesize the compounds in deuterated form by reference to the relevant literature. Commercially available deuterated starting materials can be used in preparing the deuterated compounds, or they can be synthesized using conventional techniques with deuterated reagents including, but not limited to, deuterated borane, tri-deuterated borane in tetrahydrofuran, deuterated lithium aluminum hydride, deuterated iodoethane, deuterated iodomethane, and the like. In another aspect, the hydrogen in the functional group of the compound of the present disclosure is substituted with deuterium to obtain the corresponding deuterated compound. The deuterated compound retains the selectivity and potential comparable to those of the hydrogen analog; deuterium bonds are more stable, which make "ADME", i.e., "toxic pharmacokinetics", different, thereby providing clinically beneficial effects. Toxic pharmacokinetics include the absorption, distribution, metabolism, and excretion of exogenous chemicals by an organism.

"Optional" or "optionally" means that the event or circumstance subsequently described may, but does not necessarily, occur, and this description includes instances where the event or circumstance occurs or does not occur. For example, "a heterocyclyl group optionally substituted with alkyl" means that the alkyl may, but does not necessarily, exist, and this description includes an instance where the heterocyclyl group is substituted with alkyl and an instance where it is not.

"Substituted" means that one or more, preferably up to 5, and more preferably 1 to 3, hydrogen atoms in the group are independently substituted with a corresponding number of substituents. It goes without saying that a substituent is only in its possible chemical position, and those skilled in the art will be able to determine (experimentally or theoretically) possible or impossible substitution without undue effort. For example, it may be unstable when an amino or hydroxy having free hydrogen is bound to a carbon atom having an unsaturated (e.g., olefinic) bond.

"Giving" and "treating", when applied to animals, humans, experimental subjects, cells, tissues, organs, or biological fluids, refer to contact of an exogenous drug, a therapeutic agent, a diagnostic agent, or a composition with the animals, humans, subjects, cells, tissues, organs or biological fluids. "Giving" and "treating" can refer to, for example, therapeutic, pharmacokinetic, diagnostic, research, and experimental methods. The treatment of cells comprises contacting the reagent with the cells and contacting the reagent with fluid, where the fluid is in contact with the cells. "Giving" and "treating" also refer to treating, e.g., cells by reagents, diagnosis, binding compositions or by another cell *in vitro* and *ex vivo.* "Treating", when applied to humans, veterinary or research subjects, refers to therapeutic treatment, preventive or prophylactic measures, and research and diagnostic applications. "Treatment" refers to administering a therapeutic agent, such as a composition comprising any one of the conjugation compounds of the present disclosure, either internally or externally to a patient with one or more disease symptoms on which the therapeutic agent is known to have a therapeutic effect. Typically, the therapeutic agent is administered in an amount effective to alleviate one or more disease symptoms in the patient or population being treated to induce regression of such symptoms or to inhibit the development of such symptoms to any clinically measurable degree. The amount of therapeutic agent effective to alleviate any particular disease symptom (also known as a "therapeutically effective amount") may vary depending on a variety of factors, such as the disease state, age, and weight of the patient, and the ability of the drug to produce a desired therapeutic effect in the patient. Whether a disease symptom has been alleviated can be evaluated by any clinical testing methods commonly used by doctors or other health care professionals to evaluate the severity or progression of the symptom. Although the embodiments of the present disclosure (for example treatment methods or products) may not be effective in alleviating all the target disease symptoms in every patient, as determined according to any statistical testing methods known in the art, such as Student t-test, chi-square test, Mann and Whitney's U test, Kruskal-Wallis test (H test), Jonckheere-Terpstra test and Wilcoxon test, they should reduce the target disease symptoms in a statistically significant number of patients.

"Effective amount" comprises an amount sufficient to ameliorate or prevent a symptom or disorder of a medical disease. An effective amount also refers to an amount sufficient to allow or facilitate diagnosis. The effective amount for a particular patient or veterinary subject may vary depending on factors such as the disorder to be treated, the general health of the patient, the method and route and dosage of administration, and the severity of side effects. An effective amount may be the maximum dose or administration regimen to avoid significant side effects or toxic effects.

## Examples

The present disclosure is further described below with reference to examples; however, these examples are not intended to limit the scope of the present disclosure. The experimental methods in the examples of the present disclosure in which specific conditions are not specified are generally performed under conventional conditions such as Antibodies: A Laboratory Manual and Molecular Cloning: A Laboratory Manual by Cold Spring Harbor Laboratory, or under conditions recommended by the manufacturer of the raw material or the goods. Reagents without specific origins indicated are commercially available conventional reagents.

**Example 1. Preparation of Anti-CD79b Antibodies**

The present disclosure provides an anti-CD79b antibody, obtained by immunization of mice with the extracellular domain (ECD) of human CD79b and screening. For details on the screening and functional identification processes, see WO2020156439A. The amino acid residues of the VH/VL CDRs of the antibody were determined and annotated using the Chothia numbering scheme. The sequences of the mouse hybridoma monoclonal antibody mAb015 and its humanized antibody hAb015-10 are as follows:
>mAb015 VH:

QVQLQQSGAELARPGASVKLSCKASGSSFTSYGINWVKQRTGQGLEWIGEIFPRSGNTYYN EKFEGKATLTADKSSSTAYMELRSLTSEDSAVYFCAKGDLGDFDYWGQGTTLTVSS

SEQ ID NO: 1

>mAb015 VL:

DFLMTQTPLSLPVRLGDQASISCRSSQSIVHSDGNTYFEWYLQKPGQSPKLLIYKVSNRFSG VPDRFSGSGSGTDFTLKISRVEAEDLGVYYCFQGSHVPWTFGGGTKLEIK

SEQ ID NO: 2

>hAb015-10 VH:

EVQLVQSGAEVKKPGSSVKVSCKASGSSFSSYGINWVKQAPGQGLEWIGEIFPRSGNTYYN EKFEGRATLTADKSTSTAYMELRSLRSEDTAVYYCAKGDLGDFDYWGQGTTVTVSS

SEQ ID NO: 3

>hAb015-10 VL:

DFVMTQTPLSLPVTPGEPASISCRSSQSIVHSDGNTYFEWYLQKPGQSPKLLIYKVSNRFSGV PDRFSGSGSGTDFTLKISRVEAEDVGVYYCFQGSHVPWTFGGGTKVEIK

SEQ ID NO: 4

Table 1. The CDR sequences of the anti-CD79b antibodies

| Antibody CDR | mAb015 | hAb015-10 |
|---|---|---|
| HCDR1 | GSSFTSY (SEQ ID NO: 5) | GSSFSSY (SEQ ID NO: 11) |
| HCDR2 | FPRSGN (SEQ ID NO: 6) | SEQ ID NO: 6 |
| HCDR3 | GDLGDFDY (SEQ ID NO: 7) | SEQ ID NO: 7 |
| LCDR1 | RSSQSIVHSDGNTYFE (SEQ ID NO: 8) | SEQ ID NO: 8 |
| LCDR2 | KVSNRFS (SEQ ID NO: 9) | SEQ ID NO: 9 |
| LCDR3 | FQGSHVPWT (SEQ ID NO: 10) | SEQ ID NO: 10 |

> hAb015-10 heavy chain:

EVQLVQSGAEVKKPGSSVKVSCKASGSSFSSYGINWVKQAPGQGLEWIGEIFPRSGNTYYN

EKFEGRATLTADKSTSTAYMELRSLRSEDTAVYYCAKGDLGDFDYWGQGTTVTVSSASTK
GPSVFPLAPSSKSTSGGTAALGCLVKDYFPEPVTVSWNSGALTSGVHTFPAVLQSSGLYSLSS
VVTVPSSSLGTQTYICNVNHKPSNTKVDKKVEPKSCDKTHTCPPCPAPELLGGPSVFLFPPK
PKDTLMISRTPEVTCVVVDVSHEDPEVKFNWYVDGVEVHNAKTKPREEQYNSTYRVVSVL
TVLHQDWLNGKEYKCKVSNKALPAPIEKTISKAKGQPREPQVYTLPPSREEMTKNQVSLTC
LVKGFYPSDIAVEWESNGQPENNYKTTPPVLDSDGSFFLYSKLTVDKSRWQQGNVFSCSVM
HEALHNHYTQKSLSLSPGK SEQ ID NO: 12

> hAb015-10 light chain:

DFVMTQTPLSLPVTPGEPASISCRSSQSIVHSDGNTYFEWYLQKPGQSPKLLIYKVSNRFSGV
PDRFSGSGSGTDFTLKISRVEAEDVGVYYCFQGSHVPWTFGGGTKVEIKRTVAAPSVFIFPPS
DEQLKSGTASVVCLLNNFYPREAKVQWKVDNALQSGNSQESVTEQDSKDSTYSLSSTLTLS
KADYEKHKVYACEVTHQGLSSPVTKSFNRGEC SEQ ID NO: 13

**Example 2. Preparation of Compounds**

Experimental procedures without conditions specified in the examples of the present disclosure were generally conducted according to conventional conditions or according to conditions recommended by the manufacturers of the raw material or the goods. Reagents without specific origins indicated are commercially available conventional reagents.

The drug moieties of the antibody-drug conjugates of the present disclosure can be found in WO2020063676A, CN202010073671.6, US7098308, US6884869, and CN201911390425.7, and the synthesis and testing of related compounds are incorporated herein by reference in their entirety.

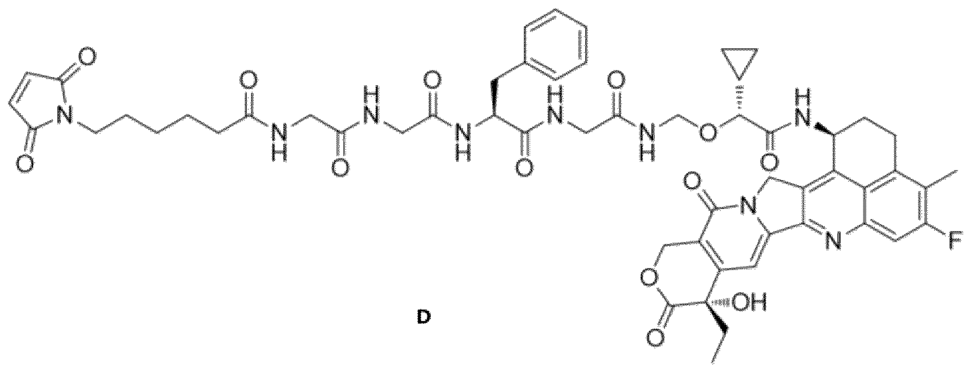

D

The method of preparing compound D can be found in, for example, Example 9 of WO2020063676A.

**Example 3. Preparation of Anti-CD79b Antibody-Drug Conjugates**

The experimental objective and principle of ADC stock solution drug loading analysis:
ADC stock solution is an antibody cross-linked drug, and the mechanism of treating diseases thereof is to transport toxin molecules into cells depending on the targeting performance of the antibody so as to kill the cells. The drug loading plays a decisive role in the drug efficacy. The drug loading of the ADC stock solution was determined using the ultraviolet method.

Method

Cuvettes containing a sodium succinate buffer were separately placed into the reference cell and sample cell, and the absorbance value of the solvent blank was subtracted. Then, a cuvette containing the test solution was placed into the sample cell, and the absorbance values at 280 nm and 370 nm were determined.

Calculation: the loading in the ADC stock solution was determined by ultraviolet spectrophotometry (instrument: Thermo nanodrop2000 ultraviolet spectrophotometer), based on the principle that the total absorbance value of the ADC stock solution at a certain wavelength was the sum of the absorbance values of the cytotoxic drug and the monoclonal antibody at that wavelength, namely:

$$(1)\ A_{280nm} = \varepsilon_{mab\text{-}280}bC_{mab}+\varepsilon_{Drug\text{-}280}bC_{Drug}$$

$\varepsilon_{Drug\text{-}280}$: the mean molar extinction coefficient of the drug at 280 nm is 5100;
$C_{Drug}$: the concentration of the drug;
$\varepsilon_{mab\text{-}280}$: the mean molar extinction coefficient of the monoclonal antibody stock solution at 280 nm is 214,600;
$C_{mab}$: the concentration of the monoclonal antibody stock solution;
b: the optical path length is 1 cm.

Similarly, an equation for the total absorbance value of the sample at 370 nm can be given as:

$$(2)\ A_{370nm} = \varepsilon_{mab\text{-}370}bC_{mab}+\varepsilon_{Drug\text{-}370}bC_{Drug}$$

$\varepsilon_{Drug\text{-}370}$: the mean molar extinction coefficient of the drug at 370 nm is 19,000;
$C_{Drug}$: the concentration of the drug;
$\varepsilon_{mab\text{-}370}$: the extinction coefficient of the monoclonal antibody stock solution at 370 nm is 0;
$C_{mab}$: the concentration of the monoclonal antibody stock solution;
b: the optical path length is 1 cm.

The drug loading can be calculated using both equations (1) and (2) as well as the extinction coefficients of the monoclonal antibody and the drug at both wavelengths and their concentrations. Drug loading = $C_{Drug}/C_{mab}$.

**Example 3-1**. **ADC-3**

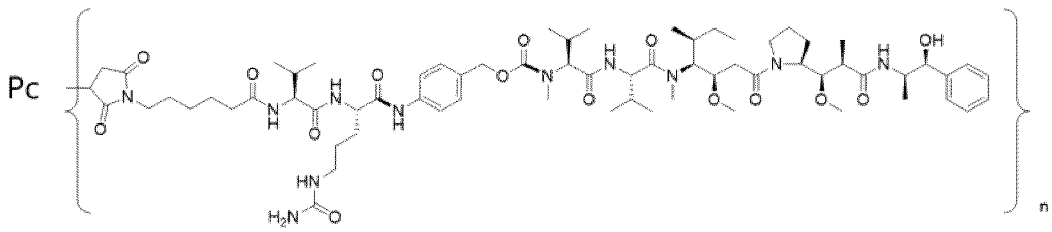

ADC-3

To antibody **hAb017-10** in aqueous PBS buffer (a pH 6.5, 0.05 M aqueous PBS buffer, 10.0 mg/mL, 5.0 mL, 0.338 μmol) at 37 °C was added a prepared aqueous tris(2-carboxyethyl)phosphine (TCEP) solution (10 mM, 85.0 μL, 0.850 μmol). The mixture was shaken on a water bath shaker at 37 °C for 3 h, and the reaction was stopped. The reaction mixture was cooled to 25 °C in a water bath. Compound **MC-vc-PAB-MMAE** (4.45 mg, 3.380 μmol) was dissolved in dimethyl sulfoxide (250 μL), and the solution was added to the above reaction mixture. The resulting mixture was shaken on a water bath shaker at 25 °C for 3 h, and the reaction was stopped. The reaction mixture was desalted and purified using a Sephadex G25 gel column (elution phase: a pH 6.5, 0.05 *M* aqueous PBS buffer, containing 0.001 M EDTA) to give the title product of this example, **ADC-3 (i.e., hAb015-10-cys-MC-vc-PAB-MMAE),** in PBS buffer (2.79 mg/mL, 17.4 mL). The product was refrigerated at 4 °C. Mean value calculation by CE-SDS: n = 3.09.

**Example 3-2. ADC-5**

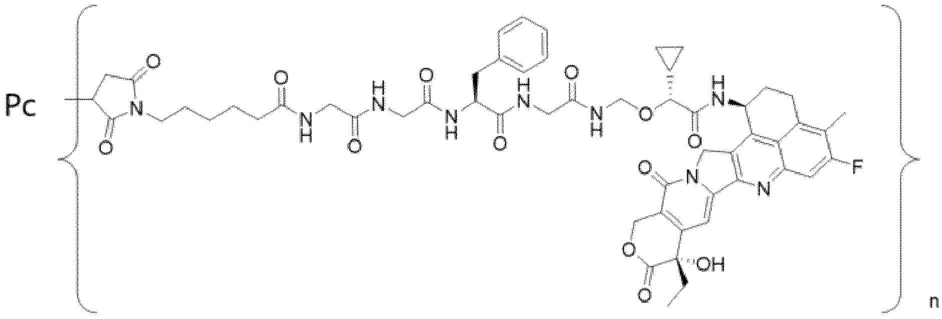

ADC-5

To antibody **hAb015-10** in aqueous PBS buffer (a pH 6.5, 0.05 M aqueous PBS buffer, 10.0 mg/mL, 1.5 mL, 0.101 μmol) at 37 °C was added a prepared aqueous tris(2-carboxyethyl)phosphine (TCEP) solution (10 mM, 16.2 μL, 0.162 μmol). The mixture was shaken on a water bath shaker at 37 °C for 3 h, and the reaction was stopped. The reaction mixture was cooled to 25 °C in a water bath. Compound **D** of this example (0.87 mg, 0.810 μmol) was dissolved in dimethyl sulfoxide (37 μL), and the solution was added to the above reaction mixture. The resulting mixture was shaken on a water bath shaker at 25 °C for 3 h, and the reaction was stopped. The reaction mixture was desalted and purified using a Sephadex G25 gel column (elution phase: a pH 6.5, 0.05 *M* aqueous PBS buffer, containing 0.001 M EDTA) to give the title product of this example, **ADC-5 (i.e., HAB015-10-cys-D, with a DAR value of about 2),** in PBS buffer (0.90 mg/mL, 14.0 mL). The product was refrigerated at 4 °C.

Mean value calculation by RP-HPLC: n = 1.81.

**Example 3-3. ADC-6**

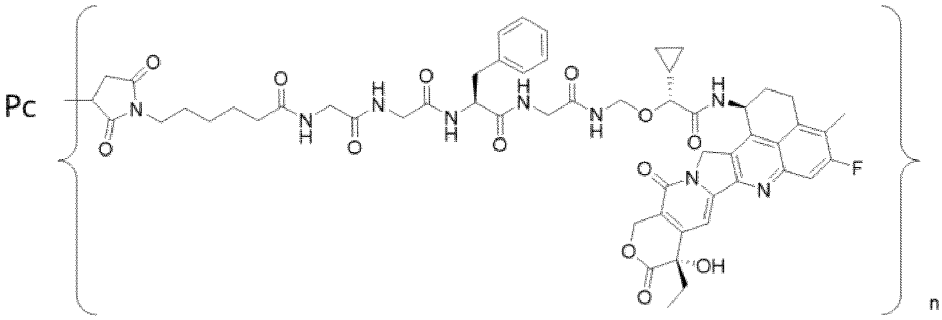

ADC-6

To antibody **hAb015-10** in aqueous PBS buffer (a pH 6.5, 0.05 M aqueous PBS buffer, 10.0 mg/mL, 1.5 mL, 0.101 μmol) at 37 °C was added a prepared aqueous tris(2-carboxyethyl)phosphine (TCEP) solution (10 mM, 25.3 μL, 0.253 μmol). The mixture was shaken on a water bath shaker at 37 °C for 3 h, and the reaction was stopped. The reaction mixture was cooled to 25 °C in a water bath. Compound **D** of Example 2 (1.09 mg, 1.015 μmol) was dissolved in dimethyl sulfoxide (45 μL), and the solution was added to the above reaction mixture. The resulting mixture was shaken on a water bath shaker at 25 °C for 3 h, and the reaction was stopped. The reaction mixture was desalted and purified using a Sephadex G25 gel column (elution phase: a pH 6.5, 0.05 M aqueous PBS buffer, containing 0.001 M EDTA) to give the title product of this example, **ADC-6 (i.e., hAb015-10-cys-D, with a DAR value of about 4),** in PBS buffer (0.71 mg/mL, 14.0 mL). The product was refrigerated at 4 °C.

Mean value calculation by RP-HPLC: n = 3.46.

**Example 3-4. ADC-7**

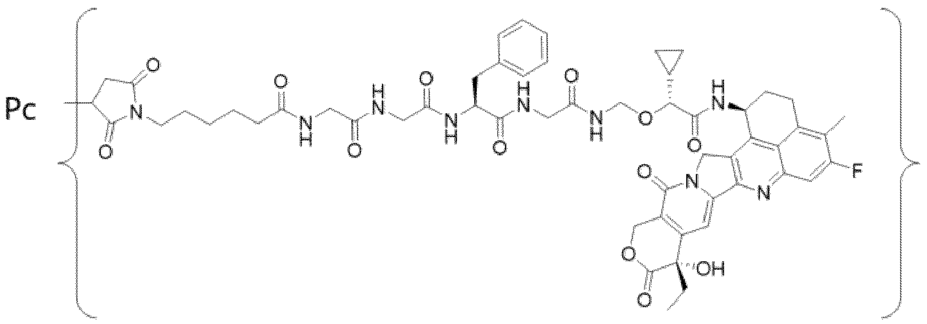

ADC-7

To antibody **hAb015-10** in aqueous PBS buffer (a pH 6.5, 0.05 M aqueous PBS buffer, 10.0 mg/mL, 1.5 mL, 0.101 μmol) at 37 °C was added a prepared aqueous tris(2-carboxyethyl)phosphine (TCEP) solution (10 mM, 50.7 μL, 0.507 μmol). The mixture was shaken on a water bath shaker at 37 °C for 3 h, and the reaction was stopped. The reaction mixture was cooled to 25 °C in a water bath.

Compound **D** of Example 2 (1.63 mg, 1.518 μmol) was dissolved in dimethyl sulfoxide (68 μL), and the solution was added to the above reaction mixture. The resulting mixture was shaken on a water bath shaker at 25 °C for 3 h, and the reaction was stopped. The reaction mixture was desalted and purified using a Sephadex G25 gel column (elution phase: a pH 6.5, 0.05 M aqueous PBS buffer, containing 0.001 M EDTA) to give the title product of this example, ADC-7 **(i.e., hAb015-10-cys-D, with a DAR value of about** 6), in PBS buffer (0.81 mg/mL, 13.5 mL). The product was refrigerated at 4 °C.

Mean value calculation by RP-HPLC: n = 5.84.

### Example 3-5. ADC-8

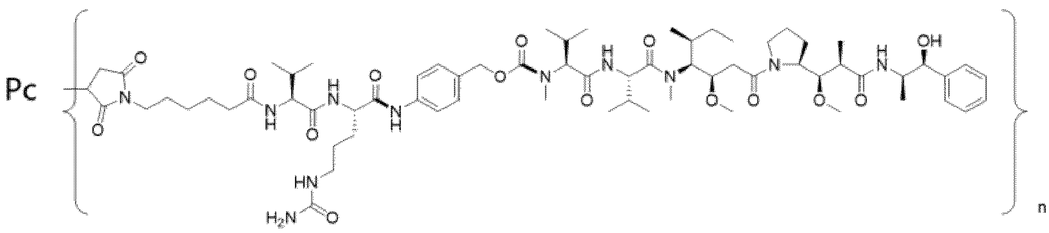

ADC-8

To antibody SN8 in aqueous PBS buffer (a pH 6.5, 0.05 M aqueous PBS buffer, 10.0 mg/mL, 79 mL, 5.338 μmol) at 37 °C was added a prepared aqueous tris(2-carboxyethyl)phosphine (TCEP) solution (10 mM, 1.388 mL, 13.88 μmol). The mixture was shaken on a water bath shaker at 37 °C for 3 h, and the reaction was stopped. The reaction mixture was cooled to 25 °C in a water bath. Compound **MC-VC-PAB-MMAE** (70.3 mg, 53.40 μmol) was dissolved in dimethyl sulfoxide (3.5 mL), and the solution was added to the above reaction mixture. The resulting mixture was shaken on a water bath shaker at 25 °C for 3 h, and the reaction was stopped. The reaction mixture was desalted and purified using a Sephadex G25 gel column (elution phase: a pH 6.5, 0.05 *M* aqueous PBS buffer, containing 0.001 M EDTA) to give the title product of this example, **ADC-8 (i.e., SN8-cys-MC-PAB-MMAE, with a DAR value of about 4),** in PBS buffer (5.83 mg/mL, 132 mL). The product was refrigerated at 4 °C.

Mean value calculation by CE-SDS: n = 3.59.

## Example 4. Biological Evaluations

### Example 4-1. Biacore Affinity Assay

The affinities of the CD79b antibodies (hAb015-10 and SN8) and ADC for CD79b protein were measured using Biacore. Data fitting was performed with a (1: 1) Langmuir model using BIAevaluation version 4.1, GE software to obtain affinity values.

The results of the affinity assay are shown in Table 2. The naked antibodies and different ADCs exhibited similar

levels of binding activity to human CD79b protein, and their binding activity levels were all higher than that of the positive drug Polivy.

Sequences of SN8 (i.e., the antibody in Polivy):

> Heavy chain amino acid sequence of SN8

EVQLVESGGGLVQPGGSLRLSCAASGYTFSSYWIEWVRQAPGKGLEWIGEILPGGGDTNYN
EIFKGRATFSADTSKNTAYLQMNSLRAEDTAVYYCTRRVPIRLDYWGQGTLVTVSSASTKG
PSVFPLAPSSKSTSGGTAALGCLVKDYFPEPVTVSWNSGALTSGVHTFPAVLQSSGLYSLSSV
VTVPSSSLGTQTYICNVNHKPSNTKVDKKVEPKSCDKTHTCPPCPAPELLGGPSVFLFPPKP
KDTLMISRTPEVTCVVVDVSHEDPEVKFNWYVDGVEVHNAKTKPREEQYNSTYRVVSVLT
VLHQDWLNGKEYKCKVSNKALPAPIEKTISKAKGQPREPQVYTLPPSREEMTKNQVSLTCL
VKGFYPSDIAVEWESNGQPENNYKTTPPVLDSDGSFFLYSKLTVDKSRWQQGNVFSCSVMH
EALHNHYTQKSLSLSPGK SEQ ID NO: 14

> Light chain amino acid sequence of SN8

DIQLTQSPSSLSASVGDRVTITCKASQSVDYEGDSFLNWYQQKPGKAPKLLIYAASNLESGV
PSRFSGSGSGTDFTLTISSLQPEDFATYYCQQSNEDPLTFGQGTKVEIKRTVAAPSVFIFPPSDE
QLKSGTASVVCLLNNFYPREAKVQWKVDNALQSGNSQESVTEQDSKDSTYSLSSTLTLSK
ADYEKHKVYACEVTHQGLSSPVTKSFNRGEC

SEQ ID NO: 15

Table 2. The binding activity of the antibodies and different ADCs to human CD79b protein

| ADC/antibody | Human CD79b $EC_{50}$ (nM) |
|---|---|
| hAb015-10 | 0.38 |
| ADC-3 | 0.40 |
| ADC-5 | 0.80 |
| ADC-6 | 0.43 |
| ADC-7 | 0.53 |
| ADC-8 | 5.98 |

**Example 4-2. *In Vitro* Endocytosis Assay**

A cell endocytosis assay was performed using DOHH-2 cells (DSMZ, ACC 47), which highly express human CD79b protein, to evaluate the endocytosis capacities of different ADCs.

It can be seen from the data in Table 3 that after 4 h of incubation with DoHH2 cells, the different ADCs all an exhibited endocytosis ratio of more than 65%, suggesting good endocytosis capacities. The endocytosis ratios of the ADCs were comparable to that of the positive drug Polivy.

Table 3. The endocytosis of the different ADCs in DoHH2 cells

| $EC_{50}$ | Endocytosis ratio (4 h) |
|---|---|
| ADC-3 | 70% |
| ADC-5 | 76% |

(continued)

| $EC_{50}$ | Endocytosis ratio (4 h) |
|---|---|
| ADC-6 | 75% |
| ADC-7 | 68% |
| ADC-8 | 76% |

**Example 4-3. Cell Proliferation Assay**

This example was intended to evaluate the effects of the different ADCs on the proliferation of DoHH2, WSU-DLCL2, and Raji cells cultured *in vitro.* According to literature reports (Leukemia. 2015 Jul; 29(7): 1578-86; Blood. 2007 Jul 15; 110(2): 616-23), DoHH2 is a cell highly expressing CD79b, WSU-DLCL2 is a cell lowly expressing CD79b, and Raji is a CD79b expression-negative cell.

Drugs: ADC-3 (DAR = 3.59), ADC-5 (DAR = 1.81), ADC-6 (DAR = 3.46), ADC-7 (DAR = 5.84), and ADC-8 (DAR = 3.59); all stored in light-resistant, airtight containers at 4 °C.

Cell strains: DOHH2 cells were purchased from DSMZ, WSU-DLCL-2 cells from American Type Culture Collection (ATCC), and Raji cells from American Type Culture Collection (ATCC).

A certain number of cells in the logarithmic growth phase were seeded into a 96-well culture plate, and drugs at different concentrations were added to the culture plate for reaction for 72 h. After the reaction was completed, an MTT working solution was added for reaction for 4 h, then blue-purple crystalline formazan was dissolved with a triplex solution. OD values at the wavelength of 570 nm and 690 nm were read using a microplate reader, and the cell growth inhibition rate was calculated by the following formula:

$$\text{Inhibition rate} = (\text{control well}_{OD570\ nm-OD690\ nm} - \text{dosing well}_{OD570\ nm-OD690\ nm})/\text{control well}_{OD570\ nm-OD690\ nm} \times 100\%$$

From the inhibition rates at various concentrations, the half maximal inhibitory concentration $IC_{50}$ was calculated using PrismGraph 8. The results are shown in Table 4.

Table 4. The *in vitro* proliferation inhibiting activity of the different ADCs

| **Compound** | **Toxin** | **$IC_{50}$ (ng/mL)** | | |
|---|---|---|---|---|
| | | **DoHH2** | **WSU-DLCL2** | **Raji** |
| ADC-3 | Tubulin polymerization inhibitor | 3.3 | 5.9 | >10000 |
| ADC-5 | Topo I inhibitor | 8.5 | 68.3 | >10000 |
| ADC-6 | | 3.3 | 54.2 | >10000 |
| ADC-7 | | 2.0 | 53.4 | ~10000 |
| ADC-8 | MMAE | 80.6 | 116.9 | >10000 |

**Example 4-4. Efficacy of ADCs Against Human Diffuse Large B-Cell Lymphoma WSU-DLCL2 Nude Mouse Subcutaneous Xenograft Tumors**

Drugs: ADC-3, ADC-6, and ADC-8 were the same as those in Example 4-3.

Cells and mice: human diffuse large B-cell lymphoma WSU-DLCL2 cells were purchased from American Type Culture Collection. Nude mice, BALB/c-nu, 35 days old, female, were purchased from Beijing Huafukang Biotechnology Co., Ltd.

Experimental steps: Each nude mouse was subcutaneously inoculated with $2.1\times10^7$ WSU-DLCL2 cells, and after the tumors grew to 100-150 $mm^3$, the animals were divided into groups according to the tumor volume (D0). The mice were dosed by intravenous injection (IV) at a volume of 10 mL/kg. The specific doses and administration regimen are shown in Table 5. The tumor volume and body weight were measured twice a week and the results were recorded.

Experimental measures and statistical analysis:

The calculation formula for tumor volume (V) was: $V = 1/2 \times a \times b^2$, where a and b represent length and width, respectively.

$T/C(\%) = (T - T_0)/(C - C_0) \times 100$, where T and C represent the tumor volumes at the end of the experiment; T0 and C0 represent the tumor volumes at the beginning of the experiment; T represents the tumor volume for the administration of an ADC, and C represents the tumor volume for the administration of IgG1 as a control group.

$$\text{Tumor growth inhibition rate \% (TGI\%)} = 100 - T/C\ (\%).$$

When the tumor starts to regress, tumor growth inhibition rate % (TGI%) = 100 - $(T - T_0)/T_0 \times 100$. If the tumor shrinks compared to its initial volume, i.e., $T < T_0$ or $C < C_0$, it is defined as partial regression (PR) of the tumor. If the tumor completely disappears, it is defined as complete regression (CR) of the tumor.

Unless otherwise indicated, the comparison of the tumor volumes of two groups was performed using two-way ANOVA test, with $P < 0.05$ defined as the presence of a statistically significant difference. ADC-3 (3 mg/kg, IV, D0) exhibited a tumor growth inhibition rate of 76% against WSU-DLCL2, with 1/6 of tumors partially regressing; ADC-6 (3 mg/kg, IV, D0) exhibited a tumor growth inhibition rate of 86% against WSU-DLCL2, with 2/6 of tumors partially regressing; ADC-8 (3 mg/kg, 10 mg/kg, IV, D0) exhibited tumor growth inhibition rates of 39% and 93%, respectively, against WSU-DLCL2, with 4/6 of tumors partially regressing in the 10 mg/kg dose group. The tumor-bearing mice well tolerated all of the above drugs: symptoms such as significant weight loss were not observed. IgG1 was used as a negative control.

The results are shown in Table 5 and FIGs. 1 and 2.

Table 5. The efficacy of the ADCs against human diffuse large B-cell lymphoma WSU-DLCL2 nude mouse subcutaneous xenograft tumors

| Group | Administration Time | Route of administration | Mean tumor volume ($mm^3$ ± SEM) | | T/C (%) D21 | Tumor growth inhibition rate (%) D21 | P value D21 | Partial regression | Number of animals at beginning/end of experiment |
|---|---|---|---|---|---|---|---|---|---|
| | | | DO | D21 | | | | | |
| IgG1 (10 mg/kg) | D0 | IV | 115.3±2.8 | 821.3±85.1 | - | - | - | - | 10/10 |
| ADC-3 (3 mg/kg) | D0 | IV | 114.4±2.9 | 284.8±50.0 | 24 | 76 | <0.0001 | 1 | 6/6 |
| ADC-6 (3 mg/kg) | D0 | IV | 118.1±4.5 | 217.4±80.3 | 14 | 86 | <0.0001 | 2 | 6/6 |
| ADC-8 (3 mg/kg) | D0 | IV | 117.6±3.0 | 547.2±38.1 | 61 | 39 | <0.0001 | 0 | 6/6 |
| ADC-8 (10 mg/kg) | D0 | IV | 115.0±3.7 | 167.8±50.3 | 7 | 93 | <0.0001 | 4 | 6/6 |

D0: the time of the first administration; P value: compared to the solvent; IV: intravenous injection; partial regression: the D21 tumor volume was less than the D0 tumor volume.

The single intravenous injections of ADC-3, ADC-6, and ADC-8 at 3 mg/kg or 10 mg/kg all exhibited significant efficacy against human diffuse large B-cell lymphoma WSU-DLCL2 nude mouse subcutaneous xenograft tumors, causing partial regression of the tumors; the drugs were significantly dose-dependent, and under the conditions of the same dose, the efficacy of the ADCs in which hAb015-10 is conjugated was better than that of the positive drug ADC-8 (i.e., Polivy). The tumor-bearing mice well tolerated all of the above drugs.

**Example 4-5. Efficacy of ADCs Against Human Diffuse Large B-Cell Lymphoma WSU-DLCL2 Nude Mouse Subcutaneous Xenograft Tumors**

Drugs: the ADC-5, ADC-6, ADC-7, and ADC-8 concentrations were the same as those in Example 4-3.

The experimental materials, steps, and measures were the same as those in Example 4-4. The statistical analysis was as follows:

Unless otherwise indicated, the comparison of the tumor volumes of two groups was performed using two-tailed Student's t-test, with $P < 0.05$ defined as the presence of a statistically significant difference.

ADC-5 (3 mg/kg, 6 mg/kg, 12 mg/kg, IV, D0) exhibited tumor growth inhibition rates of 69%, 86%, and 88%, respectively, against human diffuse large B-cell lymphoma WSU-DLCL2 nude mouse subcutaneous xenograft tumors, with 1/6 and 1/6 of tumors partially regressing in the 6 mg/kg and 12 mg/kg dose groups, respectively. ADC-6 (1.5 mg/kg, 3 mg/kg, 6 mg/kg, IV, D0) exhibited tumor growth inhibition rates of 66%, 108%, and 125%, respectively, against WSU-DLCL2 subcutaneous xenograft tumors, with 5/6 and 6/6 of tumors partially regressing in the 3 mg/kg and 6 mg/kg dose groups, respectively. ADC-7 (1 mg/kg, IV, D0) exhibited a tumor growth inhibition rate of 91% against WSU-DLCL2 subcutaneous xenograft tumors, with 1/6 of tumors partially regressing. ADC-8 (3 mg/kg, IV, D0) exhibited a tumor growth inhibition rate of 10% against WSU-DLCL2 subcutaneous xenograft tumors. The tumor-bearing mice well tolerated all of the above drugs: symptoms such as weight loss were not observed.

The specific results are shown in Table 6 and FIG. 3 to FIG. 5.

Table 6. The administration regimen and experimental results

| Group | Administration | Route | Mean tumor volume (mm3) | Mean tumor volume (mm3) | | | % T/C | % Tumor growth inhibition rate | P value | Partial regression | Number of animals per group at beginning of experiment | Number of animals per group at end of experiment |
|---|---|---|---|---|---|---|---|---|---|---|---|---|
| | | | D0 | SEM | D21 | SEM | D21 | D21 | D21 | | | |
| IgG1 negative control (12 mg/kg) | D0 | IV | 111.9 | ± 2.2 | 872.1 | ± 700 | - | - | - | 0 | 10 | 10 |
| ADC-5 (3mg/kg) | D0 | IV | 111.0 | ± 20 | 343.7 | ± 31.4 | 31 | 69 | 0.000 | 0 | 6 | 6 |
| ADC-5 (6mg/kg) | D0 | IV | 110.8 | ± 1.7 | 218.5 | ± 43.7 | 14 | 86 | 0.000 | 1 | 6 | 6 |
| ADC-5 (12mg/kg) | D0 | IV | 111.3 | ± 3.0 | 203.6 | ± 68.3 | 12 | 88 | 0.000 | 1 | 6 | 6 |
| ADC-6 (1.5mg/kg) | D0 | IV | 109.7 | ± 0.9 | 366.3 | ± 49.9 | 34 | 66 | 0.000 | 0 | 6 | 6 |
| ADC-6 (3mg/kg) | D0 | IV | 108.6 | ± 2.5 | 99.4 | ± 9.3 | -8 | 108 | 0.000 | 5 | 6 | 6 |
| ADC-6 (6mg/kg) | D0 | IV | 108.7 | ± 1.5 | 81.2 | ± 3.4 | -25 | 125 | 0.000 | 6 | 6 | 6 |
| ADC-7 (1mg/kg) | D0 | IV | 107.0 | ± 0.8 | 178.1 | ± 25.7 | 9 | 91 | 0.000 | 1 | 6 | 6 |
| ADC-1 (3mg/kg) | D0 | IV | 113.0 | ± 2.6 | 541.8 | ± 68.8 | 56 | 44 | 0.007 | 0 | 6 | 6 |
| ADC-8 (3mg/kg) | D0 | IV | 105.0 | ± 1.4 | 792.6 | ± 75.9 | 90 | 10 | 0.505 | 0 | 6 | 6 |

D0: the time of the first administration; P value: compared to the solvent; IV: intravenous injection.

**Example 4-6. Efficacy of ADCs Against Human B-Cell Lymphoma DoHH2 Nude Mouse Subcutaneous Xenograft Tumors**

Drugs: the ADC-6, and ADC-8 concentrations were the same as those in Example 4-3.

Cells and mice: human follicular lymphoma DOHH-2 cells were purchased from DSMZ, Germany. Nude mice, BALB/c-nu, 4-5 weeks old, female, were purchased from Shanghai Lingchang Biotechnology Co., Ltd.

Experimental steps: Each nude mouse was subcutaneously inoculated with $3 \times 10^7$ DOHH-2 cells, and after the tumors grew to 100-150 $mm^3$, the animals were divided into groups according to the tumor volume (D0). The mice were dosed by intravenous injection (IV) at a volume of 10 mL/kg. The specific doses and administration regimen are shown in Table 7. The tumor volume and body weight were measured twice a week and the results were recorded.

The experimental measures and statistical analysis were the same as those in Example 4-3.

ADC-1, ADC-6, and ADC-8(1 mg/kg, IV, D0) exhibited tumor growth inhibition rates of 82% (1/6 PR), 127% (5/6 PR), and 41%, respectively, against human follicular lymphoma DOHH-2 nude mouse subcutaneous xenograft tumors. The tumor-bearing mice well tolerated all of the above drugs: symptoms such as significant weight loss were not observed.

The results are shown in Table 7 and FIGs. 6 and 7.

Table 7. The efficacy of the different ADCs against human follicular lymphoma DOHH-2 nude mouse subcutaneous xenograft tumors

| Group | Administration | Route | Mean tumor volume (mm3) D0 | SEM | Mean tumor volume (mm3) D20 | SEM | T/C (%) D20 | Tumor growth inhibition rate(%) D20 | P value D20 | Partial regression | Number of animals per group at beginning/end of experiment |
|---|---|---|---|---|---|---|---|---|---|---|---|
| IgG1 (1 mg/kg) | D0 | IV | 115.7 | ± 2.7 | 1385.4 | ± 162.1 | - | - | - | 0 | 10/10 |
| ADC-6 (1 mg/kg) | D0 | IV | 112.9 | ± 3.5 | 82.4 | ± 6.3 | -27 | 127 | 0.000 | 5 | 6/6 |
| ADC-8 (1 mg/kg) | D0 | IV | 117.1 | ± 3.5 | 862.1 | ± 136.6 | 59 | 41 | 0.042 | 0 | 6/6 |

D0: the time of the first administration; P value: compared to the solvent; IV: intravenous injection; partial regression: the D21 tumor volume was less than the D0 tumor volume.

The single intravenous injection of ADC-6 at 1 mg/kg exhibited significant efficacy against human follicular lymphoma DoHH2 nude mouse subcutaneous xenograft tumors, causing partial regression of the tumors; the efficacy of ADC-6 was better than that of the positive drug ADC-8 (i.e., Polivy). The tumor-bearing mice well tolerated all of the above drugs.

The CD79b antibody-drug conjugate used in Example 5 to Example 8 was ADC-6 prepared in Example 3-3.

**Example 5. Screening for ADC Composition Formulation Buffer System pH Values**

The following buffers were prepared, and 20 mg/mL antibody formulations of the CD79b antibody-drug conjugate (ADC-6) were prepared. Samples were taken for high-temperature 40 °C and freeze-thaw stability studies.

1) 10 mM succinic acid-sodium succinate, pH 5.0
2) 10 mM succinic acid-sodium succinate, pH 5.5
3) 10 mM succinic acid-sodium succinate, pH 6.0
4) 10 mM citric acid-sodium citrate, pH 5.0
5) 10 mM citric acid-sodium citrate, pH 5.5
6) 10 mM citric acid-sodium citrate, pH 6.0
7) 10 mM histidine-histidine hydrochloride, pH 5.5
8) 10 mM histidine-histidine hydrochloride, pH 6.0

Table 8. The results of the ADC composition pH/buffer screening

| No. | Conditions | Appearance | SEC Monomer peak (%) | DAR | RCE HC+LC (%) |
|---|---|---|---|---|---|
| F1 | T0 | Colorless, clear, no particles | 97.38 | 4.03 | 98.78 |
| | 5 freeze-thaw cycles | Colorless, clear, no particles | 97.07 | 4.02 | 98.34 |
| | 40 °C 1W | Colorless, clear, no particles | 96.06 | 4.00 | 96.46 |
| | 40 °C 2W | Colorless, clear, with particles and flocs | 94.47 | 4.04 | 94.87 |
| | 40 °C 1M | Colorless, clear, with particles and flocs | 92.26 | 3.96 | 94.47 |
| F2 | T0 | Colorless, clear, no particles | 97.20 | 4.03 | 98.00 |
| | 5 freeze-thaw cycles | Colorless, clear, no particles | 96.76 | 4.02 | 98.17 |
| | 40 °C 1W | Colorless, clear, no particles | 95.79 | 4.00 | 97.25 |
| | 40 °C 2W | Colorless, slightly opalescent, with particles and flocs | 94.34 | 4.04 | 95.86 |
| | 40 °C 1M | Colorless, slightly opalescent, with particles and flocs | 92.23 | 3.98 | 94.79 |
| F3 | T0 | Colorless, clear, no particles | 96.50 | 4.03 | 98.74 |
| | 5 freeze-thaw cycles | Colorless, clear, no particles | 92.53 | 4.08 | 97.02 |
| | 40 °C 1W | Colorless, clear, with particles | 95.45 | 4.00 | 97.59 |
| | 40 °C 2W | Colorless, slightly opalescent, with particles and flocs | 93.91 | 4.06 | 96.85 |
| | 40 °C 1M | Colorless, slightly opalescent, with particles and flocs | 91.84 | 4.01 | 95.16 |

(continued)

| No. | Conditions | Appearance | SEC Monomer peak (%) | DAR | RCE HC+LC (%) |
|---|---|---|---|---|---|
| F4 | T0 | Colorless, clear, no particles | 97.41 | 4.04 | 98.42 |
| | 5 freeze-thaw cycles | Colorless, clear, no particles | 97.18 | 4.02 | 98.49 |
| | 40 °C 1W | Colorless, slightly opalescent, no particles | 94.24 | 3.96 | 97.20 |
| | 40 °C 2W | Colorless, slightly opalescent, with particles and flocs | 90.78 | 4.02 | 94.78 |
| | 40 °C 1M | Colorless, slightly opalescent, with particles and flocs | 89.34 | 3.92 | 91.88 |
| F5 | T0 | Colorless, clear, no particles | 97.12 | 4.04 | 98.33 |
| | 5 freeze-thaw cycles | Colorless, clear, no particles | 96.62 | 4.03 | 98.41 |
| | 40 °C 1W | Colorless, slightly opalescent, no particles | 95.67 | 4.00 | 97.14 |
| | 40 °C 2W | Colorless, slightly opalescent, with particles and flocs | 93.80 | 4.04 | 95.64 |
| | 40 °C 1M | Colorless, slightly opalescent, with particles and flocs | 91.35 | 3.98 | 94.12 |
| F6 | T0 | Colorless, clear, no particles | 96.78 | 4.03 | 98.32 |
| | 5 freeze-thaw cycles | Colorless, clear, no particles | 95.08 | 4.05 | 97.94 |
| | 40 °C 1W | Colorless, clear, with particles | 95.45 | 4.00 | 97.60 |
| | 40 °C 2W | Colorless, slightly opalescent, with particles and flocs | 93.70 | 4.04 | 97.14 |
| | 40 °C 1M | Colorless, slightly opalescent, with particles and flocs | 91.70 | 3.99 | 94.82 |
| F7 | T0 | Colorless, clear, no particles | 97.37 | 4.03 | 98.80 |
| | 5 freeze-thaw cycles | Colorless, clear, no particles | 97.00 | 4.04 | 98.01 |
| | 40 °C 1W | Colorless, clear, no particles | 96.77 | 4.02 | 97.19 |
| | 40 °C 2W | Colorless, clear, with particles and flocs | 95.95 | 4.03 | 96.84 |
| | 40 °C 1M | Colorless, slightly opalescent, with particles and flocs | 94.38 | 4.00 | 94.87 |

(continued)

| No. | Conditions | Appearance | SEC Monomer peak (%) | DAR | RCE HC+LC (%) |
|---|---|---|---|---|---|
| F8 | T0 | Colorless, clear, no particles | 97.31 | 4.02 | 98.27 |
| | 5 freeze-thaw cycles | Colorless, clear, no particles | 96.63 | 4.04 | 97.45 |
| | 40 °C 1W | Colorless, slightly opalescent, no particles | 96.63 | 4.00 | 97.58 |
| | 40 °C 2W | Colorless, slightly opalescent, with particles and flocs | 95.71 | 4.04 | 96.63 |
| | 40 °C 1M | Colorless, slightly opalescent, with particles and flocs | 94.37 | 4.01 | 95.76 |
| Note: W stands for week, and M stands for month. | | | | | |

The appearance results show that: after 5 freeze-thaw cycles, the appearances of F1-F8 did not change compared to the T0 appearances; as the pH increased, the 10 mM succinic acid-sodium succinate (F1-F3) and 10 mM citric acid-sodium citrate (F4-F6) system 40°C 1W samples transitioned from being particle-free to having particles, indicating that a low pH can inhibit particle formation; when the pH increased to 6.0, particles were observed in the formulas F3 and F6 (40°C 1W), whereas no significant particles were observed in the 10 mM histidine-histidine hydrochloride system (F8), indicating that the 10 mM histidine-histidine hydrochloride system is superior to the other two systems.

The SEC (FIG. 8A) results show that: there were no significant differences between the freeze-thaw SEC results and the T0 SEC results; at 40 °C, all the different formulas showed reductions, and the 10 mM histidine system showed smaller reductions than the succinic acid-sodium succinate and citric acid-sodium citrate systems.

The RCE (FIG. 8B) results show that: there were no significant differences between the freeze-thaw RCE results and the T0 RCE results; at 40 °C, all the different formulas showed reductions, with the 10 mM histidine system showing the slowest reduction, followed by the 10 mM succinic acid system. The DAR results show that: there were no significant differences between the freeze-thaw and 40 °C DAR results and the T0 DAR results.

Based on the appearance and purity results, the 10 mM succinic acid-sodium succinate pH 5.0 and 10 mM histidine-histidine hydrochloride pH 5.5-6.0 systems were selected for the subsequent tests.

**Example 6. Screening for ADC Composition Auxiliary Material and Surfactant**

The 10 mM succinic acid-sodium succinate pH 5.0 system was selected, and ADC composition formulations containing 20 mg/mL antibody-drug conjugate (ADC-6), different types and different concentrations of auxiliary materials, and different concentrations of surfactant were prepared. Samples were subjected to 5 freeze-thaw cycles at -35 °C/room temperature, shaken for 1 W (25 °C, 300 rpm), left at 40 °C for 1 W, left at 40 °C for 2 W, and left at 40 °C for 1 M to test their stability:

1) 10 mM succinic acid-sodium succinate pH 5.0, 60 mg/mL sucrose;

2) 10 mM succinic acid-sodium succinate pH 5.0, 90 mg/mL sucrose;

3) 10 mM succinic acid-sodium succinate pH 5.0, 60 mg/mL trehalose;

4) 10 mM succinic acid-sodium succinate pH 5.0, 90 mg/mL trehalose;

5) 10 mM succinic acid-sodium succinate pH 5.0, 0.2 mg/mL polysorbate 80, 60 mg/mL sucrose;

6) 10 mM succinic acid-sodium succinate pH 5.0, 0.4 mg/mL polysorbate 80, 60 mg/mL sucrose;

7) 10 mM succinic acid-sodium succinate pH 5.0, 0.6 mg/mL polysorbate 80, 60 mg/mL sucrose.

Table 9. The results of the ADC composition auxiliary material and surfactant screening

| No. | Conditions | Appearance | SEC Monomer peak (%) | DAR | Free mAb (%) | RCE HC+LC (%) |
|---|---|---|---|---|---|---|
| F1 | T0 | Colorless, clear, no particles | 97.45 | 4.04 | 3.36 | 97.59 |
| | 5 freeze-thaw cycles | Colorless, clear, no particles | 97.23 | 4.01 | 3.36 | 98.22 |
| | Shake | Colorless, slightly opalescent, no particles | 97.48 | 4.04 | 3.41 | 96.27 |
| | 40 °C 1W | Colorless, clear, no particles | 96.43 | 4.00 | 3.53 | 96.34 |
| | 40 °C 2W | Colorless, clear, with particles and flocs | 95.12 | 4.04 | 3.66 | 95.41 |
| | 40 °C 1M | Colorless, clear, with particles and flocs | 93.08 | 3.96 | 3.85 | 93.26 |
| F2 | T0 | Colorless, clear, no particles | 97.50 | 4.04 | 3.37 | 98.27 |
| | 5 freeze-thaw cycles | Colorless, clear, no particles | 97.35 | 4.01 | 3.37 | 98.27 |
| | Shake | Colorless, clear, with flocs | 97.41 | 4.03 | 3.42 | 97.76 |
| | 40 °C 1W | Colorless, clear, no particles | 96.49 | 4.00 | 3.51 | 96.39 |
| | 40 °C 2W | Colorless, clear, with particles and flocs | 95.18 | 4.03 | 3.65 | 97.09 |
| | 40 °C 1M | Colorless, clear, with particles and flocs | 93.22 | 3.96 | 3.84 | 92.9 |
| F3 | T0 | Colorless, clear, no particles | 97.45 | 4.04 | 3.38 | 98.67 |
| | 5 freeze-thaw cycles | Colorless, clear, no particles | 97.37 | 4.02 | 3.37 | 98.48 |
| | Shake | Colorless, clear, with flocs | 97.45 | 4.03 | 3.41 | 98.00 |
| | 40 °C 1W | Colorless, clear, with foreign matter | 96.49 | 4.00 | 3.54 | 96.86 |
| | 40 °C 2W | Colorless, clear, with particles and flocs | 95.24 | 4.03 | 3.64 | 95.92 |
| | 40 °C 1M | Colorless, clear, with particles and flocs | 93.08 | 3.95 | 3.83 | 94.65 |
| F4 | T0 | Colorless, clear, no particles | 97.47 | 4.04 | 3.37 | 98.11 |
| | 5 freeze-thaw cycles | Colorless, clear, no particles | 97.29 | 4.02 | 3.38 | 98.53 |
| | Shake | Colorless, clear, with flocs | 97.43 | 4.04 | 3.43 | 97.20 |
| | 40 °C 1W | Colorless, clear, no particles | 96.64 | 4.00 | 3.50 | 97.76 |
| | 40 °C 2W | Colorless, clear, with particles and flocs | 95.35 | 4.02 | 3.63 | 96.00 |
| | 40 °C 1M | Colorless, clear, with particles and flocs | 93.38 | 3.95 | 3.82 | 94.79 |

(continued)

| No. | Conditions | Appearance | SEC Monomer peak (%) | DAR | Free mAb (%) | RCE HC+LC (%) |
|---|---|---|---|---|---|---|
| F5 | T0 | Colorless, clear, no particles | 97.37 | 4.04 | 3.33 | 98.67 |
| | 5 freeze-thaw cycles | Colorless, clear, no particles | 97.21 | 4.02 | 3.35 | 97.61 |
| | Shake | Colorless, clear, no particles | 97.29 | 4.04 | 3.42 | 95.06 |
| | 40 °C 1W | Colorless, clear, no particles | 96.14 | 3.99 | 3.53 | 94.74 |
| | 40 °C 2W | Colorless, clear, with a small number of particles | 94.26 | 4.04 | 3.68 | 94.62 |
| | 40 °C 1M | Colorless, clear, with particles and flocs | 91.56 | 3.96 | 3.85 | 94.05 |
| F6 | T0 | Colorless, clear, no particles | 97.43 | 4.05 | 3.34 | 98.89 |
| | 5 freeze-thaw cycles | Colorless, clear, no particles | 97.20 | 4.03 | 3.34 | 98.01 |
| | Shake | Colorless, clear, no particles | 97.31 | 4.05 | 3.39 | 98.04 |
| | 40 °C 1W | Colorless, clear, no particles | 96.12 | 4.00 | 3.54 | 95.72 |
| | 40 °C 2W | Colorless, clear, with a small number of particles | 94.31 | 4.04 | 3.69 | 95.85 |
| | 40 °C 1M | Colorless, clear, with particles and flocs | 90.56 | 3.97 | 3.88 | 92.4 |
| F7 | T0 | Colorless, clear, no particles | 97.43 | 4.03 | 3.36 | 98.37 |
| | 5 freeze-thaw cycles | Colorless, clear, no particles | 97.14 | 4.02 | 3.38 | 98.24 |
| | Shake | Colorless, clear, no particles | 97.29 | 4.04 | 3.4 | 97.20 |
| | 40 °C 1W | Colorless, clear, no particles | 96.09 | 4.00 | 3.53 | 95.90 |
| | 40 °C 2W | Colorless, clear, with a small number of particles | 94.22 | 4.04 | 3.73 | 95.67 |
| | 40 °C 1M | Colorless, clear, with particles and flocs | 87.73 | 3.84 | 3.95 | 88.71 |
| Note: W stands for week, and M stands for month. | | | | | | |

The appearance results show that: there were no significant differences between the 5-freeze-thaw-cycle appearance results and the T0 appearance results; for the formulas containing polysorbate 80, no particles were observed in the formulations after 1 W of shaking (25 °C, 300 rpm), indicating that the addition of polysorbate 80 significantly reduced particle formation in the formulas after shakes; particles were observed in all the 40 °C 2W formulas, but no flocs were observed in the formulas containing polysorbate 80.

The SEC (FIG. 9A) results show that: at 40 °C, all the different formulas showed reductions in SEC purity, and the formulas containing different types and concentrations of sugars (F1-F4) did not significantly differ in SEC purity; as the polysorbate 80 concentration (F5-F7) increased (0.2 mg/mL, 0.4 mg/mL, and 0.6 mg/mL), the SEC purity gradually decreased.

The RCE (FIG. 9B) results show that: at 40 °C, all the different formulas showed reductions in RCE purity, and the formulas containing different sugar concentrations (F 1-F4) did not significantly differ in RCE purity; the 40 °C 1 M formulas containing trehalose (F3 and F4) exhibited a slightly higher RCE purity than the formulas containing sucrose (F 1 and F2); as the polysorbate 80 concentration (F5-F7) increased (0.2 mg/mL, 0.4 mg/mL, and 0.6 mg/mL), the RCE purity gradually decreased. The Free mAb and DAR value results show that: after high-temperature treatment, all the different formulas showed increases in the Free mAb level and slight reductions in the DAR value, and there were no significant differences between the results of the different formulas.

In summary, in the 10 mM succinic acid-sodium succinate pH 5.0 system, the sugar type and concentration did

not significantly affect the appearance, SEC, RCE, Free mAb, and DAR value of the protein; considering the economic cost and the osmotic pressure in the human body, 8% sucrose was selected as the target auxiliary material; in the 10 mM succinic acid-sodium succinate pH 5.0 system, the polysorbate 80 concentration was relatively good for the SEC and RCE purities of the antibody-drug conjugate at the high temperature of 40 °C.

**Example 7. Further ADC Composition pH Screening and Polysorbate 80 Concentration Screening**

Considering the particle issue, the 10 mM histidine-histidine hydrochloride system can be selected for further pH screening and polysorbate 80 concentration screening. The 10 mM histidine-histidine hydrochloride pH 5.5-6.0 buffer systems were selected, and ADC composition formulations containing 20 mg/mL protein (ADC-6), 0.4-0.6 mg/mL polysorbate 80, and 80 mg/mL sucrose as an auxiliary material were prepared for high-temperature 40 °C and freeze-thaw stability studies:

1) 10 mM histidine-histidine hydrochloride pH 5.5, 0.4 mg/mL polysorbate 80, 80 mg/mL sucrose;
2) 10 mM histidine-histidine hydrochloride pH 5.5, 0.6 mg/mL polysorbate 80, 80 mg/mL sucrose;
3) 10 mM histidine-histidine hydrochloride pH 5.8, 0.4 mg/mL polysorbate 80, 80 mg/mL sucrose;
4) 10 mM histidine-histidine hydrochloride pH 5.8, 0.6 mg/mL polysorbate 80, 80 mg/mL sucrose;
5) 10 mM histidine-histidine hydrochloride pH 6.0, 0.4 mg/mL polysorbate 80, 80 mg/mL sucrose;
6) 10 mM histidine-histidine hydrochloride pH 6.0, 0.6 mg/mL polysorbate 80, 80 mg/mL sucrose.

Table 10. The results of the further ADC composition pH screening and polysorbate 80 concentration screening

| No. | Conditions | Appearance | SEC Monomer peak (%) | DAR | Free mAb (%) | RCE HC+LC (%) |
|---|---|---|---|---|---|---|
| F1 | T0 | Colorless, clear, no particles | 97.18 | 4.05 | 3.74 | 98.94 |
| | 5 freeze-thaw cycles | Colorless, clear, no particles | 97.19 | 4.04 | 3.76 | 99.04 |
| | 40 °C 1W | Colorless, clear, no particles | 97.45 | 4.02 | 3.90 | 96.58 |
| | 40 °C 2W | Colorless, slightly opalescent, with particles and flocs | 96.30 | 4.02 | 3.71 | 97.12 |
| | 40 °C -4W | Colorless, slightly opalescent, with a small number of particles and flocs | 94.92 | 3.96 | 4.00 | 94.9 |
| F2 | T0 | Colorless, clear, no particles | 97.21 | 4.05 | 3.75 | 96.75 |
| | 5 freeze-thaw cycles | Colorless, clear, no particles | 97.15 | 4.04 | 3.76 | 98.16 |
| | 40 °C 1W | Colorless, clear, no particles | 97.28 | 4.02 | 3.90 | 95.75 |
| | 40 °C 2W | Colorless, slightly opalescent, with particles and flocs | 96.28 | 4.02 | 3.74 | 96.93 |
| | 40 °C 4W | Colorless, slightly opalescent, with a small number of particles and flocs | 94.79 | 3.89 | 4.00 | 94.87 |
| F3 | T0 | Colorless, clear, no particles | 97.20 | 4.04 | 3.76 | 96.23 |
| | 5 freeze-thaw cycles | Colorless, clear, no particles | 97.20 | 4.04 | 3.76 | 97.95 |
| | 40 °C 1W | Colorless, clear, no particles | 97.03 | 4.01 | 3.92 | 95.38 |
| | 40 °C 2W | Colorless, slightly opalescent, with particles and flocs | 95.77 | 4.02 | 3.75 | 97.37 |
| | 40 °C 4W | Colorless, slightly opalescent, with a small number of particles and flocs | 92.77 | 3.91 | 3.86 | 95.05 |

(continued)

| No. | Conditions | Appearance | SEC Monomer peak (%) | DAR | Free mAb (%) | RCE HC+LC (%) |
|---|---|---|---|---|---|---|
| F4 | T0 | Colorless, clear, no particles | 97.18 | 4.04 | 3.76 | 96.74 |
| | 5 freeze-thaw cycles | Colorless, clear, no particles | 97.21 | 4.04 | 3.76 | 98.19 |
| | 40 °C 1W | Colorless, clear, no particles | 97.12 | 4.01 | 3.91 | 97.51 |
| | 40 °C 2W | Colorless, slightly opalescent, with particles and flocs | 95.48 | 4.01 | 3.77 | 95.33 |
| | 40 °C 4W | Colorless, slightly opalescent, with a small number of particles and flocs | 93.54 | 3.89 | 3.97 | 95.39 |
| F5 | T0 | Colorless, clear, no particles | 97.08 | 4.03 | 3.75 | 97.64 |
| | 5 freeze-thaw cycles | Colorless, clear, no particles | 97.22 | 4.03 | 3.76 | 98.39 |
| | 40 °C 1W | Colorless, clear, no particles | 96.85 | 4.00 | 3.93 | 95.21 |
| | 40 °C 2W | Colorless, slightly opalescent, with particles and flocs | 95.54 | 4.01 | 3.79 | 96.04 |
| | 40 °C 4W | Colorless, slightly opalescent, with a small number of particles and flocs | 93.19 | 3.82 | 3.93 | 93.89 |
| F6 | T0 | Colorless, clear, no particles | 97.18 | 4.03 | 3.77 | 98.58 |
| | 5 freeze-thaw cycles | Colorless, clear, no particles | 97.22 | 4.03 | 3.77 | 97.51 |
| | 40 °C 1W | Colorless, clear, no particles | 96.85 | 4.00 | 3.93 | 96.88 |
| | 40 °C 2W | Colorless, slightly opalescent, with particles and flocs | 95.61 | 4.01 | 3.79 | 96.27 |
| | 40 °C 4W | Colorless, slightly opalescent, with a small number of particles and flocs | 93.42 | 3.87 | 3.99 | 96.63 |
| Note: W stands for week. | | | | | | |

The appearance results show that: the 5 freeze-thaw cycles did not cause significant change in appearance; particles were observed in all the 40 °C 2W formulas, and the different formulas did not significantly differ in the number of particles.

The SEC (FIG. 10A) results show that: at 40 °C, all the different formulas showed reductions in SEC purity; as the pH increased within the range of 5.5-6.0, the 40 °C 4W SEC purity decreased; the polysorbate 80 concentration (0.4 mg/mL, 0.6 mg/mL) did not significantly affect the SEC purity. The RCE (FIG. 10B) and DAR value results show that: at 40 °C, all the different formulas showed reductions in RCE purity and the DAR value, and there were no significant differences between the results of the different formulas.

The Free mAb results show that: after high-temperature treatment, all the different formulas showed increases in the Free mAb level, and there were no significant differences between the results of the different formulas.

In summary, in the 10 mM histidine-histidine hydrochloride system, lower pH values within the range of 5.5-6.0 were advantageous to the SEC purity of the antibody-drug conjugate. Considering the robustness of the formula, the target pH was determined to be 5.6; meanwhile, the polysorbate 80 concentration did not significantly affect the SEC and RCE of the protein. Considering that in the 10 mM succinic acid-sodium succinate pH 5.0 system, the polysorbate 80 concentration was relatively good for the SEC and reduced CE of the antibody-drug conjugate at 40 °C, the target polysorbate 80 concentration was finally determined to be 0.2 mg/mL. Considering the stability and appearance of the sample, the formula was preliminarily determined to be as follows: 10 mM histidine-histidine hydrochloride, 80 mg/mL sucrose, 0.2 mg/mL polysorbate 80, pH 5.6.

**Example 8. ADC Composition Formulation Component Screening and Lyophilization Stability**

To further optimize the concentration of the antibody-drug conjugate, the polysorbate 80 concentration, and the pH, DoE design was carried out using JMP software. In a 10 mM histidine-histidine hydrochloride buffer system, CD79b antibody-drug conjugate formulations containing different protein concentrations, different polysorbate 80 concentrations, and 80 mg/mL sucrose were prepared and bottled at 3.8 mL/vial. The formula designs are as follows:

1) 0.3 mg/mL polysorbate 80, pH 5.5, 15 mg/mL ADC-6
2) 0.2 mg/mL polysorbate 80, pH 6.0, 25 mg/mL ADC-6
3) 0.1 mg/mL polysorbate 80, pH 6.0, 18.15 mg/mL ADC-6
4) 0.2 mg/mL polysorbate 80, pH 5.75, 15 mg/mL ADC-6
5) 0.3 mg/mL polysorbate 80, pH 6.0, 15 mg/mL ADC-6
6) 0.1 mg/mL polysorbate 80, pH 5.5, 15 mg/mL ADC-6
7) 0.1 mg/mL polysorbate 80, pH 5.75, 25 mg/mL ADC-6
8) 0.2 mg/mL polysorbate 80, pH 5.5, 20 mg/mL ADC-6
9) 0.3 mg/mL polysorbate 80, pH 5.75, 20 mg/mL ADC-6
10) 0.3 mg/mL polysorbate 80, pH 5.5, 25 mg/mL ADC-6

The formulations were subjected to pre-freezing, primary drying, and secondary drying to obtain lyophilized products. The lyophilized products had good cake shapes and were, after reconstitution, clear in appearance with no significant changes in pH or purity, indicating that the lyophilization process is good. The stability of the lyophilized products was tested by placing them at 40 °C for 1 M.

Table 11. The results of the ADC-6 composition formulation component screening and lyophilization stability

| No. | Conditions | SEC(%) | | RCE(%) | iCIEF(%) | | | DAR value |
|---|---|---|---|---|---|---|---|---|
| | | Aggregate peak | Monomer peak | Light chain + heavy chain | Acidic peak | Main peak | Basic peak | |
| F1 | 0h | 0.33 | 99.4 | 98.5 | 35.4 | 57.4 | 0.6 | 4.03 |
| | 40 °C 1M | 0.36 | 99.4 | 98.2 | 35.1 | 63.1 | 0.5 | 4.04 |
| F2 | 0h | 0.40 | 99.3 | 98.1 | 35.1 | 61.8 | 0.6 | 4.06 |
| | 40 °C 1M | 0.43 | 99.6 | 97.9 | 35.0 | 63.0 | 0.6 | 4.07 |
| F3 | 0h | 0.39 | 99.6 | 98.4 | 35.2 | 61.0 | 0.6 | 4.06 |
| | 40 °C 1M | 0.40 | 99.6 | 95.2 | 34.9 | 60.2 | 0.6 | 4.06 |
| F4 | 0h | 0.34 | 99.4 | 98.2 | 35.0 | 61.5 | 0.7 | 4.05 |
| | 40 °C 1M | 0.34 | 99.7 | 97.5 | 34.7 | 62.8 | 0.6 | 4.05 |
| F5 | 0h | 0.36 | 99.4 | 98.2 | 34.9 | 63.1 | 0.6 | 4.06 |
| | 40 °C 1M | 0.37 | 99.6 | 98.1 | 34.6 | 63.5 | 0.6 | 4.06 |
| F6 | 0h | 0.33 | 99.4 | 98.4 | 35.3 | 59.4 | 0.6 | 4.04 |
| | 40 °C 1M | 0.36 | 99.4 | 97.2 | 34.6 | 62.7 | 0.6 | 4.03 |
| F7 | 0h | 0.37 | 99.4 | 98.1 | 35.2 | 60.5 | 0.6 | 4.05 |
| | 40 °C 1M | 0.41 | 99.6 | 97.4 | 34.6 | 62.8 | 0.6 | 4.05 |
| F8 | 0h | 0.35 | 99.4 | 98.3 | 35.3 | 59.2 | 0.6 | 4.04 |
| | 40 °C 1M | 0.39 | 99.3 | 97.7 | 34.5 | 63.2 | 0.6 | 4.03 |
| F9 | 0h | 0.36 | 99.4 | 98.3 | 35.2 | 62.4 | 0.5 | 4.05 |
| | 40 °C 1M | 0.39 | 99.3 | 97.6 | 34.6 | 63.0 | 0.6 | 4.05 |
| F10 | 0h | 0.38 | 99.4 | 98.1 | 34.9 | 62.9 | 0.6 | 4.04 |
| | 40 °C 1M | 0.42 | 99.3 | 97.6 | 34.6 | 63.1 | 0.6 | 4.03 |
| Note: M stands for month. | | | | | | | | |

The data showed that under the 40 °C 1M forced degradation conditions, the SEC monomer peak areas of all the formulas were above 99%, the RCE purity was above 95%, the neutral peak area was above 57%, and the DAR value remained stable at around 4; the different formulas did not significantly differ from the T0 formulas. That is, within the following ranges: 15-25 mg/mL, pH 5.5-6.0, and 0.1-0.3 mg/mL polysorbate 80, the CD79b antibody-drug conjugate (ADC-6) formulations did not significantly differ in purity, and the lyophilized formulations can stably exist.

## Claims

1. A pharmaceutical composition, comprising a ligand-drug conjugate and a buffer, wherein the ligand is an anti-CD79b antibody or an antigen-binding fragment thereof, and the drug is selected from the group consisting of MMAE or a derivative thereof, exatecan or a derivative thereof, and eribulin or a derivative thereof, wherein:

   the buffer is selected from the group consisting of an acetate buffer, a histidine salt buffer, a Tris-hydrochloride buffer, a Tris-citrate buffer, a phosphate buffer, and a succinate buffer;
   the anti-CD79b antibody or the antigen-binding fragment thereof comprises a heavy chain variable region and a light chain variable region, wherein the heavy chain variable region comprises a HCDR1 of the amino acid sequence set forth in SEQ ID NO: 11 or 5 and a HCDR2 and a HCDR3 of the amino acid sequences set forth in SEQ ID NO: 6 and SEQ ID NO: 7, and the light chain variable region comprises a LCDR1, a LCDR2, and a LCDR3 of the amino acid sequences set forth in SEQ ID NO: 8, SEQ ID NO: 9, and SEQ ID NO: 10;
   preferably, the buffer is a histidine salt buffer or a succinate buffer; preferably, the buffer is a histidine-hydrochloride buffer or a succinic acid-sodium succinate buffer.

2. The pharmaceutical composition according to claim 1, wherein the buffer or the pharmaceutical composition has a pH of 4.0 to 8.5, preferably 5.0 to 6.5, and more preferably 5.5 to 6.0.

3. The pharmaceutical composition according to claim 1 or 2, wherein the concentration of the buffer is 1 mM to 30 mM, preferably 5 mM to 15 mM, and more preferably about 10 mM.

4. The pharmaceutical composition according to any one of claims 1 to 3, wherein the concentration of the ligand-drug conjugate is 0.1 mg/mL to 50 mg/mL, preferably 10 mg/mL to 30 mg/mL, and more preferably 15 mg/mL to 25 mg/mL.

5. The pharmaceutical composition according to any one of claims 1 to 4, further comprising a surfactant, wherein:

   preferably, the surfactant is selected from the group consisting of poloxamer 188, polysorbate 80, and polysorbate 20;
   more preferably, the surfactant is polysorbate 80.

6. The pharmaceutical composition according to claim 5, wherein the concentration of the surfactant is 0.01 mg/mL to 1 mg/mL, preferably 0.05 mg/mL to 0.6 mg/mL, and more preferably 0.1 mg/mL to 0.3 mg/mL.

7. The pharmaceutical composition according to any one of claims 1 to 6, further comprising an osmotic pressure regulator, wherein:

   preferably, the osmotic pressure regulator is selected from the group consisting of one of, or a combination of more than one of, sucrose, trehalose, sorbitol, arginine, proline, glycine, and sodium chloride;
   more preferably, the osmotic pressure regulator is sucrose.

8. The pharmaceutical composition according to claim 7, wherein the concentration of the osmotic pressure regulator is 1 mg/mL to 300 mg/mL, preferably 30 mg/mL to 130 mg/mL, and more preferably about 80 mg/mL.

9. A pharmaceutical composition, wherein the pharmaceutical composition comprises:

   (a) 0.1 mg/mL to 50 mg/mL ligand-drug conjugate,
   (b) 0.5 mM to 50 mM histidine salt buffer or succinate buffer,
   (c) 1 mg/mL to 300 mg/mL sucrose or trehalose, and
   (d) 0.01 mg/mL to 1 mg/mL polysorbate,
   the pharmaceutical composition having a pH of 4.0 to 8.5;

or,

(a) 10 mg/mL to 30 mg/mL ligand-drug conjugate,
(b) 1 mM to 30 mM histidine salt buffer or succinate buffer,
(c) 30 mg/mL to 130 mg/mL sucrose or trehalose, and
(d) 0.05 mg/mL to 0.6 mg/mL polysorbate 80,

the pharmaceutical composition having a pH of 5.0 to 6.5;
or,

(a) 10 mg/mL to 50 mg/mL ligand-drug conjugate,
(b) 1 mM to 30 mM histidine salt buffer or succinate buffer,
(c) 30 mg/mL to 130 mg/mL sucrose or trehalose, and
(d) 0.05 mg/mL to 0.6 mg/mL polysorbate 80,

the pharmaceutical composition having a pH of 5.0 to 6.5;
or,

(a) 15 mg/mL to 25 mg/mL ligand-drug conjugate,
(b) 5 mM to 15 mM histidine salt buffer or succinate buffer,
(c) 70 mg/mL to 90 mg/mL sucrose, and
(d) 0.1 mg/mL to 0.3 mg/mL polysorbate 80,

the pharmaceutical composition having a pH of 5.0 to 6.5;
or,

(a) 15 mg/mL to 25 mg/mL ligand-drug conjugate,
(b) about 10 mM histidine salt buffer,
(c) about 80 mg/mL sucrose, and
(d) 0.1 mg/mL to 0.3 mg/mL polysorbate 80,

the pharmaceutical composition having a pH of 5.5 to 6.0;
or,

(a) 15 mg/mL to 25 mg/mL ligand-drug conjugate,
(b) about 10 mM succinate buffer,
(c) about 80 mg/mL sucrose, and
(d) 0.1 mg/mL to 0.3 mg/mL polysorbate 80,

the pharmaceutical composition having a pH of 5.0 to 6.0;
or,

(a) 15 mg/mL to 25 mg/mL ligand-drug conjugate,
(b) about 10 mM histidine-hydrochloride buffer,
(c) about 80 mg/mL sucrose, and
(d) 0.1 mg/mL to 0.3 mg/mL polysorbate 80,

the pharmaceutical composition having a pH of 5.5 to 6.0;
or,

(a) 15 mg/mL to 25 mg/mL ligand-drug conjugate,
(b) about 10 mM succinic acid-sodium succinate buffer,
(c) about 80 mg/mL sucrose, and
(d) 0.1 mg/mL to 0.3 mg/mL polysorbate 80,

the pharmaceutical composition having a pH of 5.0 to 6.0; preferably, the pharmaceutical composition comprises:

(a) 15 mg/mL to 25 mg/mL ligand-drug conjugate,

(b) about 10 mM histidine-histidine hydrochloride,
(c) about 80 mg/mL sucrose, and
(d) about 0.2 mg/mL polysorbate 80,

the pharmaceutical composition having a pH of 5.6;
or,

(a) 15 mg/mL to 25 mg/mL ligand-drug conjugate,
(b) about 10 mM histidine-histidine hydrochloride,
(c) about 80 mg/mL sucrose, and
(d) about 0.2 mg/mL polysorbate 80,

the pharmaceutical composition having a pH of about 5.5;
or,

(a) 15 mg/mL to 25 mg/mL ligand-drug conjugate,
(b) about 10 mM histidine-histidine hydrochloride,
(c) about 80 mg/mL sucrose, and
(d) about 0.2 mg/mL polysorbate 80,

the pharmaceutical composition having a pH of about 5.75;
or,

(a) 15 mg/mL to 25 mg/mL ligand-drug conjugate,
(b) about 10 mM histidine-histidine hydrochloride,
(c) about 80 mg/mL sucrose, and
(d) about 0.2 mg/mL polysorbate 80,

the pharmaceutical composition having a pH of about 6.0;
or,

(a) 15 mg/mL to 25 mg/mL ligand-drug conjugate,
(b) about 10 mM histidine-histidine hydrochloride,
(c) about 80 mg/mL sucrose, and
(d) about 0.3 mg/mL polysorbate 80,

the pharmaceutical composition having a pH of about 5.5;
or,

(a) 15 mg/mL to 25 mg/mL ligand-drug conjugate,
(b) about 10 mM histidine-histidine hydrochloride,
(c) about 80 mg/mL sucrose, and
(d) about 0.3 mg/mL polysorbate 80,

the pharmaceutical composition having a pH of about 5.75;
or,

(a) 15 mg/mL to 25 mg/mL ligand-drug conjugate,
(b) about 10 mM histidine-histidine hydrochloride,
(c) about 80 mg/mL sucrose, and
(d) about 0.3 mg/mL polysorbate 80,

the pharmaceutical composition having a pH of about 6.0;
or,

(a) 15 mg/mL to 25 mg/mL ligand-drug conjugate,
(b) about 10 mM histidine-histidine hydrochloride,
(c) about 80 mg/mL sucrose, and

(d) about 0.1 mg/mL polysorbate 80,

the pharmaceutical composition having a pH of about 5.5;
or,

(a) 15 mg/mL to 25 mg/mL ligand-drug conjugate,
(b) about 10 mM histidine-histidine hydrochloride,
(c) about 80 mg/mL sucrose, and
(d) about 0.1 mg/mL polysorbate 80,

the pharmaceutical composition having a pH of about 5.75;
or,

(a) 15 mg/mL to 25 mg/mL ligand-drug conjugate,
(b) about 10 mM histidine-histidine hydrochloride,
(c) about 80 mg/mL sucrose, and
(d) about 0.1 mg/mL polysorbate 80,

the pharmaceutical composition having a pH of about 6.0;
the ligand is an anti-CD79b antibody or an antigen-binding fragment thereof, and the anti-CD79b antibody or the antigen-binding fragment thereof comprises a heavy chain variable region and a light chain variable region, wherein the heavy chain variable region comprises a HCDR1 of the amino acid sequence set forth in SEQ ID NO: 11 or 5 and a HCDR2 and a HCDR3 of the amino acid sequences set forth in SEQ ID NO: 6 and SEQ ID NO: 7, and the light chain variable region comprises a LCDR1, a LCDR2, and a LCDR3 of the amino acid sequences set forth in SEQ ID NO: 8, SEQ ID NO: 9, and SEQ ID NO: 10;
the drug is selected from the group consisting of MMAE or a derivative thereof, exatecan or a derivative thereof, and eribulin or a derivative thereof, and is preferably exatecan or a derivative thereof.

**10.** The pharmaceutical composition according to any one of claims 1 to 9, wherein the anti-CD79b antibody or the antigen-binding fragment thereof comprises an antibody heavy chain variable region and an antibody light chain variable region, wherein: the sequence of the heavy chain variable region comprises SEQ ID NO: 3 or an amino acid sequence having at least 90% identity thereto, and the sequence of the light chain variable region comprises SEQ ID NO: 4 or an amino acid sequence having at least 90% identity thereto.

**11.** The pharmaceutical composition according to any one of claims 1 to 10, wherein the anti-CD79b antibody or the antigen-binding fragment thereof comprises a heavy chain and a light chain, wherein:
the sequence of the heavy chain comprises SEQ ID NO: 12 or an amino acid sequence having at least 80% identity thereto, and the sequence of the light chain comprises SEQ ID NO: 13 or an amino acid sequence having at least 80% identity thereto.

**12.** The pharmaceutical composition according to any one of claims 1 to 11, wherein the ligand-drug conjugate is represented by general formula (Pc-L-Y-D) of formula (I):

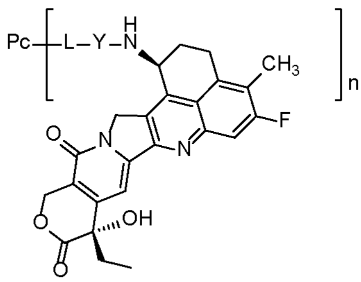

(Pc-L-Y-D) formula (I)

wherein:

Y is selected from the group consisting of -O-$(CR^aR^b)_m$-$CR^1R^2$-C(O)-, -O-$CR^1R^2$-$(CR^aR^b)_m$-, -O-$CR^1R^2$-,

-NH-$(CR^aR^b)_m$-$CR^1R^2$-C(O)-, and -S-$(CR^aR^b)_m$-$CR^1R^2$-C(O)-;
$R^a$ and $R^b$ are identical or different and are each independently selected from the group consisting of a hydrogen atom, a deuterium atom, halogen, alkyl, haloalkyl, deuterated alkyl, alkoxy, hydroxy, amino, cyano, nitro, hydroxyalkyl, cycloalkyl, and heterocyclyl; or, $R^a$ and $R^b$, together with the carbon atom to which they are attached, form cycloalkyl and heterocyclyl;
$R^1$ is selected from the group consisting of a hydrogen atom, halogen, haloalkyl, deuterated alkyl, cycloalkyl, cycloalkylalkyl, alkoxyalkyl, heterocyclyl, aryl, and heteroaryl; $R^2$ is selected from the group consisting of a hydrogen atom, halogen, haloalkyl, deuterated alkyl, cycloalkyl, cycloalkylalkyl, alkoxyalkyl, heterocyclyl, aryl, and heteroaryl; or, $R^1$ and $R^2$, together with the carbon atom to which they are attached, form cycloalkyl or heterocyclyl;
or, $R^a$ and $R^2$, together with the carbon atom to which they are attached, form cycloalkyl or heterocyclyl;
m is an integer from 0 to 4;
n is 1 to 10, and n is a decimal or integer; preferably, n is 2 to 8 or 5 to 9;
L is a linker unit;
Pc is an anti-CD79b antibody or an antigen-binding fragment thereof;
preferably, the ligand-drug conjugate has a structure represented by the following formula:

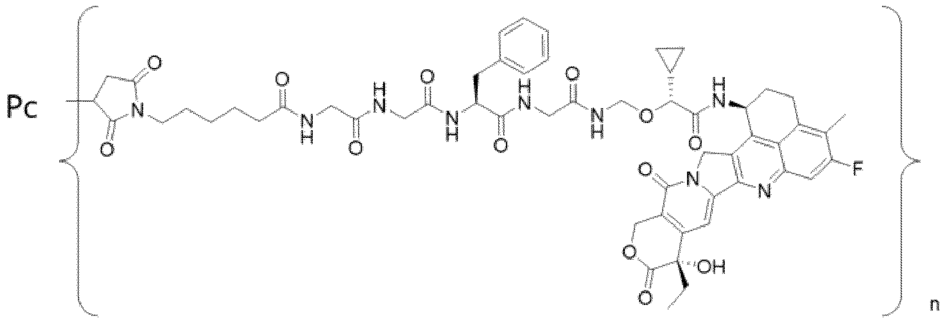

n is 1 to 10, and n is a decimal or integer;
more preferably, n is 1 to 6, and n is a decimal or integer.

**13.** A lyophilized formulation, wherein the lyophilized formulation is obtained by lyophilizing the pharmaceutical composition according to any one of claims 1 to 12, or the lyophilized formulation is capable of being reconstituted to form the pharmaceutical composition according to any one of claims 1 to 12.

**14.** A method for preparing a lyophilized formulation, comprising the step of lyophilizing the pharmaceutical composition according to any one of claims 1 to 12.

**15.** A reconstituted solution, prepared by reconstituting the lyophilized formulation according to claim 13.

**16.** The pharmaceutical composition according to any one of claims 1 to 12 or the reconstituted solution according to claim 15, being an agent for intravenous, subcutaneous, intraperitoneal, or intramuscular injection, preferably an agent for intravenous injection.

**17.** A product, comprising a container, wherein the container contains the pharmaceutical composition according to any one of claims 1 to 12, the lyophilized formulation according to claim 13, or the reconstituted solution according to claim 15.

**18.** Use of the pharmaceutical composition according to any one of claims 1 to 12, the lyophilized formulation according to claim 13, the reconstituted solution according to claim 15, or the product according to claim 17 in the preparation of a medicament for treating or preventing a proliferative disease or delaying the progression of a proliferative disease, wherein:

preferably, the proliferative disorder is a cancer or tumor;
more preferably, the cancer or tumor is selected from the group consisting of lymphoma, diffuse large B-cell lymphoma, non-Hodgkin's lymphoma (NHL), aggressive NHL, recurrent and aggressive NHL, recurrent and indolent NHL, refractory NHL, refractory and indolent NHL, chronic lymphocytic leukemia (CLL), small lymphocytic lymphoma, leukemia, hairy cell leukemia (HCL), acute lymphocytic leukemia (ALL), and/or mantle cell

lymphoma.

**19.** A method for treating or preventing a proliferative disease or delaying the progression of a proliferative disease, comprising administering to a subject in need thereof a therapeutically or prophylactically effective amount of the pharmaceutical composition according to any one of claims 1 to 12, the lyophilized formulation according to claim 13, the reconstituted solution according to claim 15, or the product according to claim 17, wherein:

preferably, the proliferative disorder is a cancer or tumor;
more preferably, the cancer or tumor is selected from the group consisting of lymphoma, diffuse large B-cell lymphoma, non-Hodgkin's lymphoma (NHL), aggressive NHL, recurrent and aggressive NHL, recurrent and indolent NHL, refractory NHL, refractory and indolent NHL, chronic lymphocytic leukemia (CLL), small lymphocytic lymphoma, leukemia, hairy cell leukemia (HCL), acute lymphocytic leukemia (ALL), and/or mantle cell lymphoma.

**20.** A method for enhancing immune function in a subject having a B-cell proliferative disorder or an autoimmune disorder, comprising administering to the subject an amount, effective in treating or delaying the disease, of the pharmaceutical composition according to any one of claims 1 to 12, the lyophilized formulation according to claim 13, the reconstituted solution according to claim 15, or the product according to claim 17, wherein:

preferably, the B-cell proliferative disorder is a cancer or tumor;
more preferably, the B-cell proliferative disorder is lymphoma, non-Hodgkin's lymphoma (NHL), aggressive NHL, recurrent and aggressive NHL, recurrent and indolent NHL, refractory NHL, refractory and indolent NHL, chronic lymphocytic leukemia (CLL), small lymphocytic lymphoma, leukemia, hairy cell leukemia (HCL), acute lymphocytic leukemia (ALL), and/or mantle cell lymphoma.

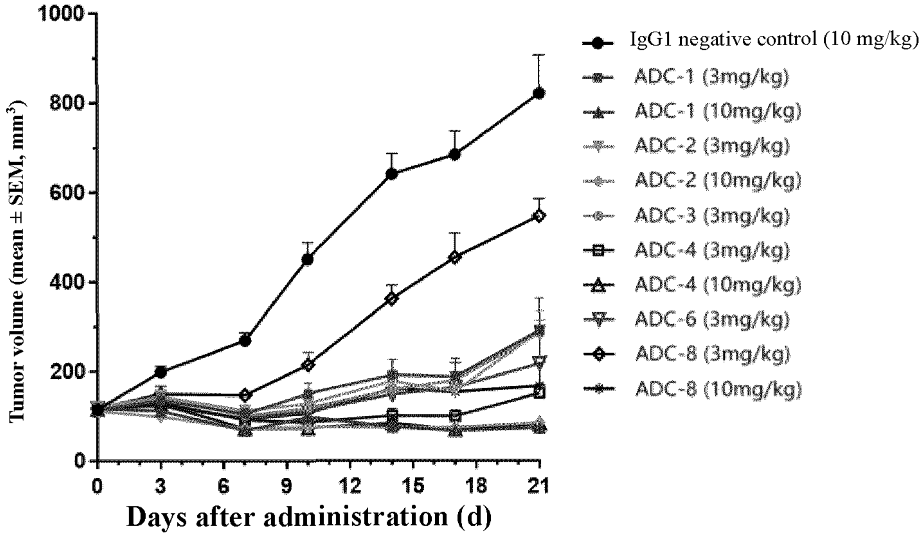
Tumor volume (mean ± SEM, mm³)
Days after administration (d)
IgG1 negative control (10 mg/kg)
ADC-1 (3mg/kg)
ADC-1 (10mg/kg)
ADC-2 (3mg/kg)
ADC-2 (10mg/kg)
ADC-3 (3mg/kg)
ADC-4 (3mg/kg)
ADC-4 (10mg/kg)
ADC-6 (3mg/kg)
ADC-8 (3mg/kg)
ADC-8 (10mg/kg)

FIG. 1A

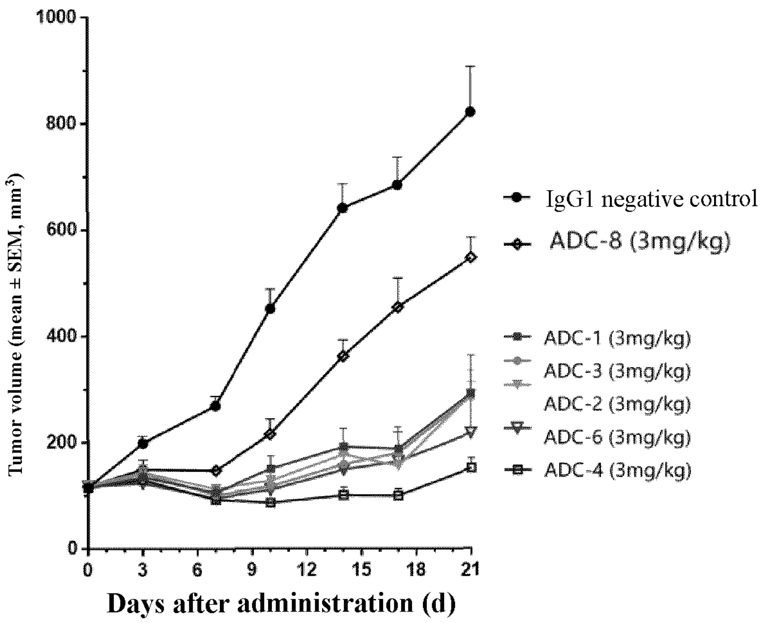
Tumor volume (mean ± SEM, mm³)
Days after administration (d)
IgG1 negative control
ADC-8 (3mg/kg)
ADC-1 (3mg/kg)
ADC-3 (3mg/kg)
ADC-2 (3mg/kg)
ADC-6 (3mg/kg)
ADC-4 (3mg/kg)

FIG. 1B

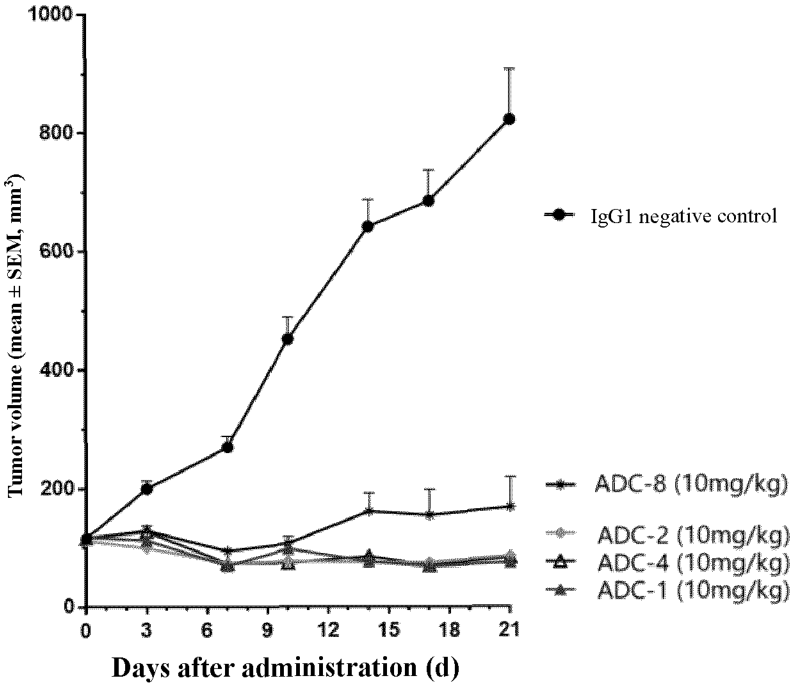
1000
800
600
400
200
0
Tumor volume (mean ± SEM, mm$^3$)
0
3
6
9
12
15
18
21
Days after administration (d)
IgG1 negative control
ADC-8 (10mg/kg)
ADC-2 (10mg/kg)
ADC-4 (10mg/kg)
ADC-1 (10mg/kg)

FIG. 1C

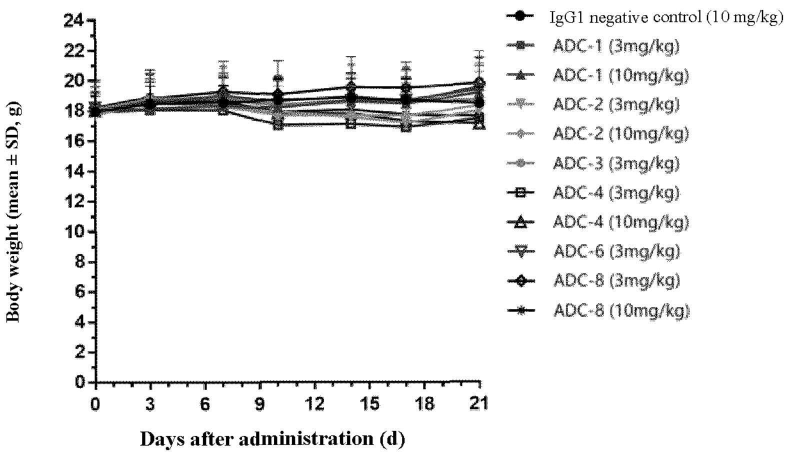
24
22
20
18
16
14
12
10
8
6
4
2
0
Body weight (mean ± SD, g)
0
3
6
9
12
15
18
21
Days after administration (d)
IgG1 negative control (10 mg/kg)
ADC-1 (3mg/kg)
ADC-1 (10mg/kg)
ADC-2 (3mg/kg)
ADC-2 (10mg/kg)
ADC-3 (3mg/kg)
ADC-4 (3mg/kg)
ADC-4 (10mg/kg)
ADC-6 (3mg/kg)
ADC-8 (3mg/kg)
ADC-8 (10mg/kg)

FIG. 2

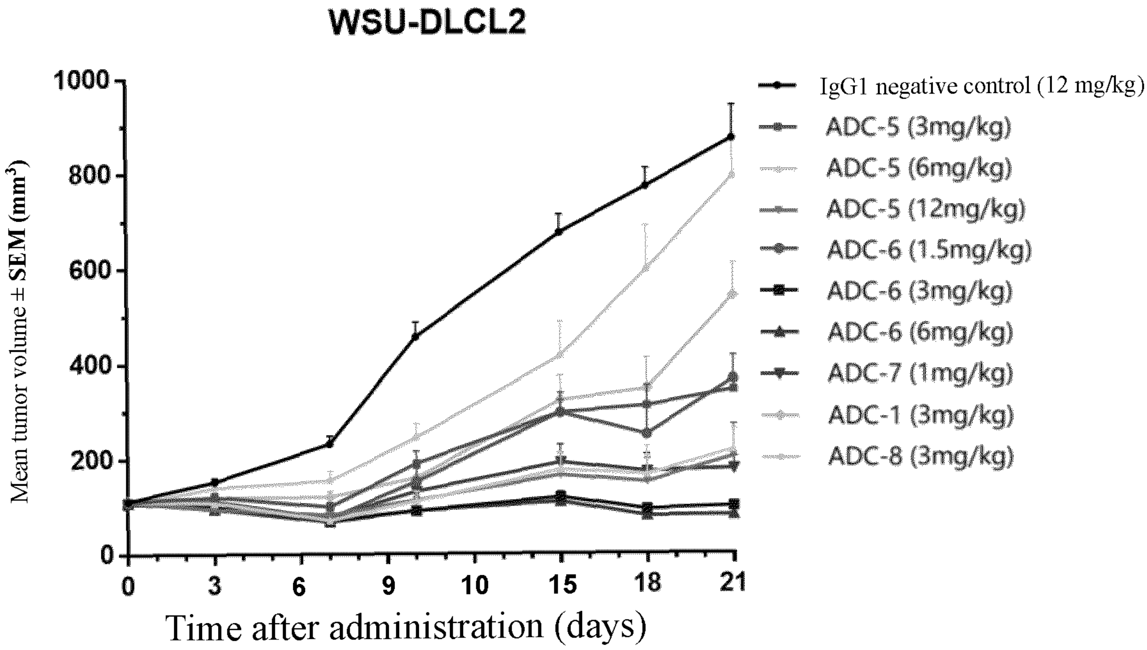
WSU-DLCL2
Mean tumor volume ± SEM (mm³)
1000
800
600
400
200
0
0
3
6
9
10
15
18
21
Time after administration (days)
IgG1 negative control (12 mg/kg)
ADC-5 (3mg/kg)
ADC-5 (6mg/kg)
ADC-5 (12mg/kg)
ADC-6 (1.5mg/kg)
ADC-6 (3mg/kg)
ADC-6 (6mg/kg)
ADC-7 (1mg/kg)
ADC-1 (3mg/kg)
ADC-8 (3mg/kg)

FIG. 3

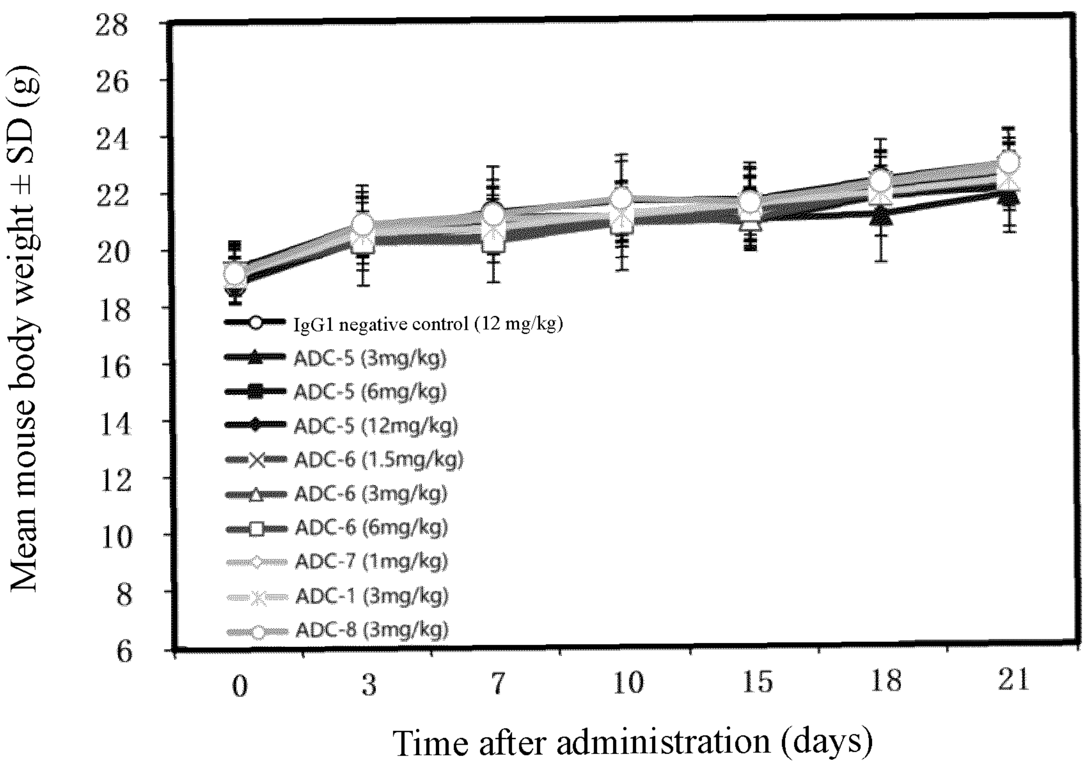
Mean mouse body weight ± SD (g)
28
26
24
22
20
18
16
14
12
10
8
6
IgG1 negative control (12 mg/kg)
ADC-5 (3mg/kg)
ADC-5 (6mg/kg)
ADC-5 (12mg/kg)
ADC-6 (1.5mg/kg)
ADC-6 (3mg/kg)
ADC-6 (6mg/kg)
ADC-7 (1mg/kg)
ADC-1 (3mg/kg)
ADC-8 (3mg/kg)
0
3
7
10
15
18
21
Time after administration (days)

FIG. 4

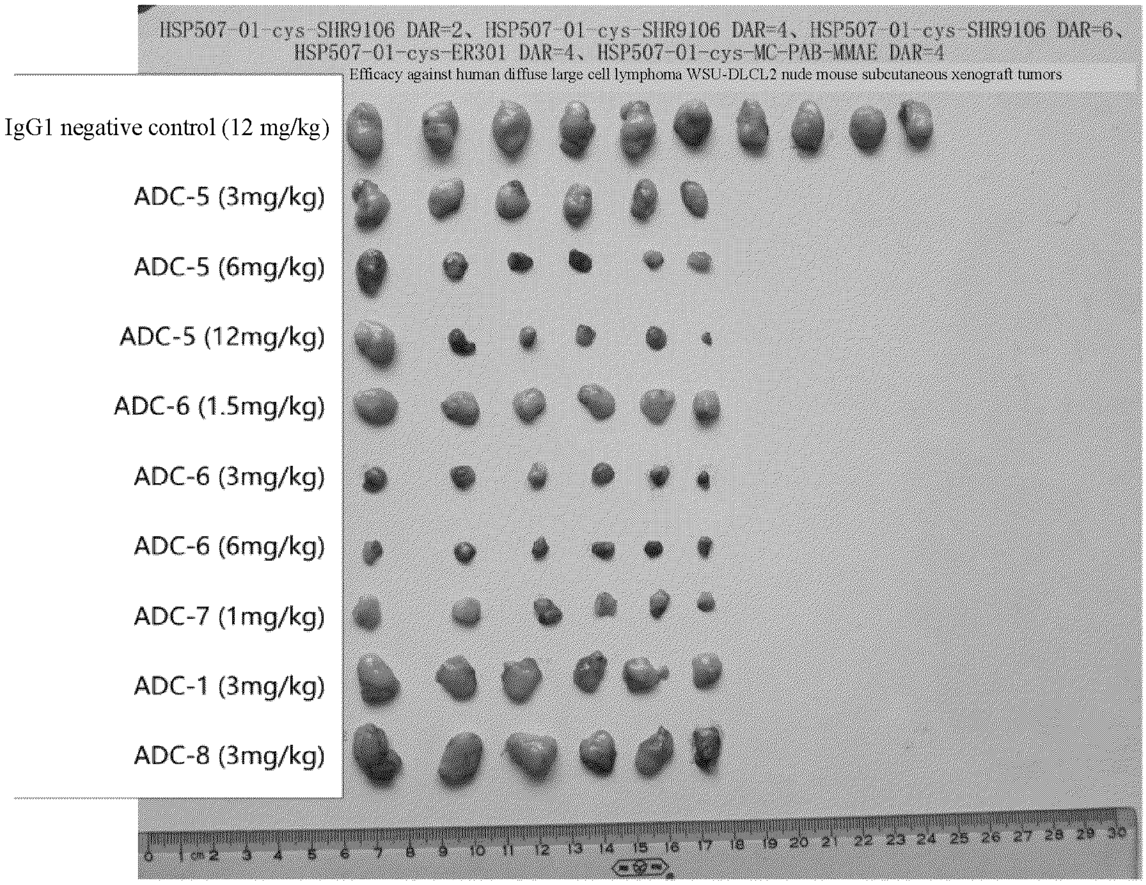
HSP507-01-cys-SHR9106 DAR=2、HSP507-01-cys-SHR9106 DAR=4、HSP507-01-cys-SHR9106 DAR=6、
HSP507-01-cys-ER301 DAR=4、HSP507-01-cys-MC-PAB-MMAE DAR=4
Efficacy against human diffuse large cell lymphoma WSU-DLCL2 nude mouse subcutaneous xenograft tumors
IgG1 negative control (12 mg/kg)
ADC-5 (3mg/kg)
ADC-5 (6mg/kg)
ADC-5 (12mg/kg)
ADC-6 (1.5mg/kg)
ADC-6 (3mg/kg)
ADC-6 (6mg/kg)
ADC-7 (1mg/kg)
ADC-1 (3mg/kg)
ADC-8 (3mg/kg)

FIG. 5

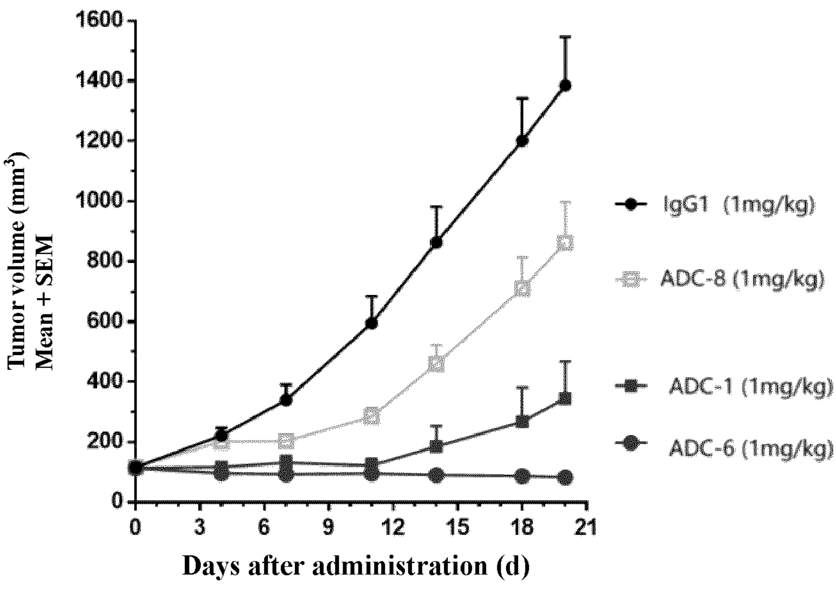
Tumor volume (mm3)
Mean + SEM
1600
1400
1200
1000
800
600
400
200
0
0
3
6
9
12
15
18
21
Days after administration (d)
IgG1 (1mg/kg)
ADC-8 (1mg/kg)
ADC-1 (1mg/kg)
ADC-6 (1mg/kg)

FIG. 6

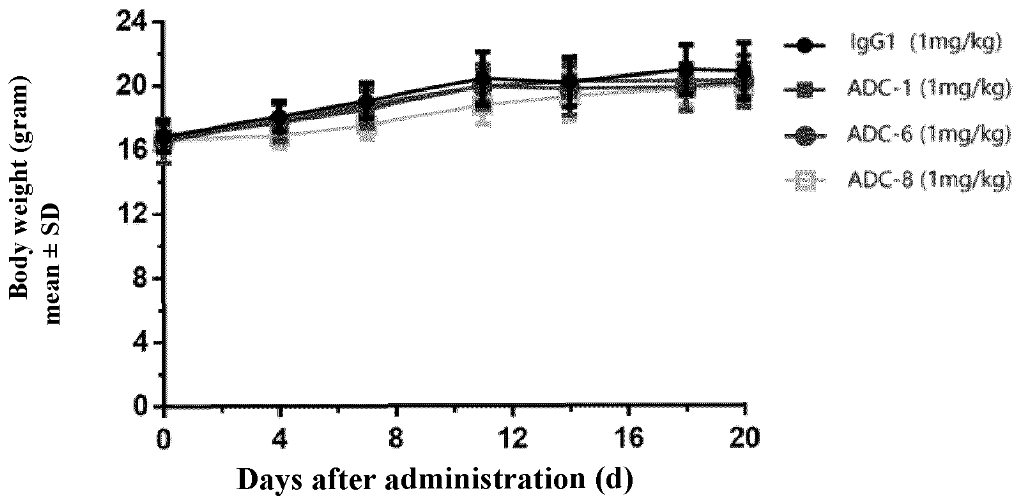


FIG. 7

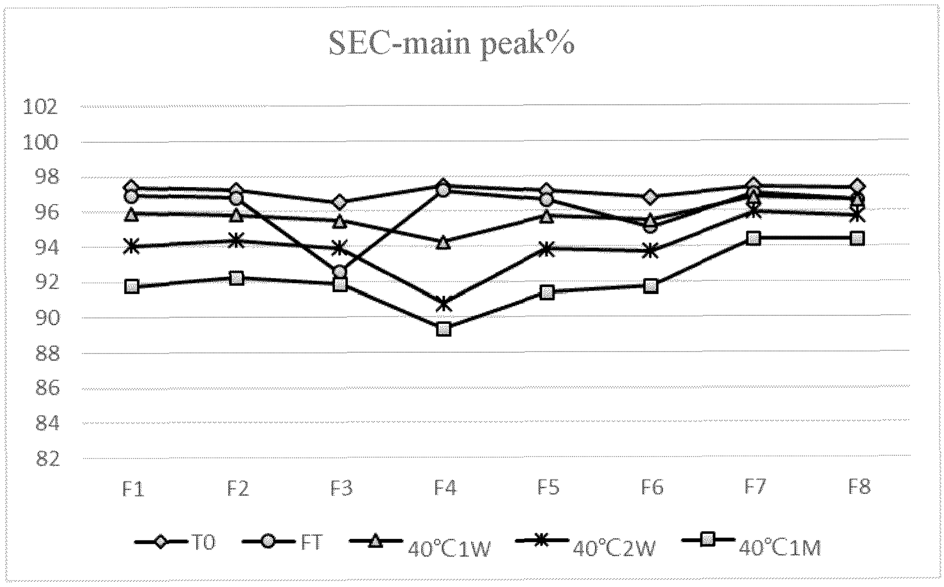


FIG. 8A

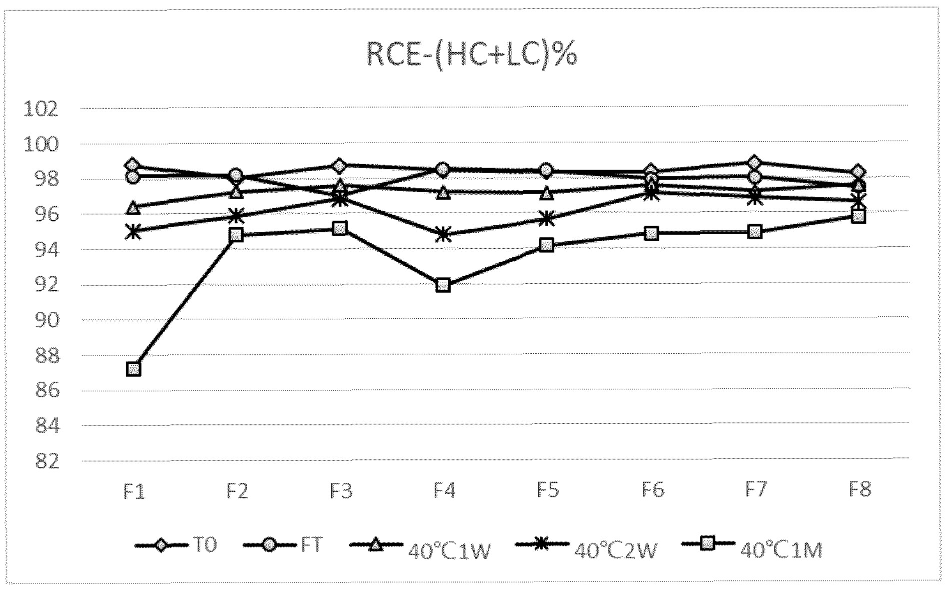


FIG. 8B

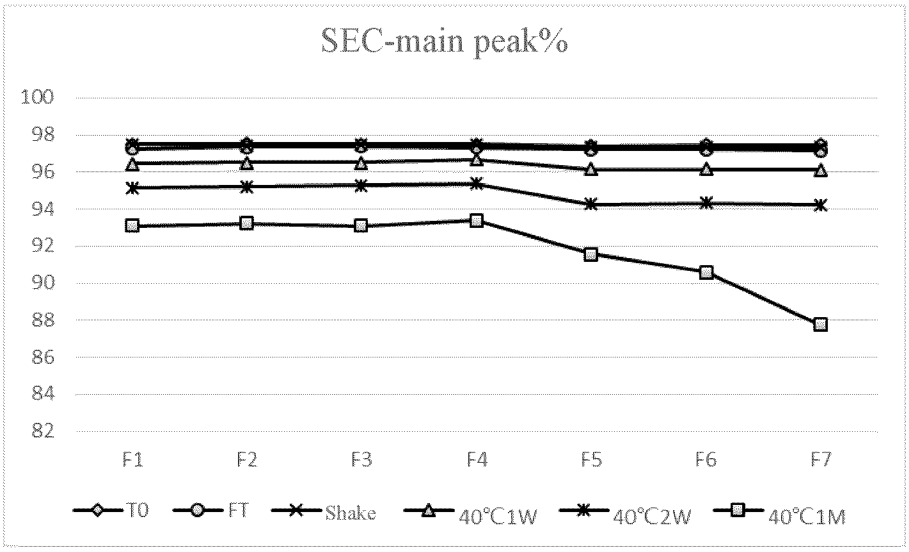


FIG. 9A

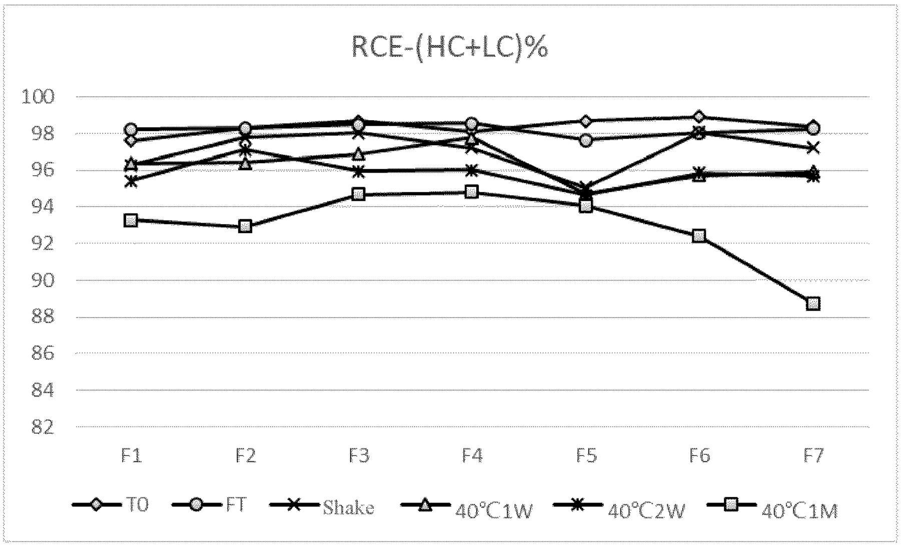


FIG. 9B

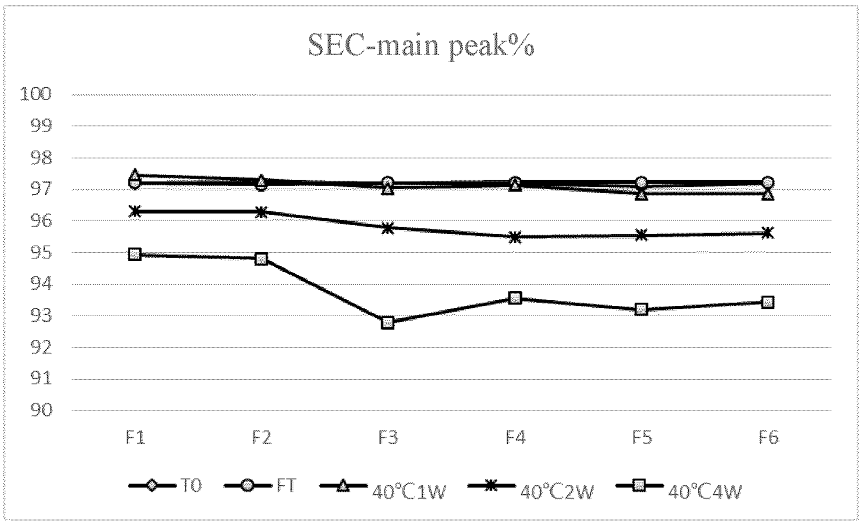


FIG. 10A

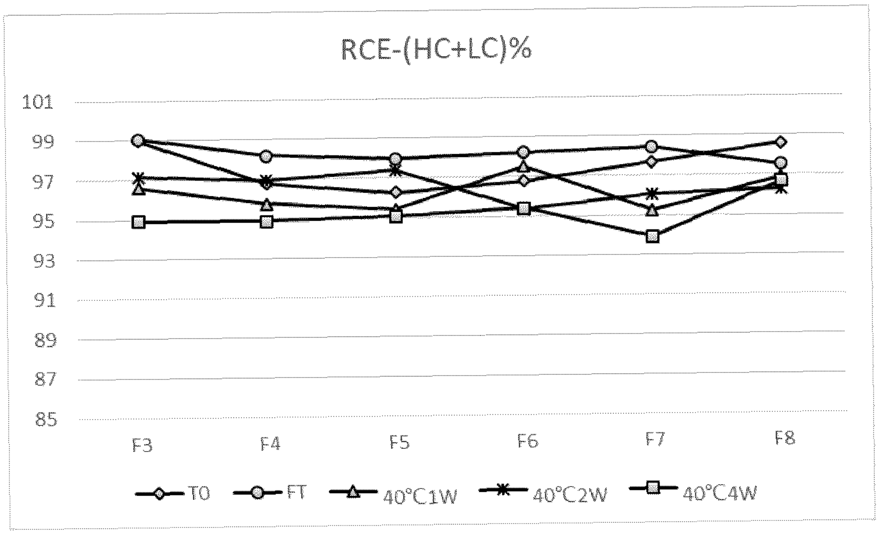
RCE-(HC+LC)%
101
99
97
95
93
91
89
87
85
F3
F4
F5
F6
F7
F8
T0
FT
40°C1W
40°C2W
40°C4W

FIG. 10B

**INTERNATIONAL SEARCH REPORT**

International application No.
**PCT/CN2023/073047**

**A. CLASSIFICATION OF SUBJECT MATTER**

A61K47/68(2017.01)i;C07K16/28(2006.01)i;A61K31/4745(2006.01)i;A61K9/08(2006.01)i;A61K9/19(2006.01)i;A61P35/00(2006.01)i

According to International Patent Classification (IPC) or to both national classification and IPC

**B. FIELDS SEARCHED**

Minimum documentation searched (classification system followed by classification symbols)

IPC: A61K C07K A61P

Documentation searched other than minimum documentation to the extent that such documents are included in the fields searched

Electronic data base consulted during the international search (name of data base and, where practicable, search terms used)

DWPI; CNABS; WPABS; WPABSC; ENTXTC; CNTXT; CJFD; CNKI; STN; VEN; PubMed: 抗CD79b, 抗体, 偶联, 缀合, 耦联, 偶合, 冻干, 表面活性剂, 缓冲, 组氨酸, 琥珀酸, 柠檬酸, 海藻糖, 蔗糖, 聚山梨酯, 吐温, 结构检索, structural search, 式(I), formula (I), 权利要求12结构, claim 12 structure, ADC-5结构, ADC-5 structure, ADC, Antibody, Drug, Conjugation, Lyophili+, Surfactant, Buffer, Histidine, Succinic Acid, Citric Acid, Trehalose, Sucrose, Polysorbate, Tween

**C. DOCUMENTS CONSIDERED TO BE RELEVANT**

| Category* | Citation of document, with indication, where appropriate, of the relevant passages | Relevant to claim No. |
|---|---|---|
| X | WO 2021148003 A1 (SHANGHAI SENHUI MEDICINE CO., LTD.; SHANGHAI SHENGDI PHARMACEUTICAL CO., LTD.; SHANGHAI HENGRUI PHARMACEUTICAL CO., LTD.; JIANGSU HENGRUI MEDICINE CO., LTD.) 29 July 2021 (2021-07-29) claims 44-46, description page 19 lines 14-20, page 21 paragraphs 6-7, page 53 paragraphs 1-2, embodiments 2-18 | 1-2, 4, 10, 11, 18 |
| Y | WO 2021148003 A1 (SHANGHAI SENHUI MEDICINE CO., LTD.; SHANGHAI SHENGDI PHARMACEUTICAL CO., LTD.; SHANGHAI HENGRUI PHARMACEUTICAL CO., LTD.; JIANGSU HENGRUI MEDICINE CO., LTD.) 29 July 2021 (2021-07-29) claims 44-46, description page 19 lines 14-20, page 21 paragraphs 6-7, page 53 paragraphs 1-2, embodiments 2-18 | 3, 5-9, 12-17 |
| Y | WO 2021190581 A1 (JIANGSU HENGRUI MEDICINE CO., LTD.; SHANGHAI HENGRUI PHARMACEUTICAL CO., LTD.) 30 September 2021 (2021-09-30) claims 1-7, 13-19 | 3, 5-9, 12-17 |
| Y | CN 113116812 A (BIO-THERA SOLUTIONS, LTD.) 16 July 2021 (2021-07-16) claims 1-3, 6, 11-13, description paragraphs [0057], [0209] | 1-18 |

☑ Further documents are listed in the continuation of Box C.    ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **27 April 2023** | **07 May 2023** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No. **PCT/CN2023/073047**

**C. DOCUMENTS CONSIDERED TO BE RELEVANT**

| Category* | Citation of document, with indication, where appropriate, of the relevant passages | Relevant to claim No. |
|---|---|---|
| Y | WO 2020156439 A1 (TUOJIE BIOTECH (SHANGHAI) CO., LTD.) 06 August 2020 (2020-08-06)<br>embodiments 3-4, table 3 | 1-18 |
| Y | WO 2020233534 A1 (BIO-THERA SOLUTIONS, LTD.) 26 November 2020 (2020-11-26)<br>claims 6-12 | 1-18 |
| PY | WO 2022022508 A1 (TUOJIE BIOTECH (SHANGHAI) CO., LTD.) 03 February 2022 (2022-02-03)<br>claims 1-4, 7, 11, 37, 40-42, embodiments 3-5 ADC-5 | 1-18 |

Form PCT/ISA/210 (second sheet) (July 2022)

**INTERNATIONAL SEARCH REPORT**

International application No.
**PCT/CN2023/073047**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**

International application No.
**PCT/CN2023/073047**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **19-20**
because they relate to subject matter not required to be searched by this Authority, namely:

Claim 19 sets forth a method for treating or preventing proliferative diseases or delaying the progression of proliferative diseases, and claim 20 sets forth a method for enhancing an immune function in a subject having B cell proliferative disorders or autoimmune disorders, which belong to methods for treatment of diseases, and fall within the cases set forth in PCT Rule 39.1(iv) for which a search is not required.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/CN2023/073047**

| Patent document cited in search report | Publication date (day/month/year) | Patent family member(s) | Publication date (day/month/year) |
|---|---|---|---|
| WO 2021148003 A1 | 29 July 2021 | AU 2021210079 A1 | 25 August 2022 |
| | | BR 112022014398 A2 | 13 September 2022 |
| | | EP 4094779 A1 | 30 November 2022 |
| | | TW 202140078 A | 01 November 2021 |
| | | JP 2023511163 A | 16 March 2023 |
| | | KR 20220130160 A | 26 September 2022 |
| | | CA 3168654 A1 | 29 July 2021 |
| WO 2021190581 A1 | 30 September 2021 | BR 112022019073 A2 | 08 November 2022 |
| | | KR 20220157998 A | 29 November 2022 |
| | | AU 2021243073 A1 | 13 October 2022 |
| | | TW 202144013 A | 01 December 2021 |
| | | EP 4129345 A1 | 08 February 2023 |
| | | CA 3175733 A1 | 30 September 2021 |
| CN 113116812 A | 16 July 2021 | WO 2021136274 A1 | 08 July 2021 |
| WO 2020156439 A1 | 06 August 2020 | CA 3127556 A1 | 06 August 2020 |
| | | TW 202031686 A | 01 September 2020 |
| | | EP 3919516 A1 | 08 December 2021 |
| | | EP 3919516 A4 | 30 November 2022 |
| | | JP 2022518062 A | 11 March 2022 |
| | | KR 20210121102 A | 07 October 2021 |
| | | AU 2020213565 A1 | 05 August 2021 |
| | | US 2022162304 A1 | 26 May 2022 |
| WO 2020233534 A1 | 26 November 2020 | CN 111939267 A | 17 November 2020 |
| | | HK 40034012A0 A0 | 23 April 2021 |
| WO 2022022508 A1 | 03 February 2022 | AU 2021317378 A1 | 09 March 2023 |
| | | CA 3184724 A1 | 03 February 2022 |
| | | BR 112023001359 A2 | 14 February 2023 |
| | | TW 202214306 A | 16 April 2022 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202210093514
- WO 2020063673 A
- US 20050238649 A1
- US 5208020 A
- WO 2018142322 A
- WO 2020156439 A
- WO 2020063676 A
- CN 202010073671
- US 7098308 B
- US 6884869 B
- CN 201911390425


### Non-patent literature cited in the description


- *J. Biol. Chem*, 1968, vol. 243, 3558
- **HOLLIGER et al.** *Proc. Natl. Acad. Sci. USA*, 1993, vol. 90, 6444-6448
- **ALFTHAN et al.** *Protein Eng.*, 1995, vol. 8, 725-731
- **CHOI et al.** *Eur. J. Immunol.*, 2001, vol. 31, 94-106
- **HU et al.** *Cancer Res.*, 1996, vol. 56, 3055-3061
- **KIPRIYANOV et al.** *J. Mol. Biol.*, 1999, vol. 293, 41-56
- **ROOVERS et al.** *Cancer Immunol.*, 2001
- **KABAT E.A. et al.** *Sequences of proteins of immunological interest.*, 1991
- **KABAT et al.** Sequences of Proteins of Immunological Interest. Public Health Service, National Institutes of Health, 1991
- **AL-LAZIKANI et al.** *JMB*, 1997, vol. 273, 927-948
- **LEFRANC M. P.** *Immunologist*, 1999, vol. 7, 132-136
- **LEFRANC, M. P. et al.** *Dev. Comp. Immunol.*, 2003, vol. 27, 55-77
- Epitope Mapping Protocols in Methods in Molecular Biology. 1996, vol. 66
- **CHARI et al.** *Cancer Research*, 1992, vol. 52, 127-131
- Antibodies: A Laboratory Manual and Molecular Cloning: A Laboratory Manual. Cold Spring Harbor Laboratory
- *Leukemia.*, July 2015, vol. 29 (7), 1578-86
- *Blood.*, 15 July 2007, vol. 110 (2), 616-23